# EUROPEAN PATENT APPLICATION

(11) **EP 4 651 157 A1**
(43) Date of publication of application: **19.11.2025**
(21) Application number: 25175599.7
(22) Date of filing: 12.05.2025
(51) Int. Cl.: H01B 1/12, A61B 5/25

(54) **BIO-ELECTRODE COMPOSITION, BIO-ELECTRODE, AND METHOD FOR PRODUCING BIO-ELECTRODE**

(30) Priority: 16.05.2024 JP 2024080130
(71) Applicant: SHIN-ETSU CHEMICAL CO., LTD., Tokyo 1000005 (JP)
(72) Inventor: Hatakeyama, Jun, Niigata (JP); Ohashi, Masaki, Niigata (JP); Ikeda, Joe, Tokyo, 1000-0005 (JP)
(74) Representative: Schicker, Silvia

(57) **Abstract**

The present invention is a bio-electrode composition includes (A) an ionic resin, wherein the component (A) contains a resin having a structure selected from an ammonium salt, a lithium salt, a sodium salt, and a potassium salt of trissulfonium methide. This provides a bio-electrode composition capable of forming a living body contact layer for a bio-electrode, which is excellent in electric conductivity and biocompatibility and lightweight, can be produced at low cost, causes no significant decrease in the electric conductivity even when gets wet from water or when dried, and is soft with excellent stretchability and adhesiveness; a bio-electrode including a living body contact layer formed from the bio-electrode composition; and a method for producing the bio-electrode.

## Description

### TECHNICAL FIELD

The present invention relates to: a bio-electrode that is used in contact with skin of a living body and capable of detecting physical conditions such as heart rate by an electric signal from the skin; a method for producing the bio-electrode; and a bio-electrode composition suitable for use for a bio-electrode.

### BACKGROUND ART

In recent years, as IoT (Internet of Things) becomes more widespread, development of wearable devices is promoted. Typical examples thereof include watches and eyeglasses with Internet access. Also in the fields of medicine and sports, wearable devices capable of constantly monitoring physical conditions are demanded and considered as future growth sectors.

In the field of medicine, wearable devices have been examined for monitoring a state of the body's organs by sensing an extremely weak current, as in electrocardiogram measurement which detects heart activity by electric signals, for example. The electrocardiogram measurement is conducted by attaching an electrode coated with electro-conductive paste to a body, and is only one-time measurement for a short period of time. In contrast, the development of the medical wearable devices as described above aims to provide a device that continuously monitors health conditions for a few weeks. Accordingly, a bio-electrode used in a medical wearable device is required to have no change in electric conductivity even during long-term use and cause no skin allergies. In addition to these, it is also required that the bio-electrode is lightweight and can be produced at low cost.

Medical wearable devices include a type of a device that is attached to a body and a type of a device that is incorporated into clothes. As the type of the device that is attached to a body, a bio-electrode has been proposed, which uses water-soluble gel containing water and an electrolyte as materials for the above electro-conductive paste (Patent Document 1). The water-soluble gel contains sodium, potassium, or calcium as the electrolyte in a water-soluble polymer for retaining water, and converts a change of ion concentration from skin into electricity. On the other hand, as the type of the device that is incorporated into clothes, the proposed method uses, for electrodes, a cloth in which an electro-conductive polymer such as PEDOT-PSS (Poly-3,4-ethylenedioxythiophene-Polystyrenesulfonate) or silver paste is incorporated into fibers, (Patent Document 2).

However, the use of the above water-soluble gel containing water and the electrolyte has a problem of electric conductivity loss when the water dries out. Meanwhile, the use of metal with high ionization tendency such as copper has a risk of causing some people to suffer from skin allergies. The use of an electro-conductive polymer such as PEDOT-PSS also has a risk of causing skin allergies due to strong acidity of the electro-conductive polymer, as well as a problem of peeling off of the electro-conductive polymer from fibers during washing.

Furthermore, the use of metal nanowire, carbon black, carbon nanotube, and the like as electrode materials has been considered because of their excellent electric conductivity (Patent Documents 3, 4, and 5). Because of a higher probability of metal nanowire contacting with each other, the metal nanowire in a small addition amount is capable of conducting electricity. However, since the metal nanowire is a thin material with sharp tips, it causes skin allergies. Accordingly, even if a material itself causes no allergic reaction, biocompatibility may be degraded depending on the shape and stimulativeness of the material, thereby making it difficult to achieve both the electric conductivity and biocompatibility.

Although metal films seem to function as an excellent bio-electrode thanks to extremely high electric conductivity, this is not always the case. Heartbeat causes the skin to release not only an extremely weak current but also a sodium ion, a potassium ion, and a calcium ion. It is thus necessary to convert a change of ion concentration into a current. Noble metal, however, is difficult to ionize and thus inefficient in converting ions from skin to a current. Therefore, a bio-electrode using noble metal leads to high impedance and high resistance to the skin during electrical conduction.

Meanwhile, a battery with an ionic liquid added thereto has been considered (Patent Document 6). The ionic liquid is characterized by high thermal and chemical stability and excellent electric conductivity, and application thereof to a battery becomes widespread. However, the ionic liquid having a small molecular weight as disclosed in Patent Document 6 dissolves in water, and thus the use of the bio-electrode with the ionic liquid added thereto causes extraction of the ionic liquid due to perspiration from skin, resulting in not only reduced electric conductivity, but also penetration thereof into skin to cause skin problems.

Moreover, a battery using a polymer-type sulfonimide lithium salt has been considered (Non Patent Document 1). However, while lithium has been applied to batteries because of its high ion mobility, it is not a material having biocompatibility. Furthermore, a lithium salt of fluorosulfonic acid in a form of a pendant on silicone has also been considered (Non Patent Document 2).

Bio-electrode materials in which an ionic polymer is mixed with a silicone adhesive have been proposed (Patent Documents 7 and 8). These materials not only have high ionic conductivity, but also exhibits high electronic conductivity when conductive powder such as carbon and silver is added thereto. Hence, they function as an excellent bio-electrode. By combining a silicone adhesive that has a low probability of skin allergies, high water repellency, and an effect of suppressing itching and skin redness after removal thereof, with an ionic polymer that has high ionic conductivity and does not penetrate into skin, it is possible to obtain stable biological signals without peeling off even in a case of long-term attachment, including daily bathing and exercise. However, further improvement in the comfort in the case of long-term attachment has been demanded.

Here, health effects of perfluoroalkyl compounds (PFAS) have been pointed out, and there is a movement to restrict production and sale of the PFAS compounds under REACH in Europe. It is urgent to develop materials having no PFAS structures.

### CITATION LIST

### PATENT LITERATURE

Patent Document 1: WO 2013/039151 A1
Patent Document 2: JP 2015-100673 A
Patent Document 3: JP H05-095924 A
Patent Document 4: JP 2003-225217 A
Patent Document 5: JP 2015-019806 A
Patent Document 6: JP 2004-527902 A
Patent Document 7: JP 2018-126496 A
Patent Document 8: JP 2018-130533 A

### NON PATENT LITERATURE

Non Patent Document 1: J. Mater. Chem. A, 2016, 4, p10038-10069
Non Patent Document 2: J. of the Electrochemical Society, 150(8) A1090-A1094 (2003)

### SUMMARY OF INVENTION

### TECHNICAL PROBLEM

The present invention has been made to solve the above problems, and aims to provide: a bio-electrode composition capable of forming a living body contact layer for a bio-electrode, which is excellent in electric conductivity and biocompatibility and lightweight, can be produced at low cost, and is soft with excellent stretchability and adhesiveness; a bio-electrode including a living body contact layer formed from the bio-electrode composition; and a method for producing the bio-electrode.

### SOLUTION TO PROBLEM

To achieve the above object, the present invention provides a bio-electrode composition including (A) an ionic resin,
wherein the component (A) contains a resin having a structure selected from an ammonium salt, a lithium salt, a sodium salt, and a potassium salt of trissulfonium methide.

Such bio-electrode composition is capable of forming a living body contact layer for a bio-electrode, which is excellent in electric conductivity and biocompatibility and lightweight, can be produced at low cost, causes no significant decrease in the electric conductivity even when gets wet from water or when dried, and is soft with excellent stretchability and adhesiveness.

Further, the resin having a structure selected from an ammonium salt, a lithium salt, a sodium salt, and a potassium salt of trissulfonium methide preferably has a chemical structure represented by the following general formula (1), 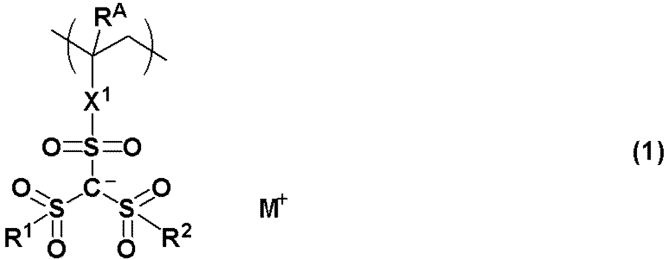 wherein R^{A} represents a hydrogen atom or a methyl group; X¹ each independently represents a single bond, a phenylene group, or a linking group having 1 to 20 carbon atoms and containing at least one selected from an ester bond, an ether bond, an urethane bond, a lactone ring, and a halogen atom; each of R¹ and R² independently represents a hydrocarbyl group having 1 to 20 carbon atoms and optionally containing a heteroatom; and M⁺ represents any of an ammonium ion, a lithium ion, a sodium ion, and a potassium ion.

Specific examples of the structure selected from an ammonium salt, a lithium salt, a sodium salt, and a potassium salt of trissulfonium methide include these structures.

Further, the resin having a structure selected from an ammonium salt, a lithium salt, a sodium salt, and a potassium salt of trissulfonium methide preferably contains an ammonium ion represented by the following general formula (2) as the M⁺, 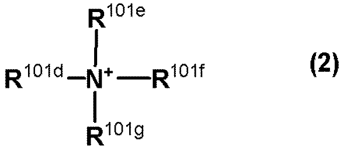 wherein each of R^{101d}, R^{101e}, R^{101f}, and R^{101g} represents a hydrogen atom, a linear, branched, or cyclic alkyl group having 1 to 12 carbon atoms, a linear, branched, or cyclic alkenyl group or alkynyl group having 2 to 12 carbon atoms, or an aromatic group having 4 to 20 carbon atoms, and optionally contains one or more selected from an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring with a nitrogen atom attached thereto, and when forming the ring, R^{101d} and R^{101e}, or R^{101d}, R^{101e}, and R^{101f} represent an alkylene group having 3 to 10 carbon atoms, or form a heteroaromatic ring having the nitrogen atom in the formula in the ring.

A specific example of the ammonium ion in the ammonium salt of trissulfonium methide is such ammonium ion.

Furthermore, a resin other than the component (A) is preferably contained as a component (B).

By containing the resin of the component (B), it is possible to retain the respective components contained in the composition and further improve adhesiveness of the composition.

Further, the component (B) is preferably one or more selected from a silicone resin, a (meth)acrylate resin, and an urethane resin.

Such resins can be suitably used as the resin of the component (B).

Further, the component (B) preferably has adhesiveness.

A resin having adhesiveness is suitable as the resin of the component (B).

Further, a silicone resin having a RₓSiO_{(4-x)/2} unit (R represents a substituted or unsubstituted monovalent hydrocarbon group having 1 to 10 carbon atoms; and "x" is within a range of 2.5 to 3.5) and a SiO₂ unit is preferably contained as the component (B).

Such a silicone resin can also be suitably used as the resin of the component (B).

Furthermore, carbon powder and/or metal powder is preferably contained as a component (C).

The use of such component (C) can improve electric conductivity of the composition.

In this aspect, the carbon powder is preferably one or both of carbon black and carbon nanotube.

These can be suitably used as the carbon powder.

Further, the metal powder is preferably powder of a metal selected from gold, silver, platinum, copper, tin, titanium, nickel, aluminum, tungsten, molybdenum, ruthenium, chromium, and indium.

In this aspect, the metal powder is preferably silver powder.

These can be suitably used as the metal powder.

Further, the bio-electrode composition preferably further includes an organic solvent as a component (D).

Such composition will exhibit excellent application performance.

Additionally, the present invention provides a bio-electrode including an electro-conductive base material and a living body contact layer formed on the electro-conductive base material, wherein the living body contact layer is a cured material of the above-described bio-electrode composition.

Such bio-electrode can include a living body contact layer that is excellent in electric conductivity and biocompatibility and lightweight, can be produced at low cost, causes no significant decrease in the electric conductivity even when gets wet from water or when dried, and is soft with excellent stretchability and adhesiveness.

Further, the electro-conductive base material preferably includes one or more selected from gold, silver, silver chloride, platinum, aluminum, magnesium, tin, tungsten, iron, copper, nickel, stainless steel, chromium, titanium, carbon, and an electro-conductive polymer.

These can be suitably used as the electro-conductive base material.

Additionally, the present invention provides a method for producing a bio-electrode including an electro-conductive base material and a living body contact layer formed on the electro-conductive base material, the method including: applying the above-described bio-electrode composition onto the electro-conductive base material; and curing the bio-electrode composition to form the living body contact layer.

The bio-electrode of the present invention can be produced in this manner.

Further, the electro-conductive base material to be used preferably includes one or more selected from gold, silver, silver chloride, platinum, aluminum, magnesium, tin, tungsten, iron, copper, nickel, stainless steel, chromium, titanium, carbon, and an electro-conductive polymer.

These can be suitably used as the electro-conductive base material.

### ADVANTAGEOUS EFFECTS OF INVENTION

As described above, with the bio-electrode composition according to the embodiment of the present invention, it is possible to provide: a bio-electrode composition capable of forming a living body contact layer for a bio-electrode, which is excellent in electric conductivity and biocompatibility and lightweight, can be produced at low cost, causes no significant decrease in the electric conductivity even when gets wet from water or when dried, and is soft with excellent stretchability and adhesiveness; a bio-electrode including a living body contact layer formed from the bio-electrode composition; and a method for producing the bio-electrode.

### BRIEF DESCRIPTION OF DRAWINGS

FIG. 1 is a schematic cross-sectional view of a bio-electrode according to an embodiment of the present invention;
FIG. 2 is a schematic cross-sectional view of a bio-electrode attached to a living body, according to an embodiment of the present invention;
FIG. 3 is a schematic illustration of printed bio-electrodes fabricated in Example of the present invention;
FIG. 4 is a schematic illustration of one of bio-electrodes fabricated in Example of the present invention, wherein the bio-electrode is cut out and an adhesive layer and electric wire are attached thereto;
FIG. 5 is a diagram showing attachment positions of electrodes and an earth on a human body upon measurement of biological signals in Example of the present invention; and
FIG. 6 is one electrocardiogram waveform obtained by using a bio-electrode in Example of the present invention.

### DESCRIPTION OF EMBODIMENTS

As described above, it has been demanded to develop: a bio-electrode composition capable of forming a living body contact layer for a bio-electrode, which is excellent in electric conductivity and biocompatibility and lightweight, can be produced at low cost, is soft with stretchability and adhesiveness, enables to stably obtain electric signals even when attached to skin for a long time and gets wet from water, such as in bathing, or when dried, and leaves no residue on the skin after peeled off from the skin; a bio-electrode including a living body contact layer formed from the bio-electrode composition; and a method for producing the bio-electrode.

Sodium, potassium, and calcium ions are released from a skin surface in association with heartbeat. A bio-electrode should convert the increase and decrease of the ions released from the skin to electric signals. Accordingly, a material with excellent ionic conductivity is required to transmit the increase and decrease of the ions.

Softness, stretchability, and adhesiveness are necessary for a bio-electrode film to be attached to skin to stably obtain biological signals. Corneum of the outermost layer of the skin is renewed daily, and old corneum (dirt) accumulates between the attached bio-electrode film and the skin. The old corneum easily peels off from the outermost layer of the skin, so that the bio-electrode is detached and as a result, biological signals cannot be obtained. Therefore, it is necessary for the bio-electrode to cause no decrease in the adhesiveness even during long-term attachment. Meanwhile, if a residue is left on the skin after peeling off the bio-electrode subsequent to long-term attachment thereof, it may cause eruption or rough skin.

Biological signals include ECG for detecting the movement of the heart, RPM for detecting pulmonary respiration, EEG for detecting brain waves, EGG for detecting the movement of visceral organs, EMG for detecting the movement of muscles, and the like. A dry electrode of the present invention is used as a sensor for detecting them. Additionally, it is also used as an electrode for supplying electric signals to a body.

High acidity of acid for forming a neutralized salt leads to strong ionic polarization, resulting in improved ionic conductivity. This is why lithium salts of bis(trifluoromethanesulfonyl) imidic acid and tris(trifluoromethanesulfonyl) methide acid show high ionic conductivity as a lithium-ion battery. On the other hand, the higher acid strength of the acid before formed into the neutralized salt results in a problem of stronger irritation to a living body caused by this salt. That is, there is a trade-off relation between the ionic conductivity and the irritation to a living body. However, a salt applied to a bio-electrode should achieve both of high ionic conductivity and low irritation to a living body.

In trissulfonium methide, a carbon atom that has electrons drawn from three directions by a sulfonyl group is negatively charged, and it is acidic even if there is no fluorine atom at the end of the sulfonyl group. If the carbon atom at the end of the sulfonyl group has a fluorine atom, the acidity is too high and the neutralized salt thereof is highly irritating to skin; while trissulfonium methide having no fluorine atom can properly achieve both of high ionic conductivity and low irritation to a living body. Since it has no fluorine atom, it does not fall under the definition of PFAS and has low environmental impact.

Trissulfonium methide has high steric hindrance around the negatively charged carbon atom, which increases the distance to positively charged ammonium, lithium, sodium, and potassium. This tends to easily cause hopping motion of ammonium, lithium, sodium, potassium, etc., thereby exhibiting high ionic conductivity.

An ionic compound has a property that the higher molecular weight thereof leads to decreased permeability into skin and less irritation to the skin. Accordingly, the ionic compound is preferably a polymer type with the higher molecular weight. Thus, the problem of irritation to skin can be avoided by polymerizing this ionic compound in a form having a polymerizable double bond to form a polymer, or by bonding the ionic compound to silicone, polyurethane, polyether, polyester, etc.

Furthermore, by using a mixture of this salt with a silicone-based, acrylic-based, or urethane-based adhesive (resin), for example, it is possible to constantly keep close adhesion on skin to obtain stable electric signals for a long period of time.

Thus, the present invention is a bio-electrode composition including (A) an ionic resin, wherein the component (A) contains a resin having a structure selected from an ammonium salt, a lithium salt, a sodium salt, and a potassium salt of trissulfonium methide.

Hereinafter, the present invention will be described in detail. However, the present invention is not limited thereto.

### <Bio-Electrode Composition>

The bio-electrode composition of the present invention includes (A) a resin having a structure selected from an ammonium salt, a lithium salt, a sodium salt, and a potassium salt of trissulfonium methide. Hereinafter, each component will be described in more detail.

### (A) Ionic Resin Having Ammonium Salt, Lithium Salt, Sodium Salt, or Potassium Salt of Trissulfonium Methide

The bio-electrode composition of the present invention is characterized by containing (A) an ionic resin having a structure selected from an ammonium salt, a lithium salt, a sodium salt, and a potassium salt of trissulfonium methide.

The ionic resin having a structure selected from an ammonium salt, a lithium salt, a sodium salt, and a potassium salt of trissulfonium methide preferably has a partial structure represented by the following general formula (1'), 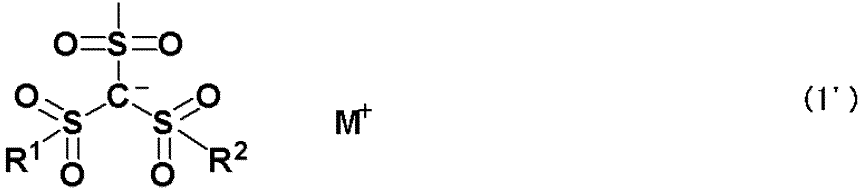 wherein each of R¹ and R² independently represents a hydrocarbyl group having 1 to 20 carbon atoms and optionally containing a heteroatom; and M⁺ represents any of an ammonium ion, a lithium ion, a sodium ion, and a potassium ion.

The partial structure of an ammonium salt, a lithium salt, a sodium salt, and a potassium salt of trissulfonium methide represented by the general formula (1') is, for example, attached to a resin selected from a resin obtained by polymerization of a monomer having a double bond, a silicone resin, and a polyurethane resin.

### (Repeating Unit-a)

The partial structure of an ammonium salt, a lithium salt, a sodium salt, and a potassium salt of trissulfonium methide represented by the above general formula (1') is preferably attached to a resin obtained by polymerizing monomers as a repeating unit-a represented by the following general formula (1), 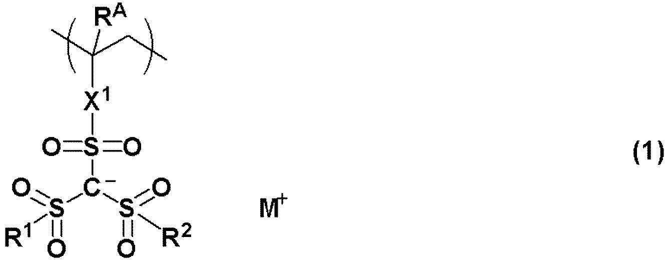 wherein R^{A} represents a hydrogen atom or a methyl group; X¹ each independently represents a single bond, a phenylene group, or a linking group having 1 to 20 carbon atoms and containing at least one selected from an ester bond, an ether bond, an urethane bond, a lactone ring, and a halogen atom; each of R¹ and R² independently represents a hydrocarbyl group having 1 to 20 carbon atoms and optionally containing a heteroatom; and M⁺ represents any of an ammonium ion, a lithium ion, a sodium ion, and a potassium ion.

A monomer to give the repeating unit-a represented by the above general formula (1) is represented by the following general formula (1)-1, 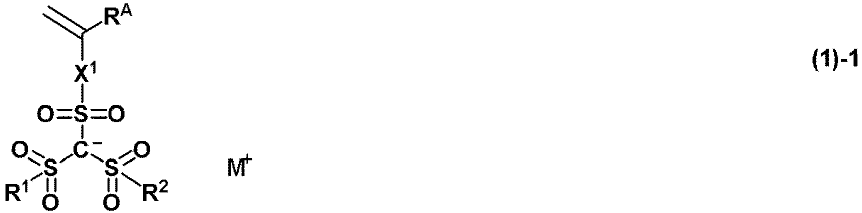 in the general formula, R^{A}, R¹, R², X₁, and M⁺ are as described above.

Specific examples of the monomer (anion moiety) represented by the above general formula (1)-1 are shown below.

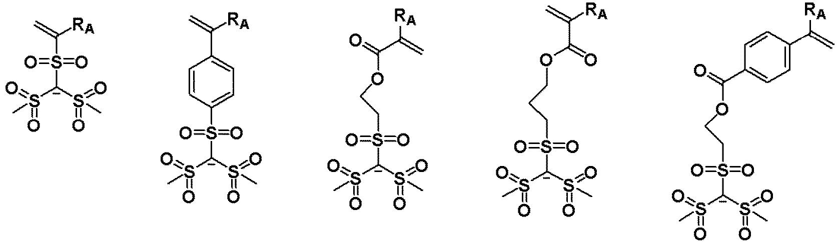

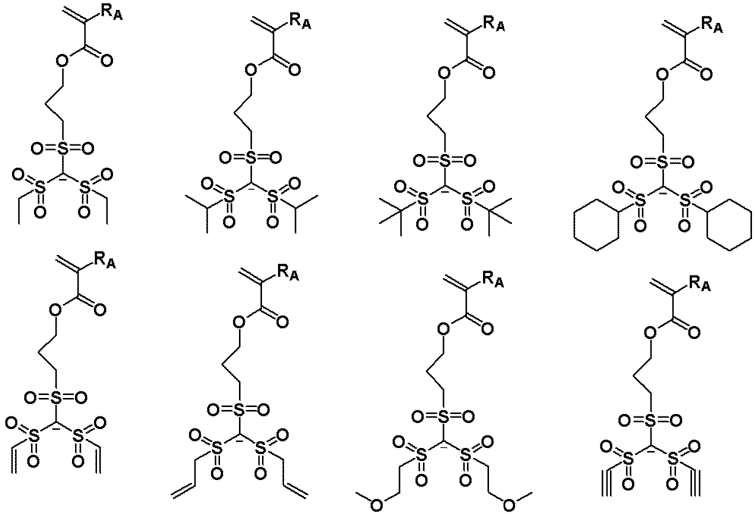

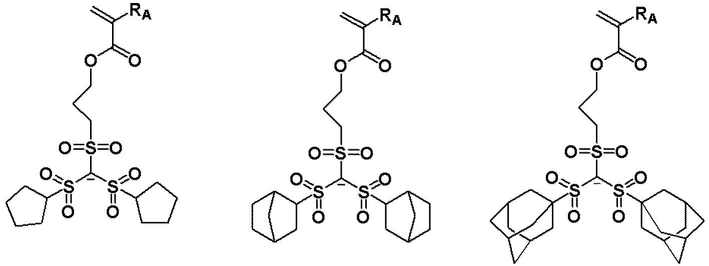

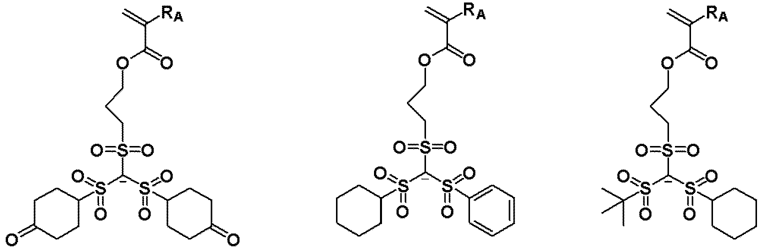

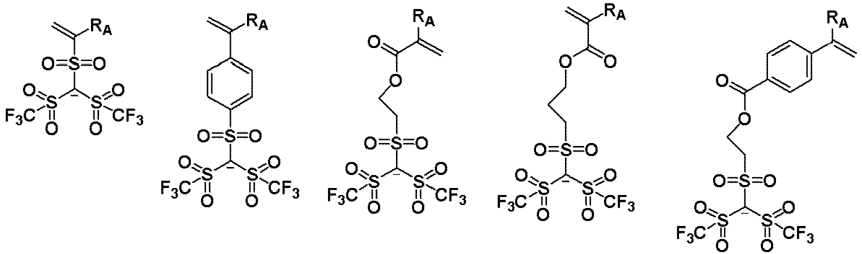

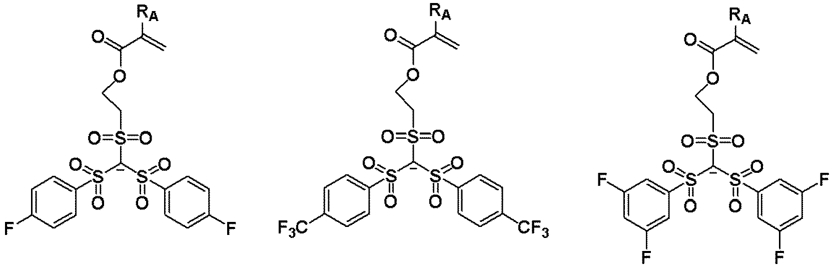

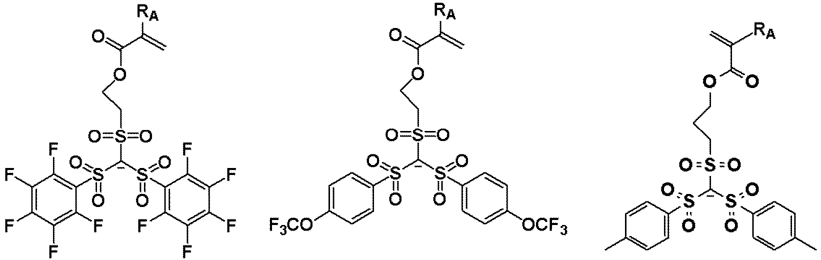

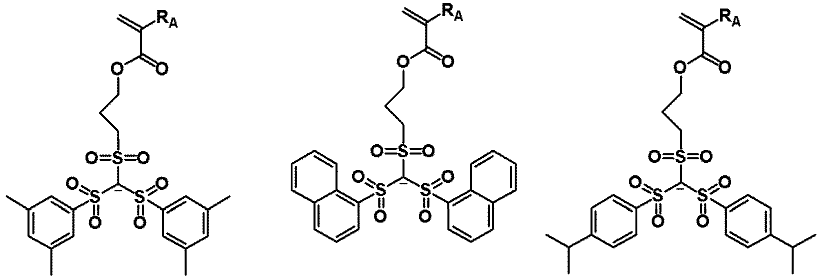

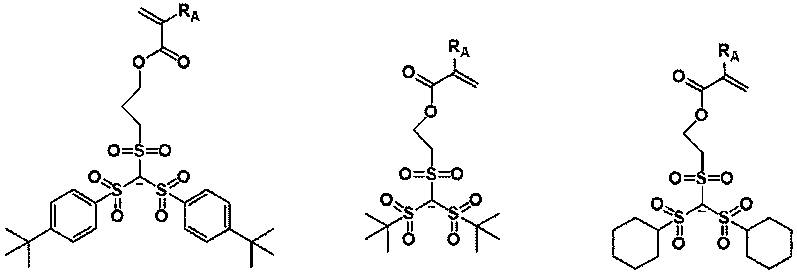

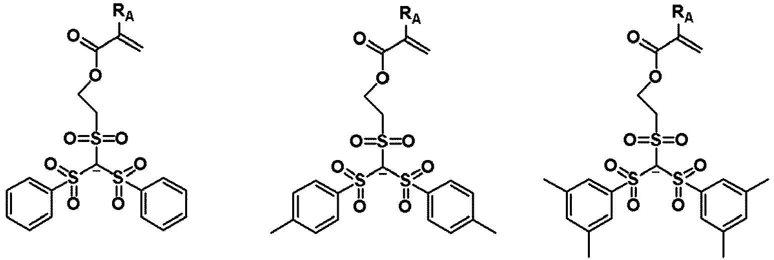

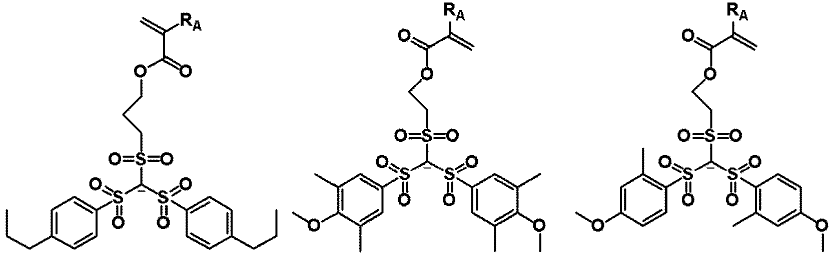

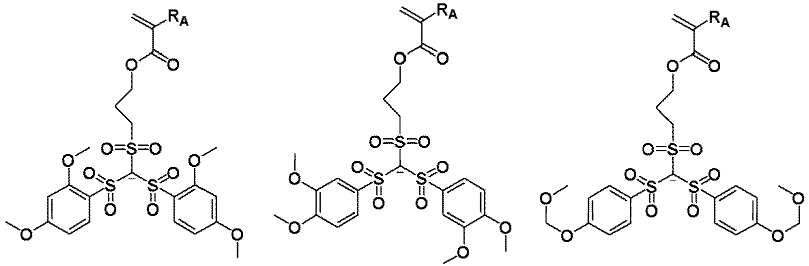

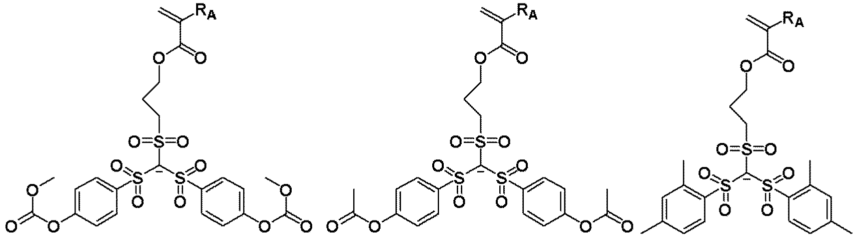

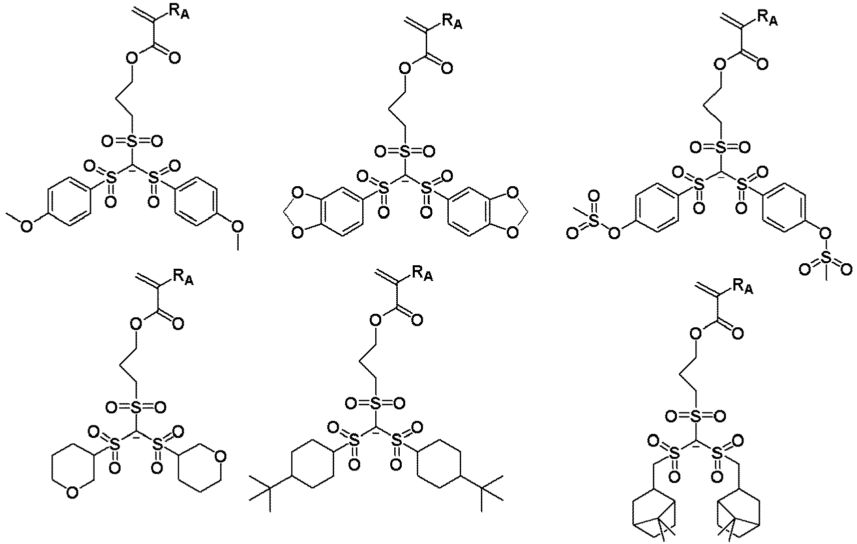

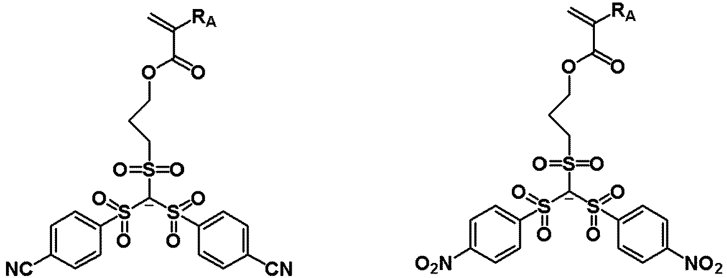

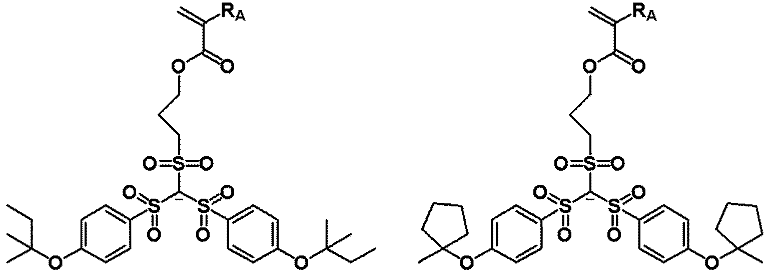

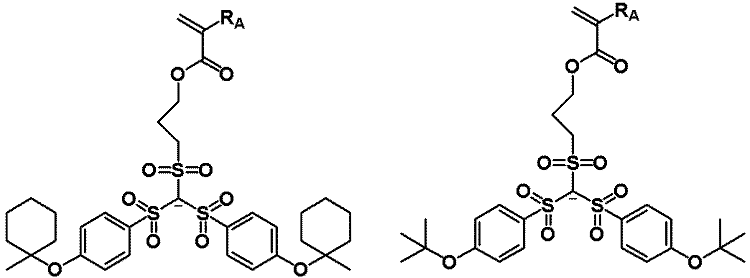

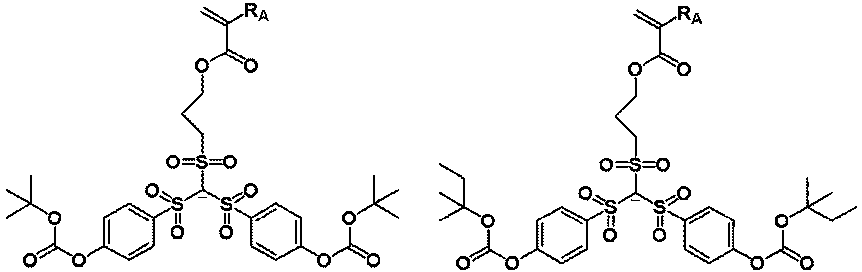

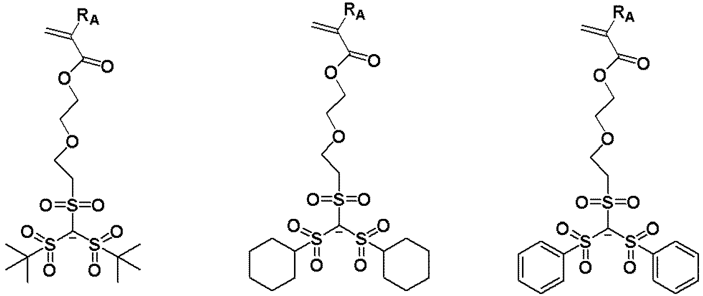

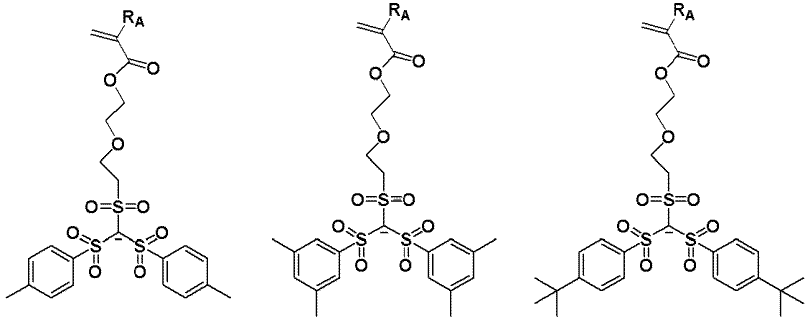

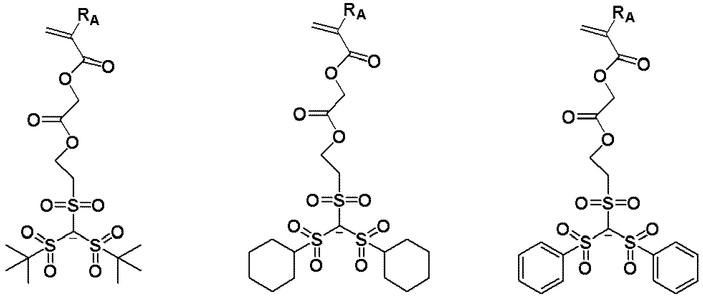

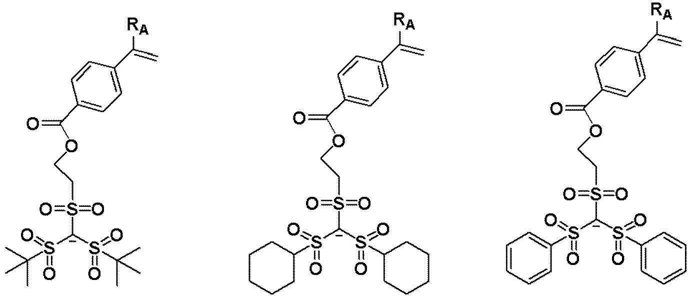

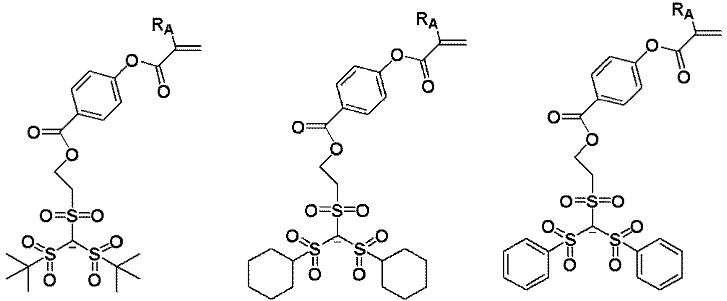

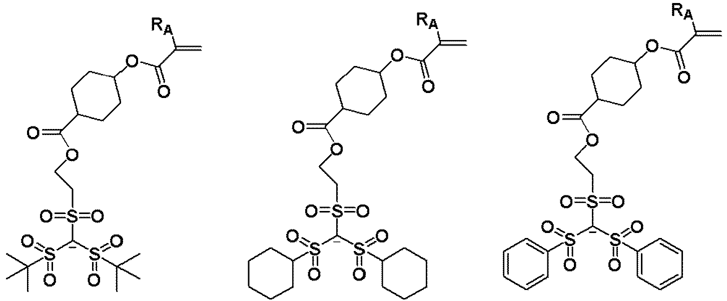

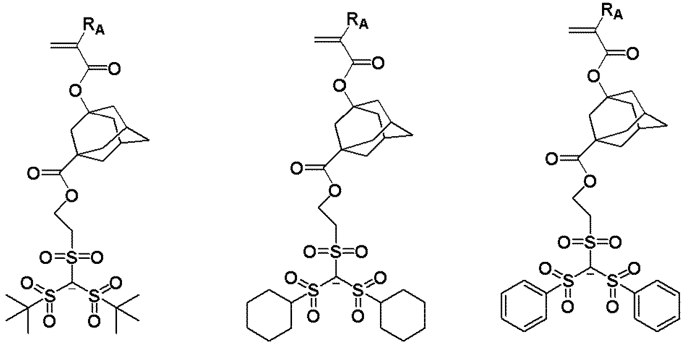

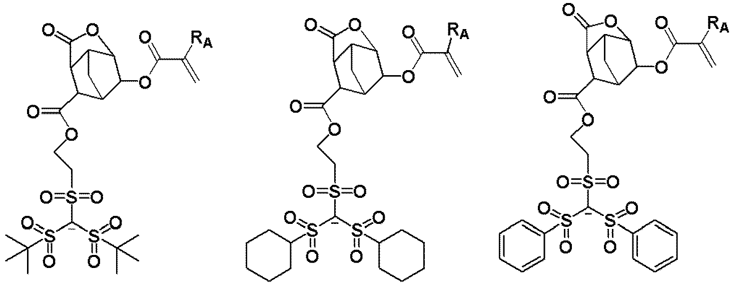

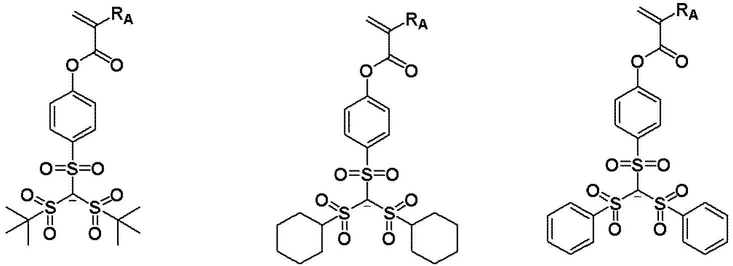

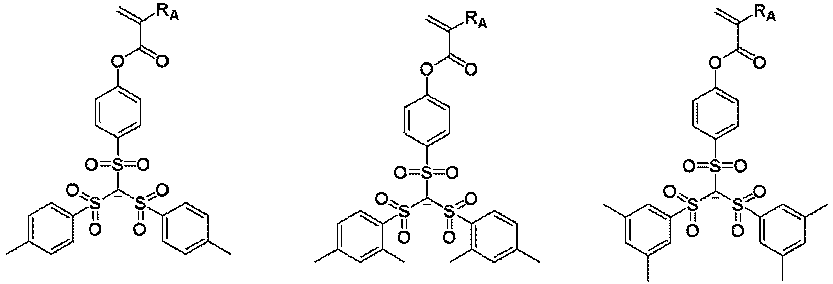

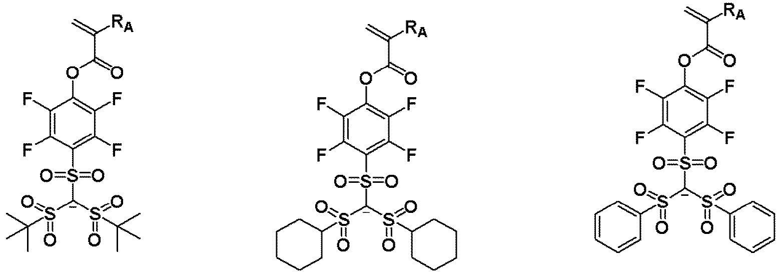

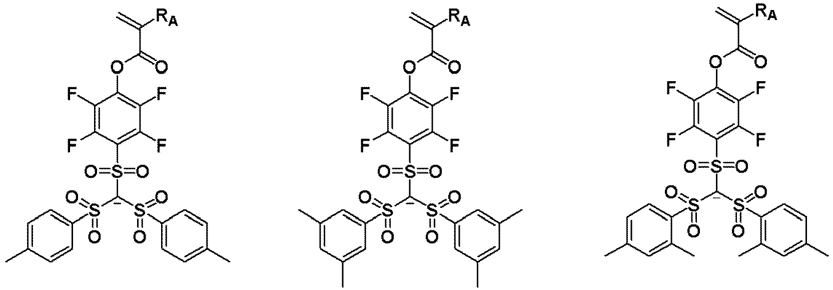

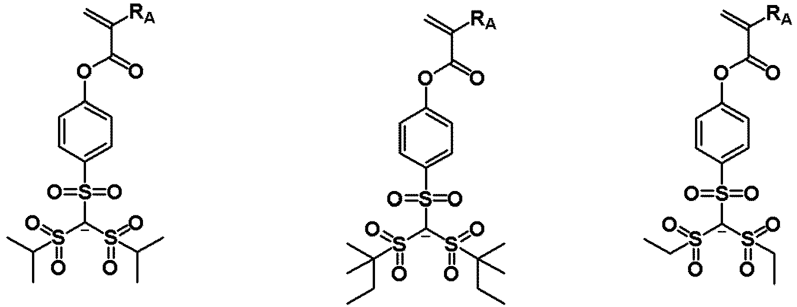

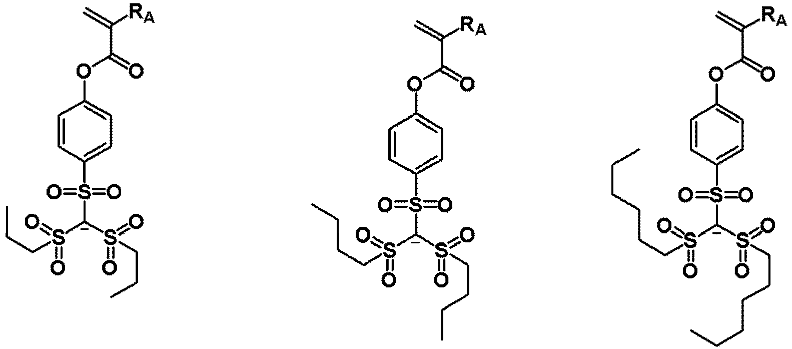

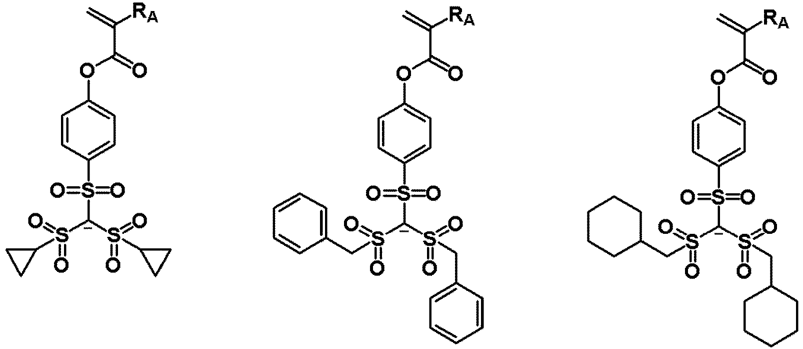

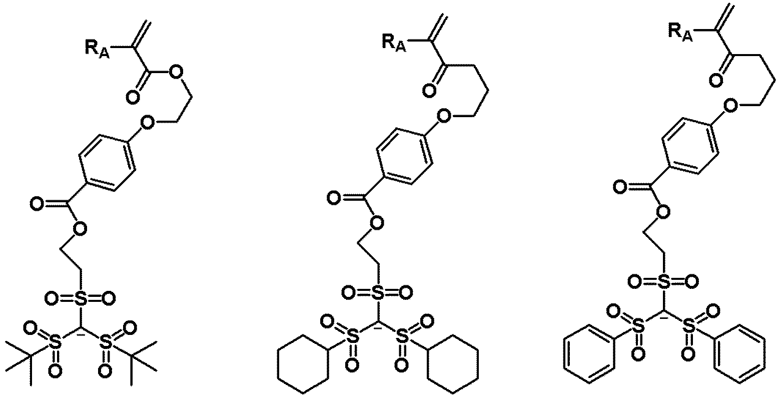

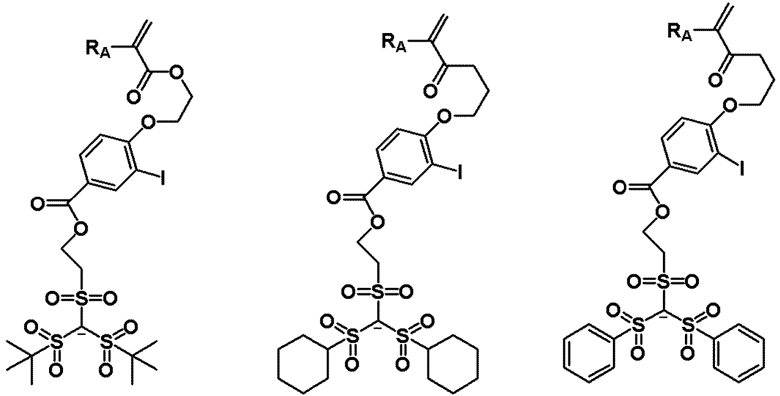

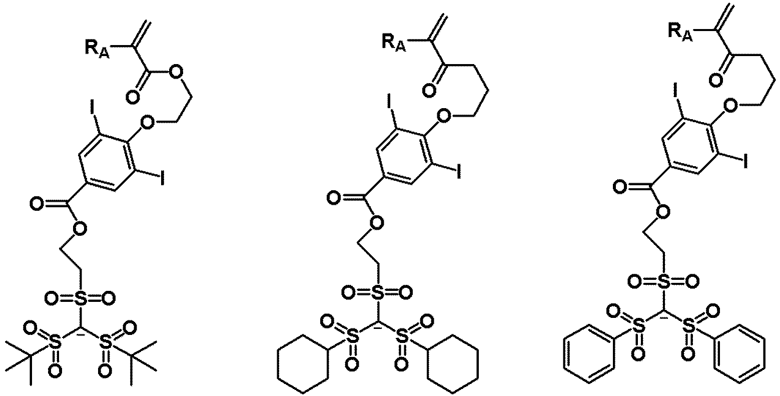

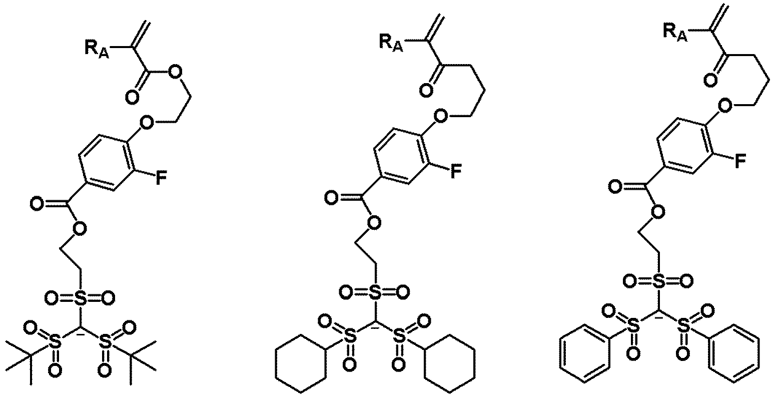

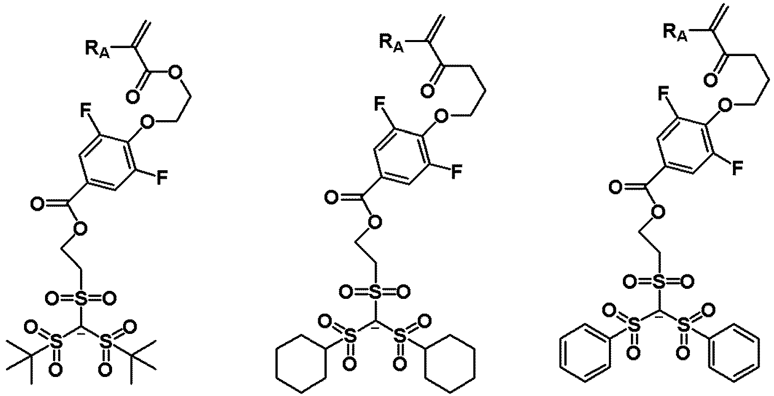

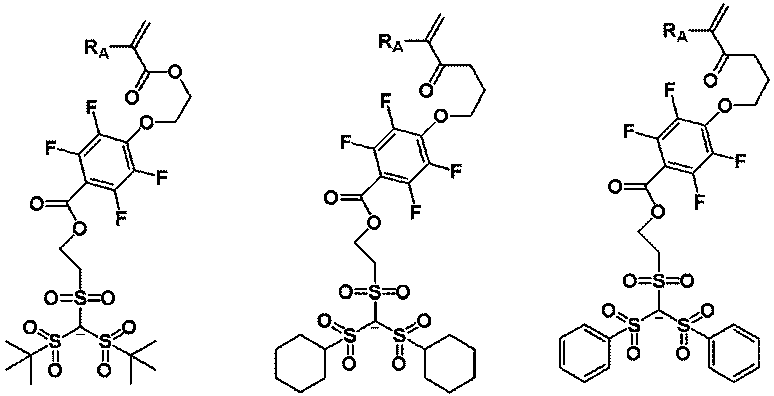

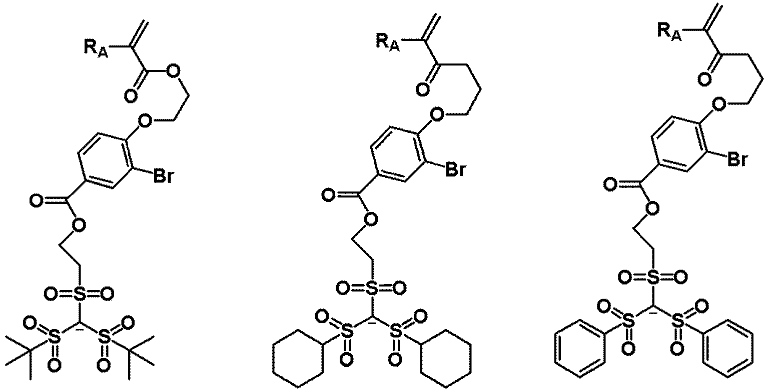

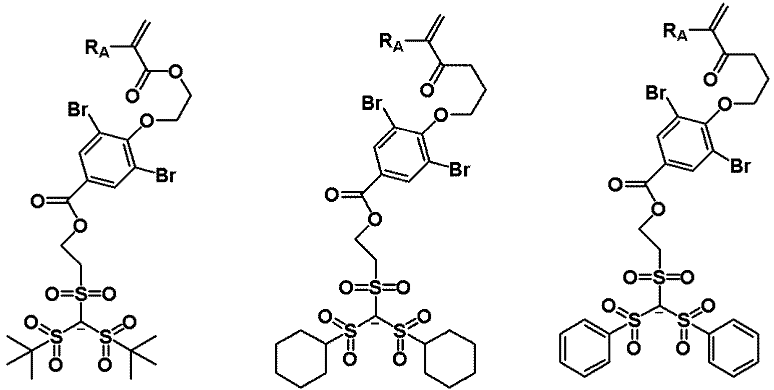

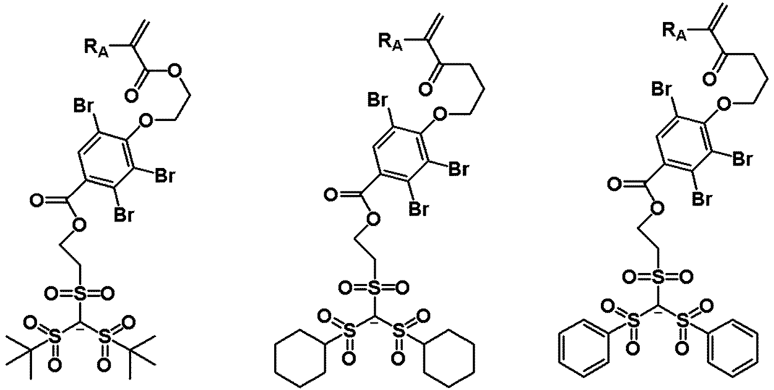

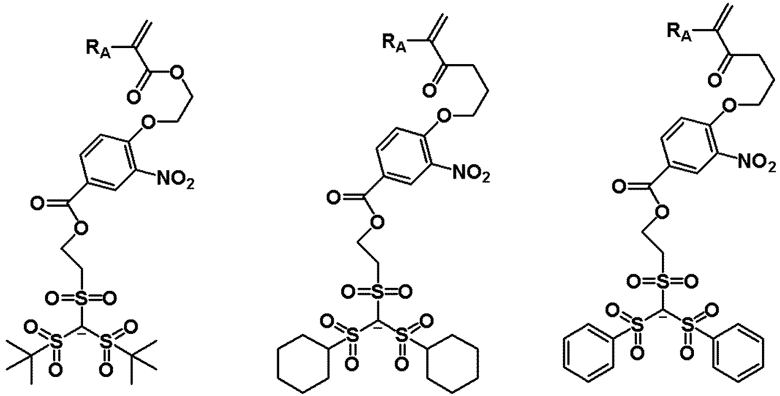

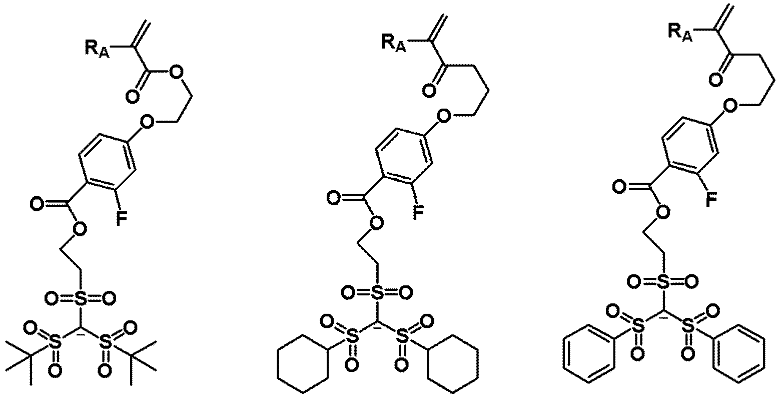

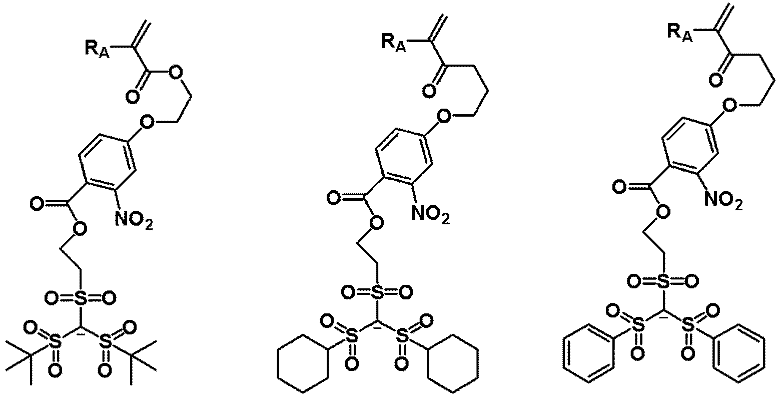

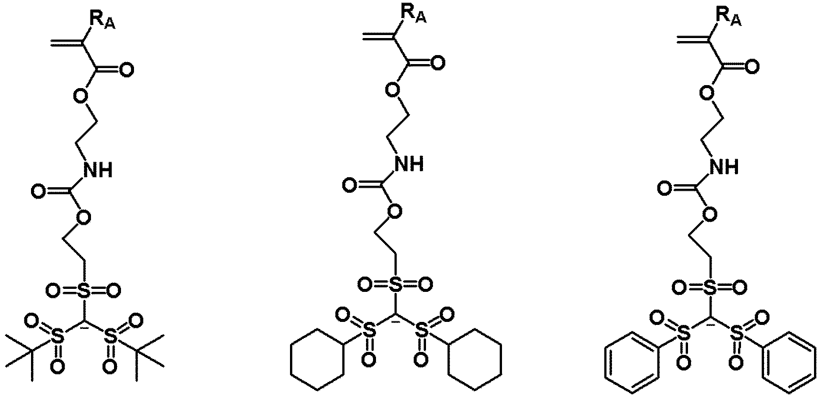

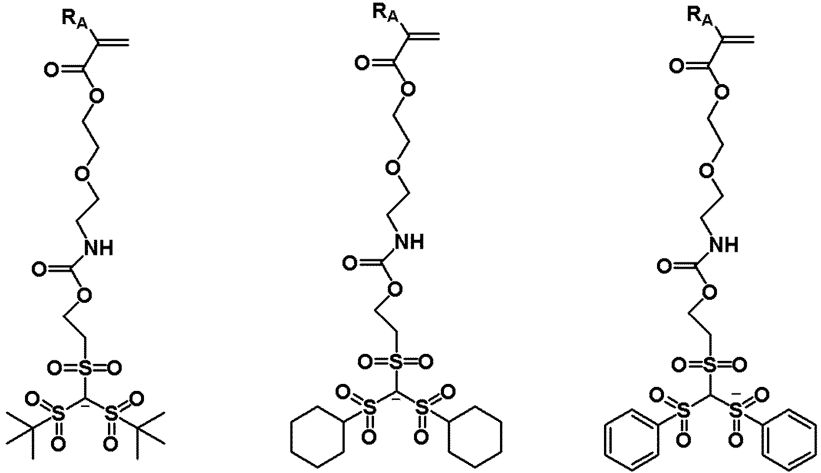

### (In the formulae, R^{A} is as described above.)

As a synthesis method of the above monomers, the method disclosed in JP 2020-055797 A can be employed.

Moreover, the component (A) preferably contains, as M⁺ in the repeating unit-a, an ammonium ion (ammonium cation) represented by the following general formula (2), 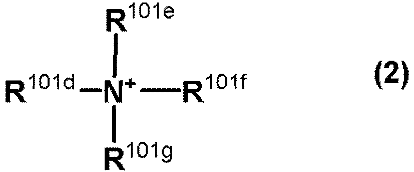 wherein each of R^{101d}, R^{101e}, R^{101f}, and R^{101g} represents a hydrogen atom, a linear, branched, or cyclic alkyl group having 1 to 12 carbon atoms, a linear, branched, or cyclic alkenyl group or alkynyl group having 2 to 12 carbon atoms, or an aromatic group having 4 to 20 carbon atoms, and optionally contains one or more selected from an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring with a nitrogen atom attached thereto, and when forming the ring, R^{101d} and R^{101e}, or R^{101d}, R^{101e}, and R^{101f} represent an alkylene group having 3 to 10 carbon atoms, or form a heteroaromatic ring having the nitrogen atom in the formula in the ring.

Specific examples of the ammonium ion represented by the above general formula (2) are shown below.

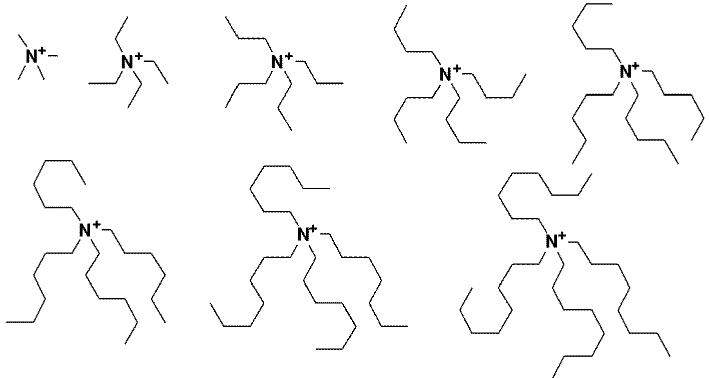

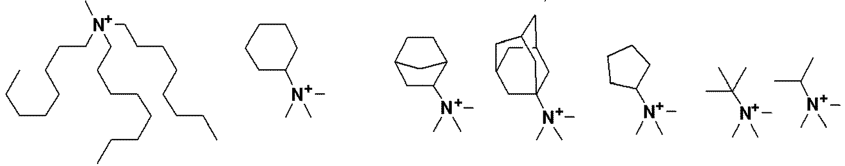

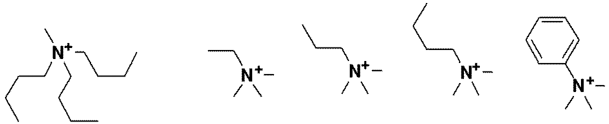

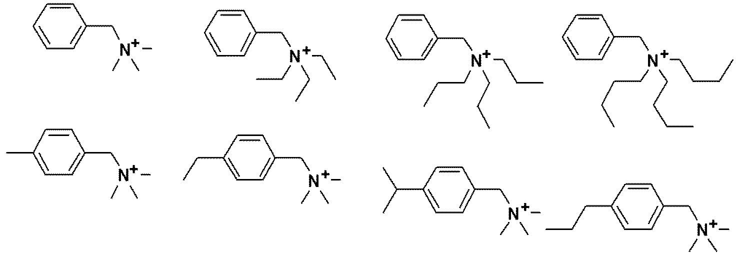

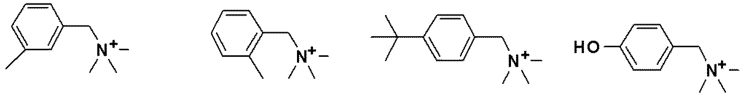

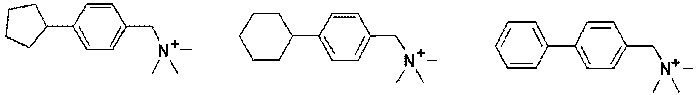

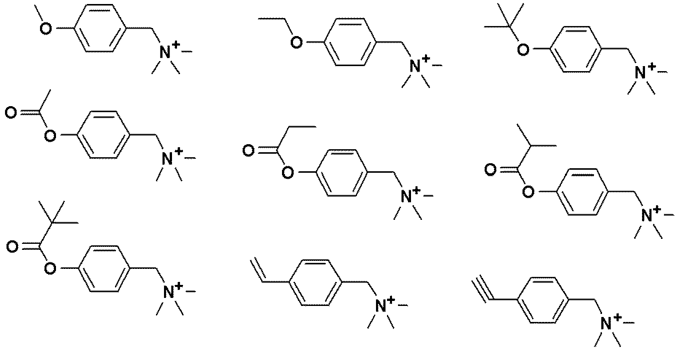

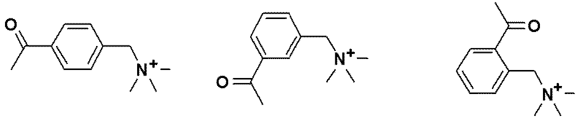

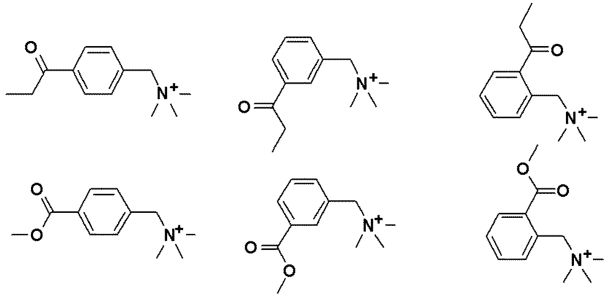

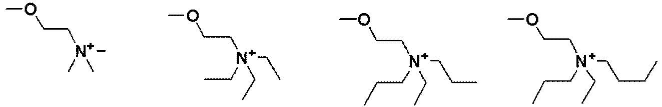

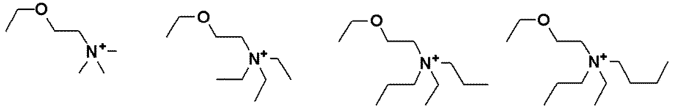

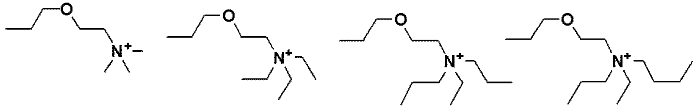

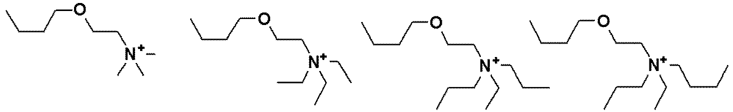

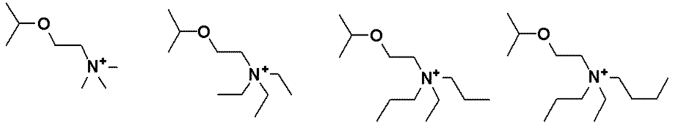

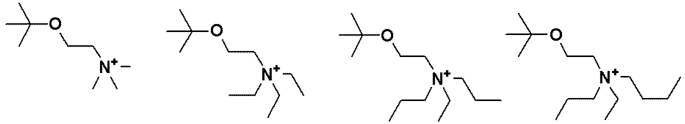

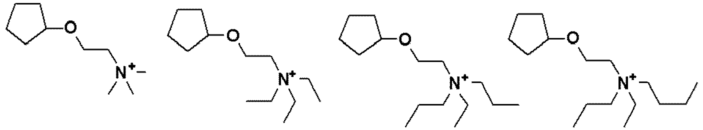

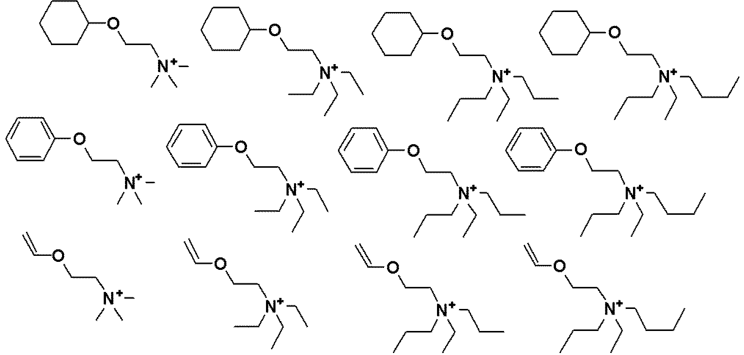

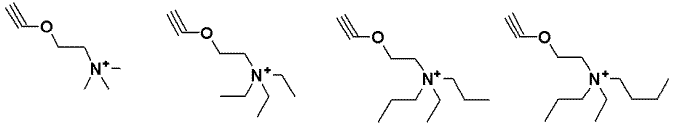

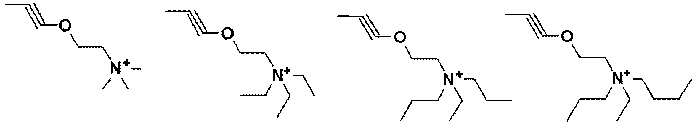

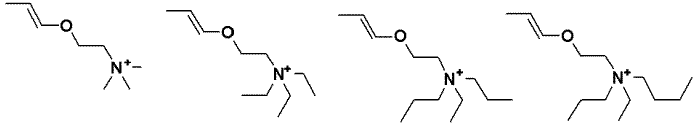

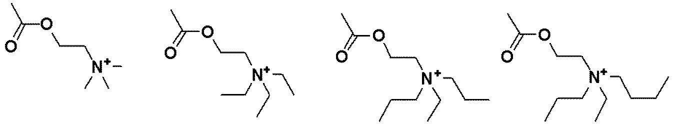

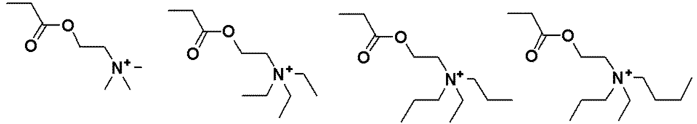

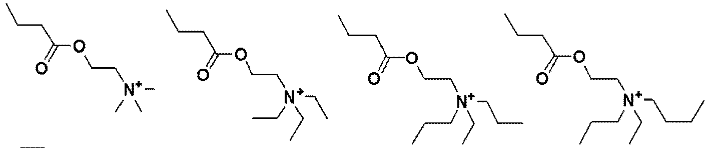

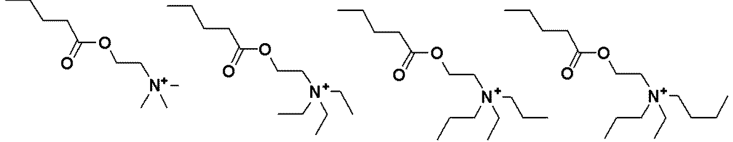

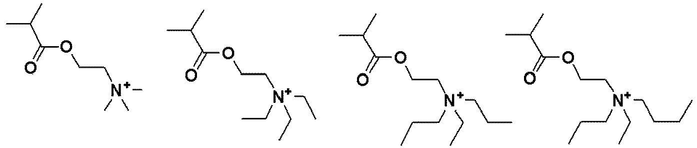

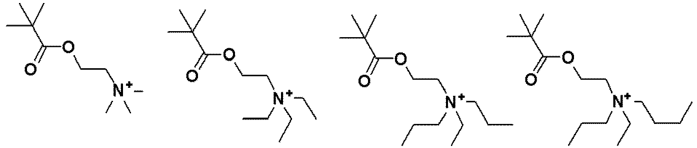

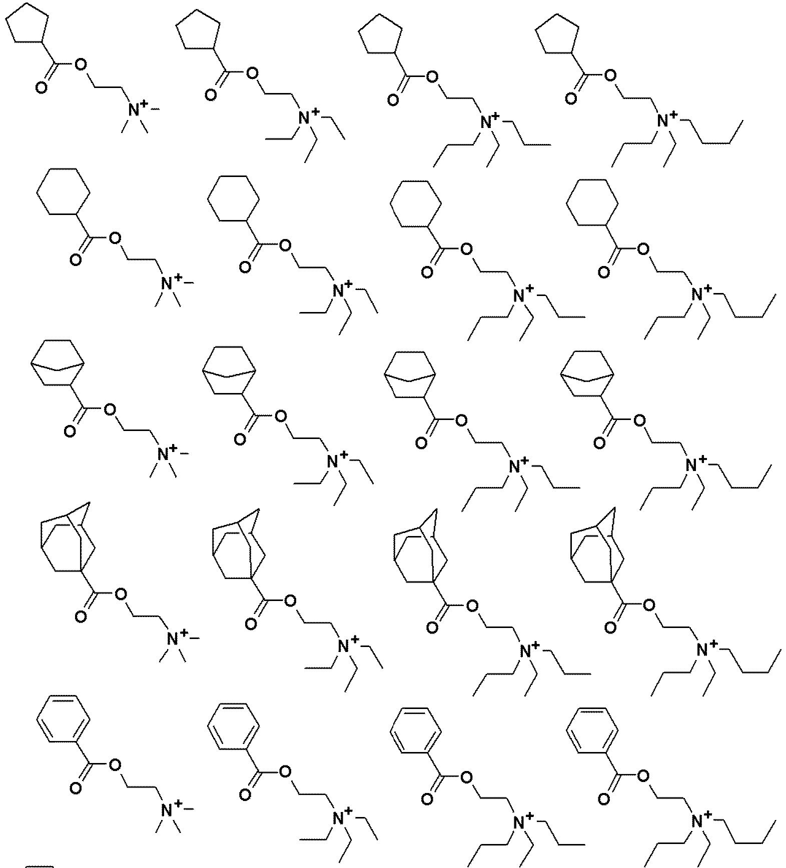

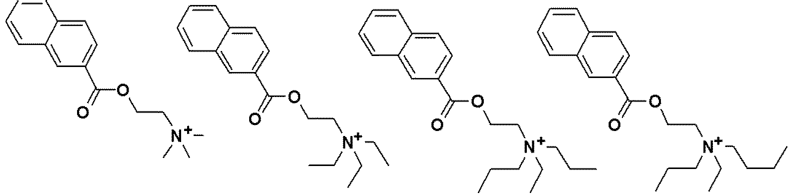

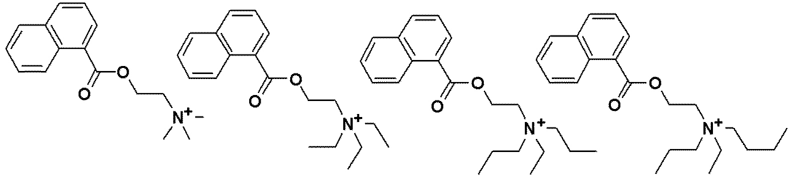

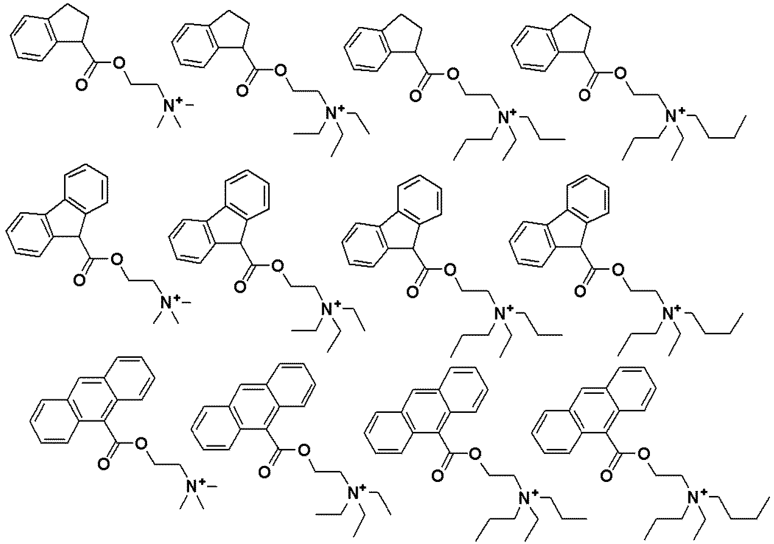

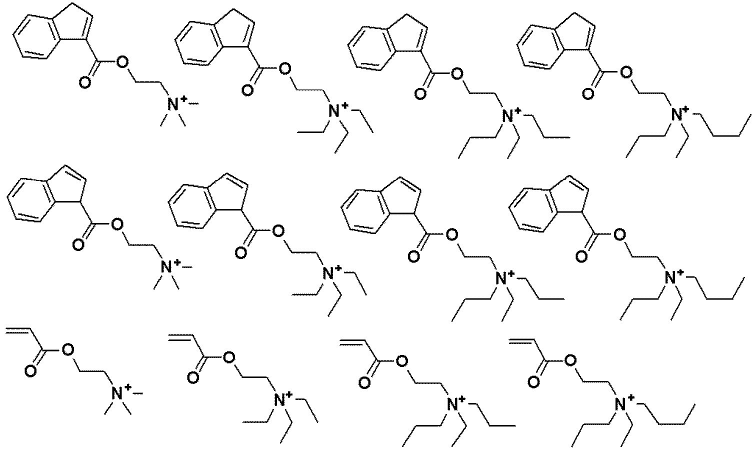

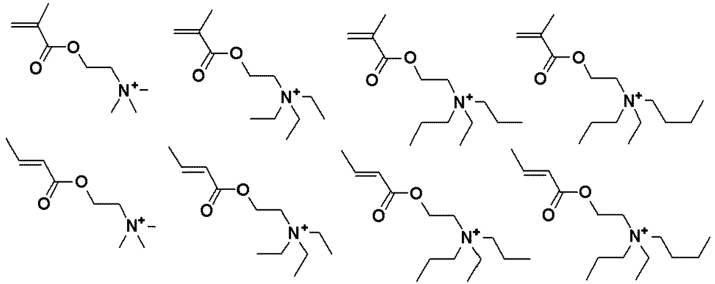

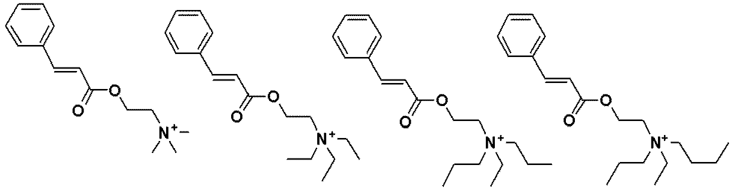

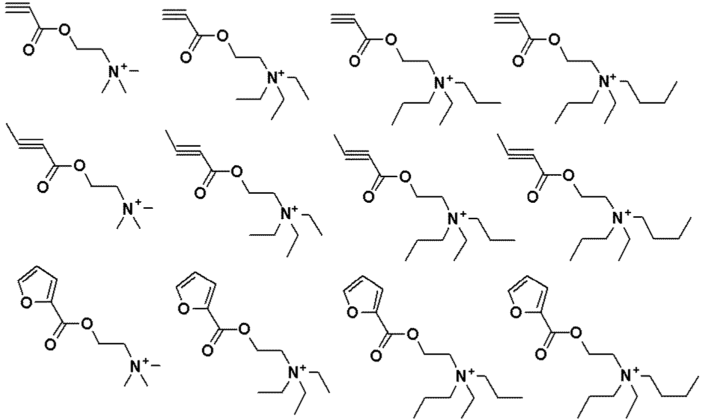

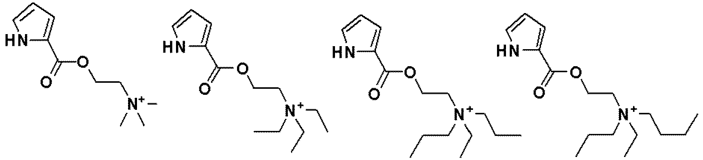

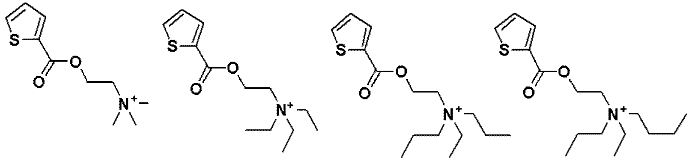

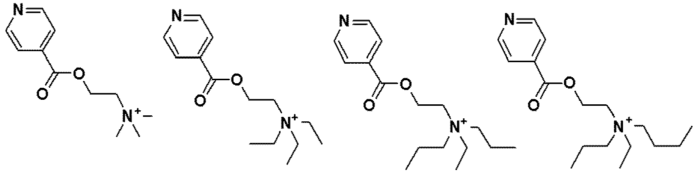

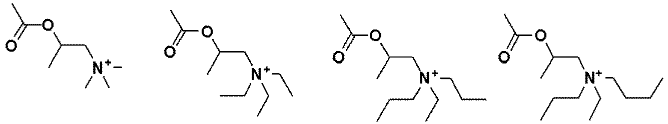

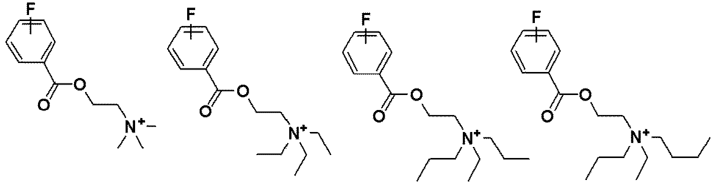

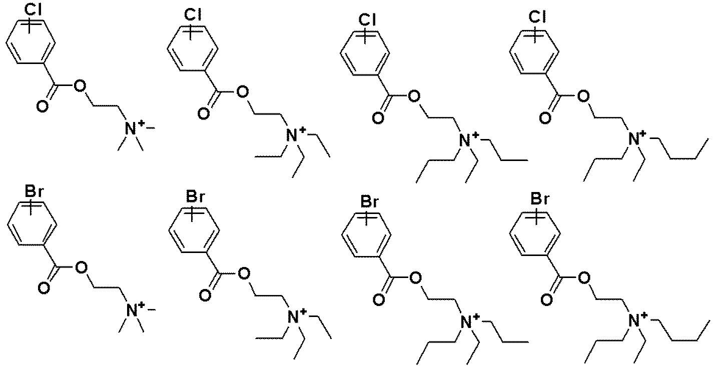

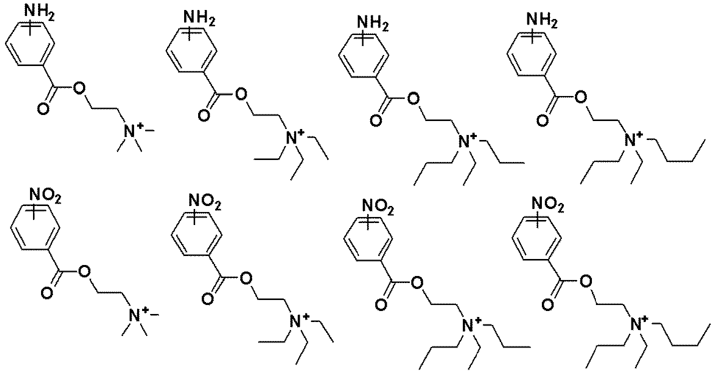

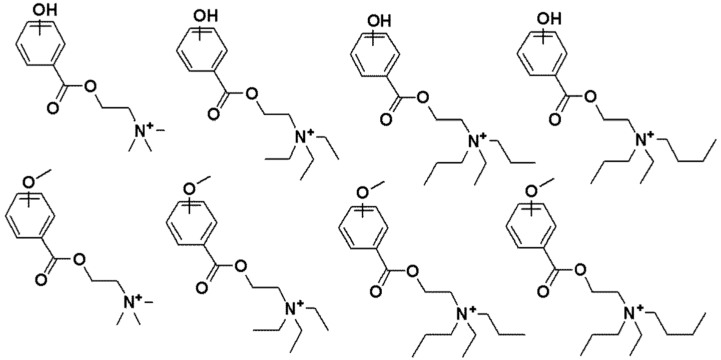

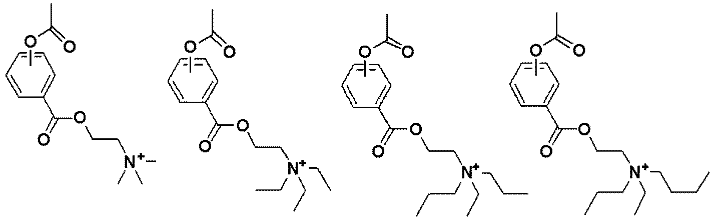

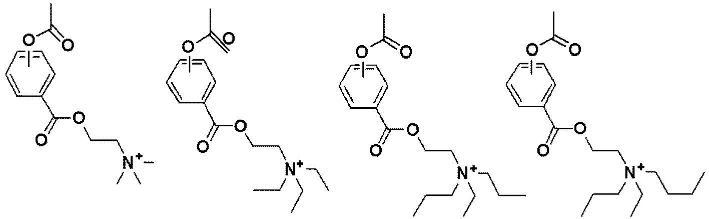

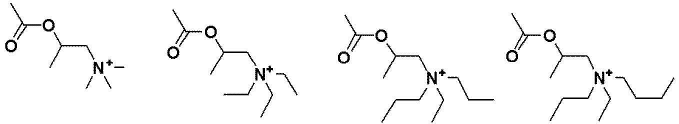

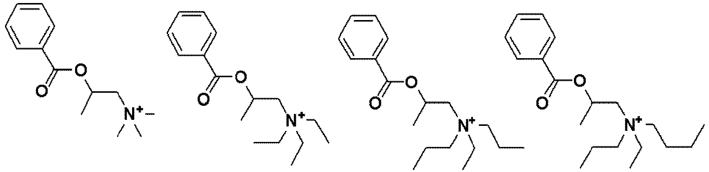

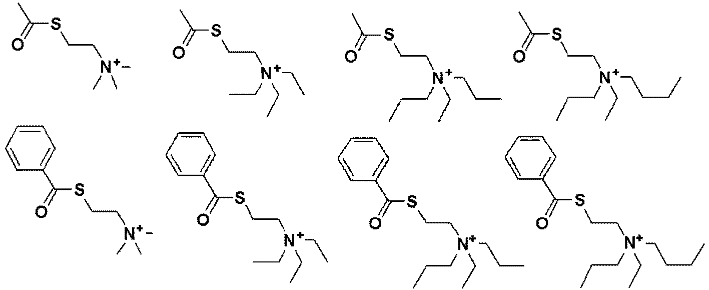

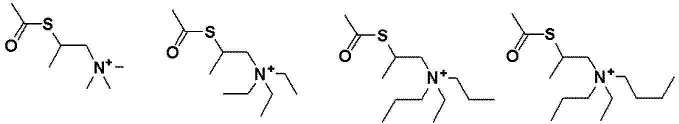

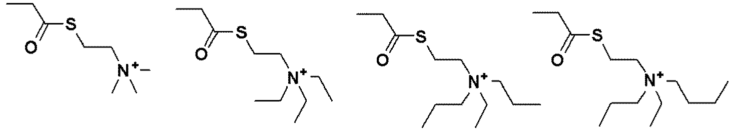

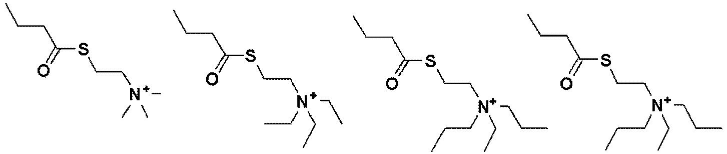

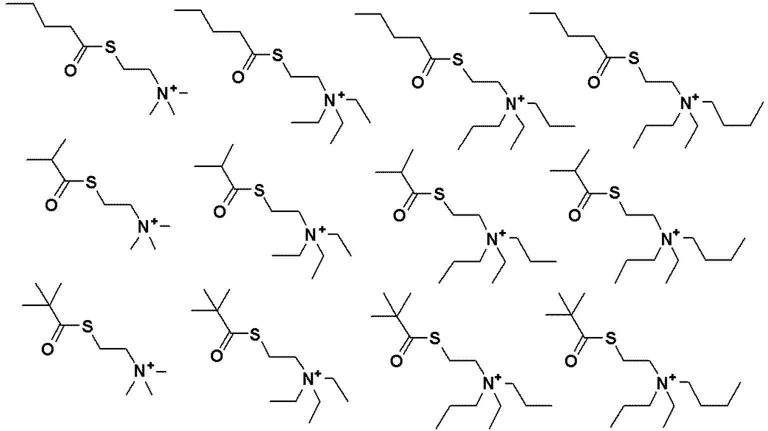

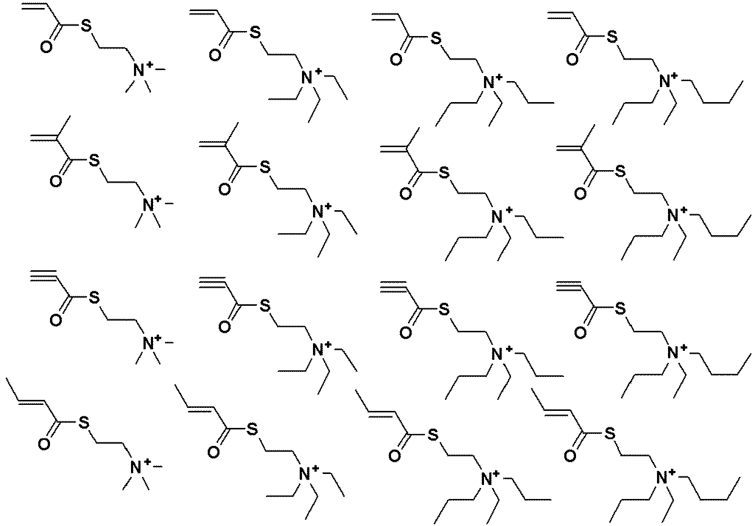

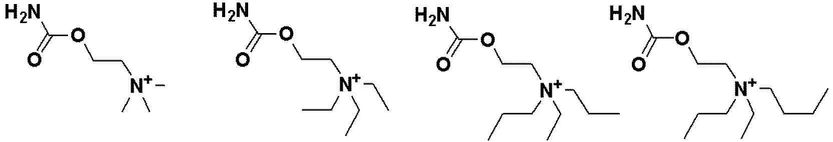

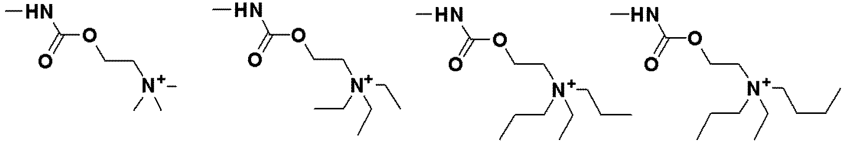

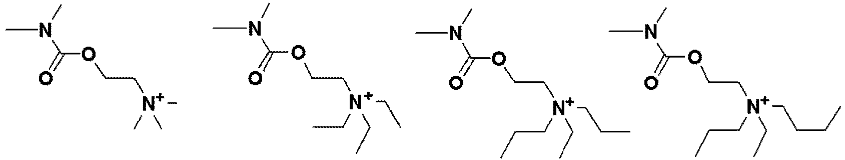

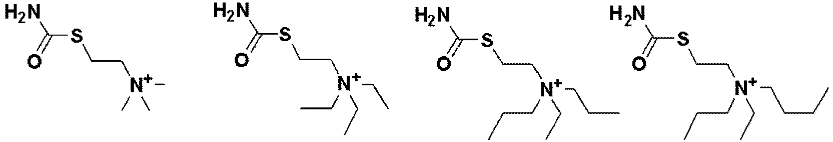

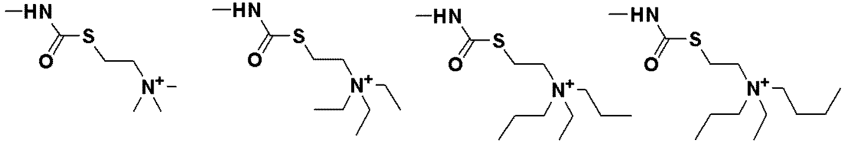

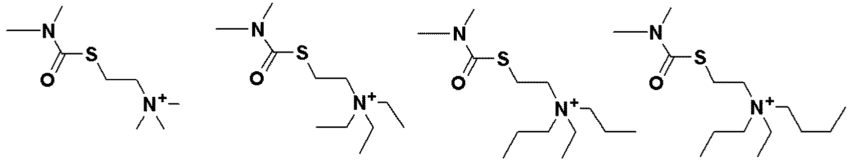

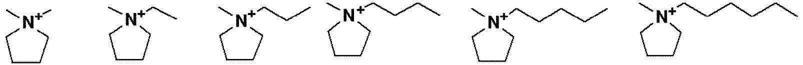

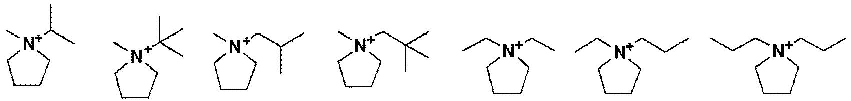

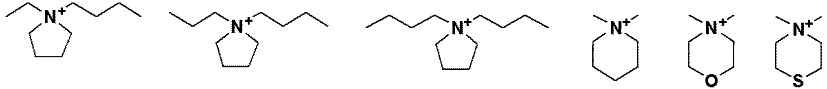

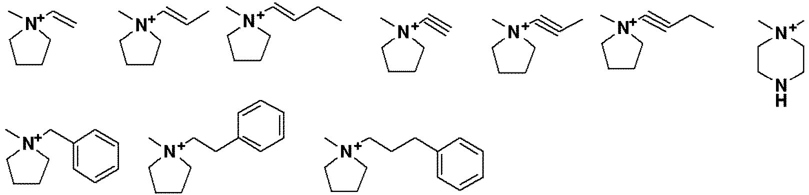

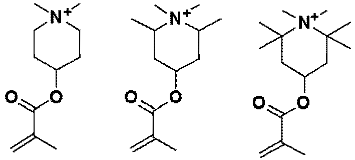

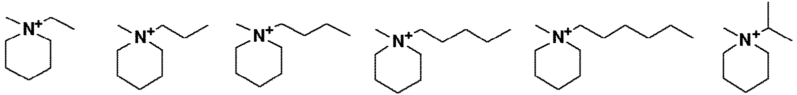

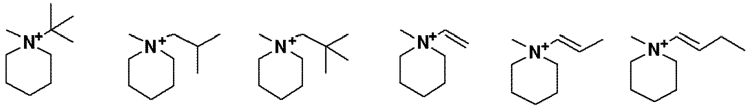

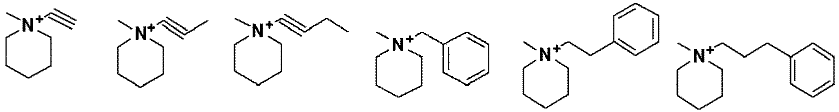

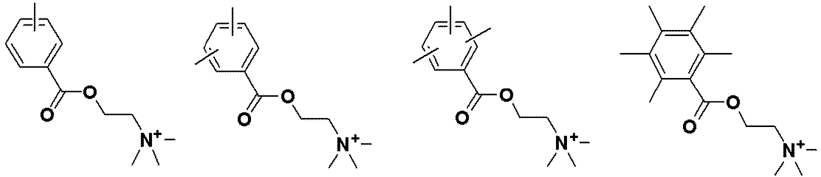

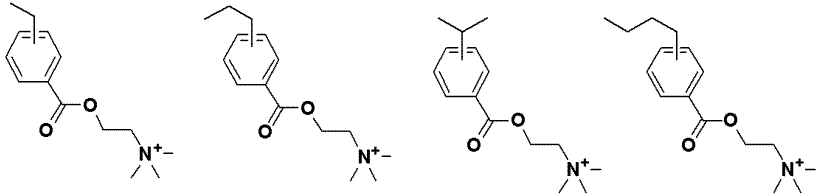

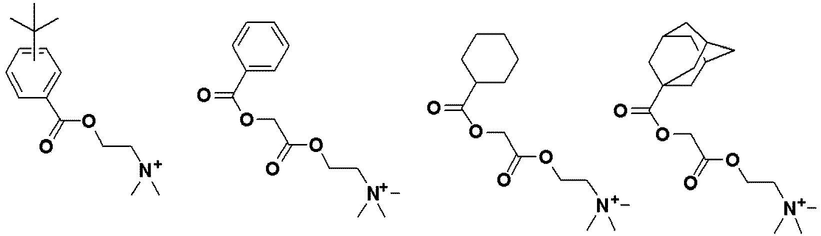

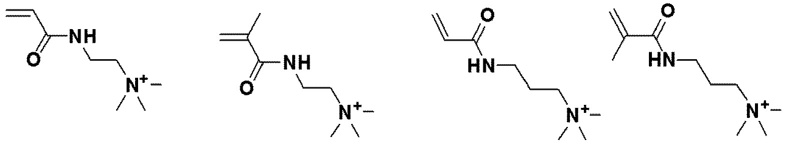

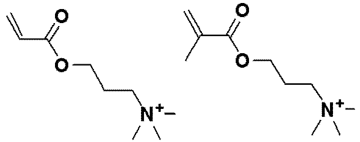

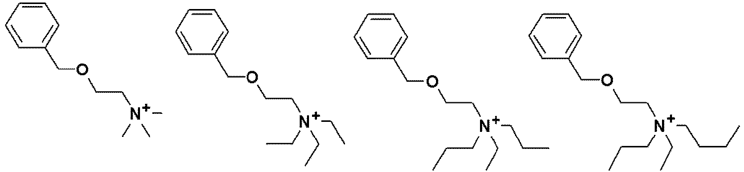

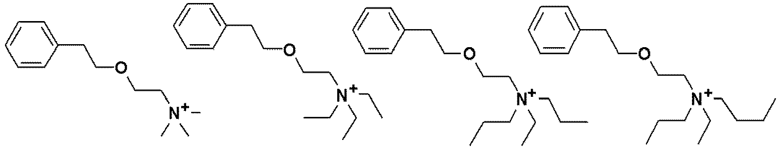

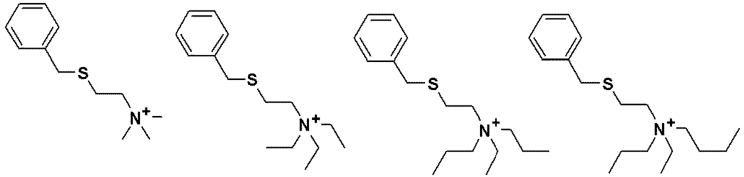

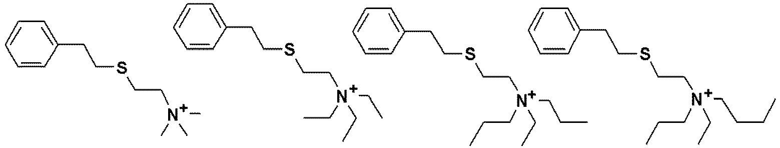

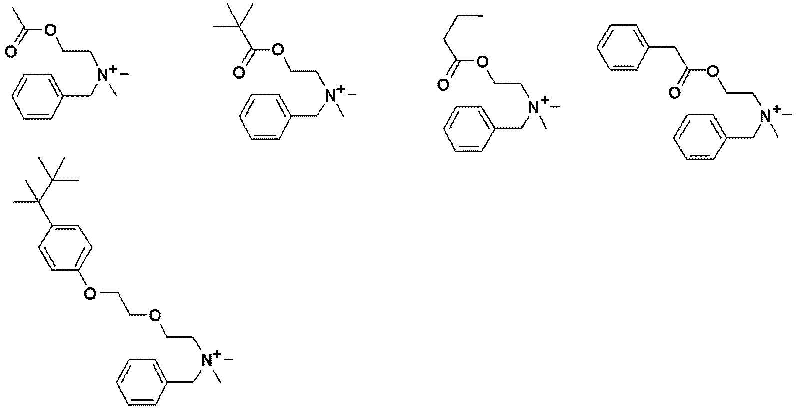

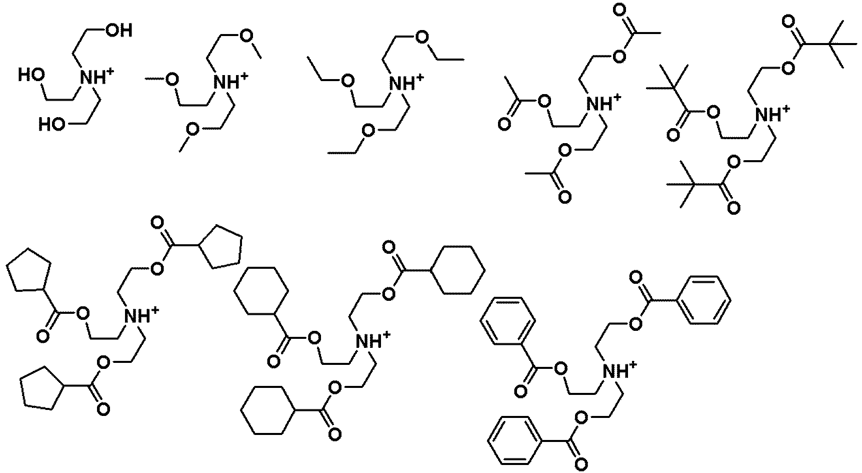

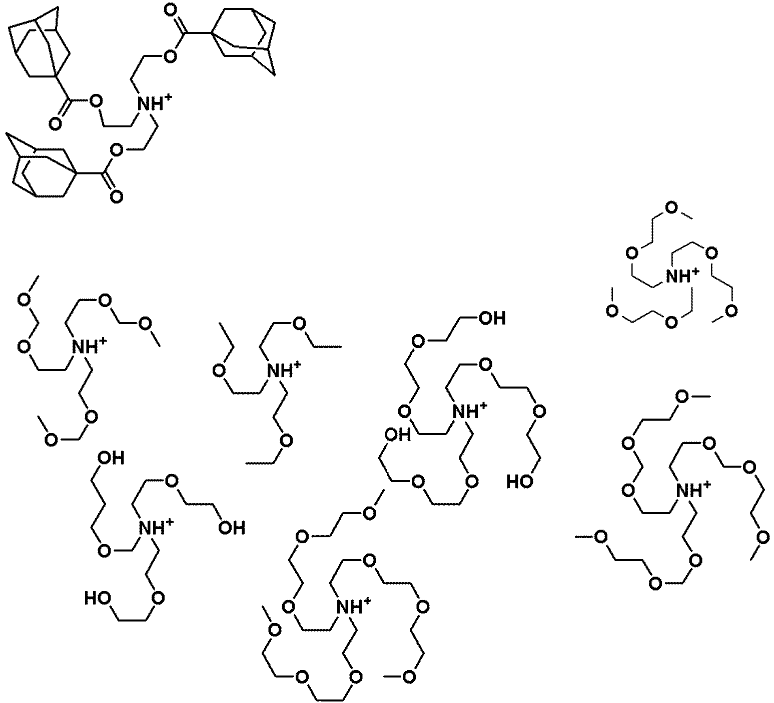

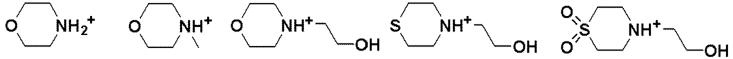

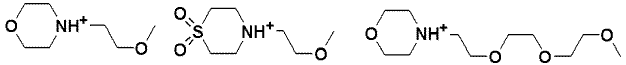

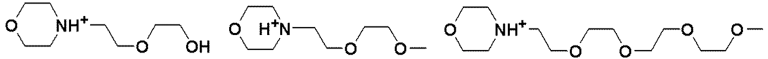

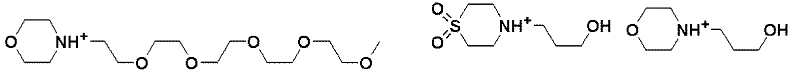

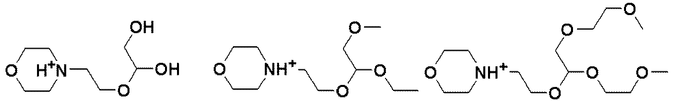

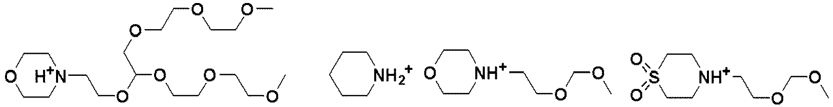

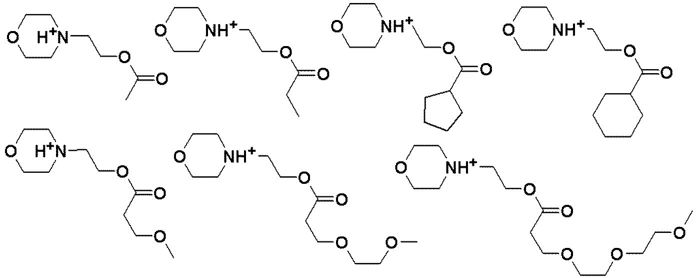

As the ammonium ion represented by the above general formula (2), a tertiary or quaternary ammonium ion is particularly preferable.

### (Repeating Unit-b)

In the component (A) of the bio-electrode composition of the present invention, a repeating unit-b having a glyme chain can also be copolymerized in addition to the above repeating unit-a obtained by polymerization of a monomer having a polymerizable double bond, in order to improve electric conductivity. Specific examples of a monomer to give the repeating unit-b having a glyme chain are shown below. The copolymerization with the repeating unit having a glyme chain can facilitate movement of ions released from skin in a dry electrode film, thereby enhancing sensitivity of the dry electrode.

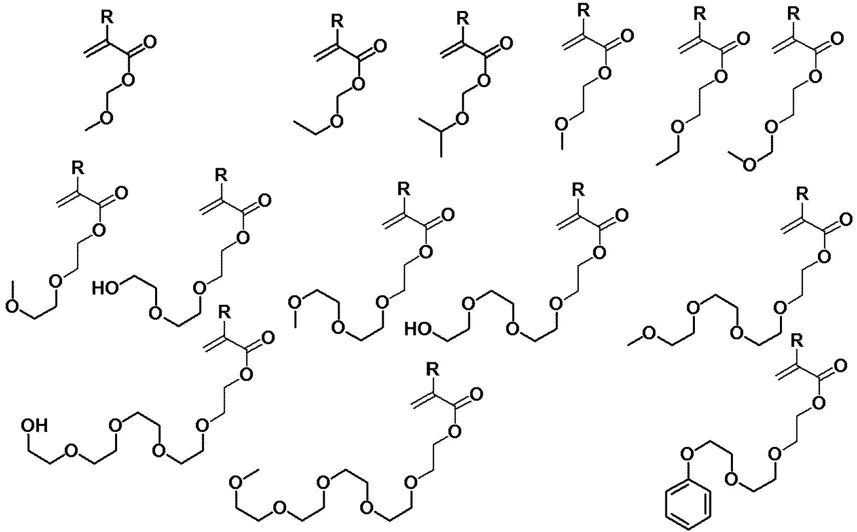

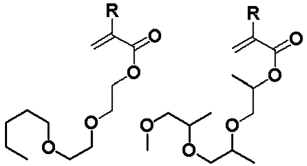

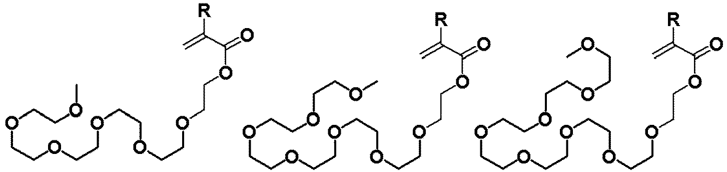

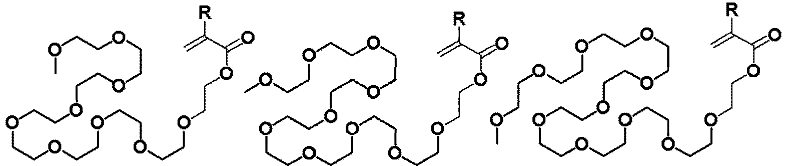

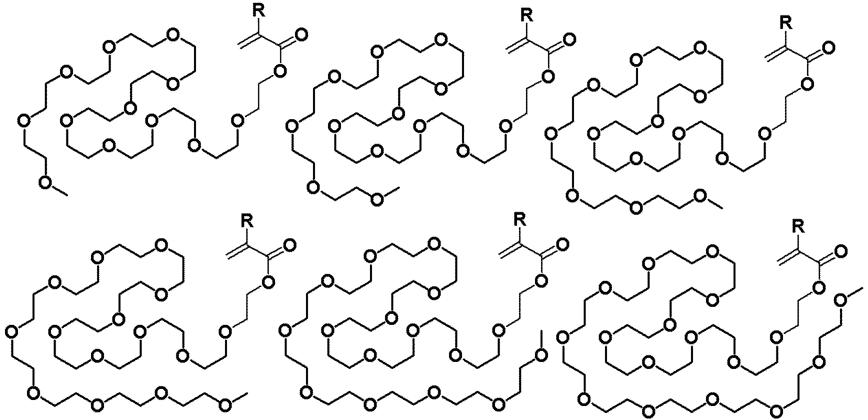

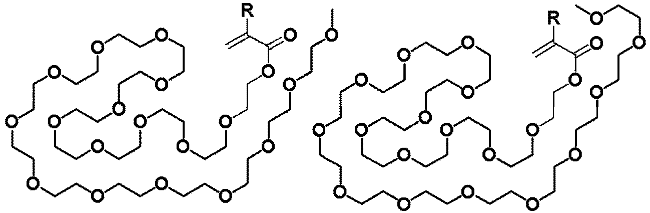

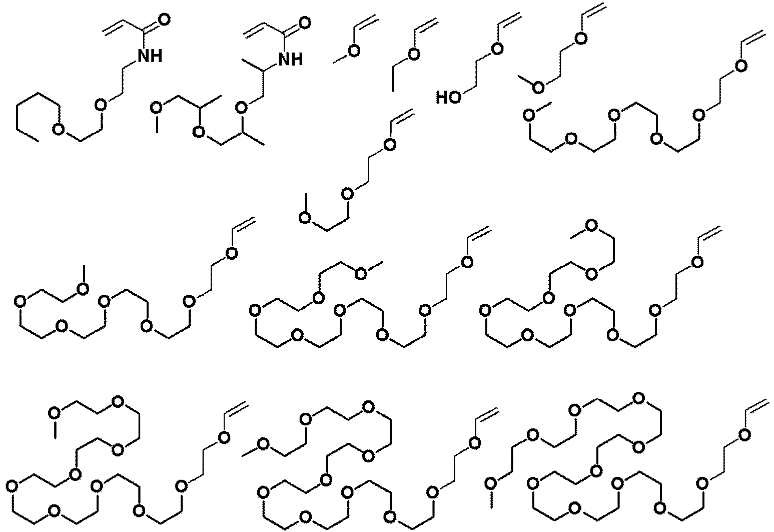

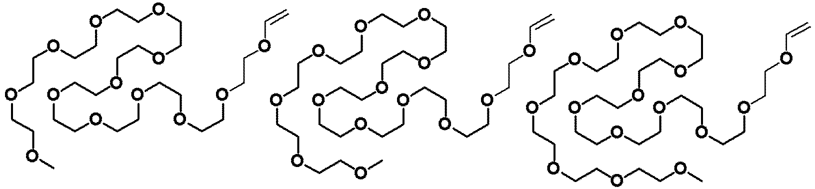

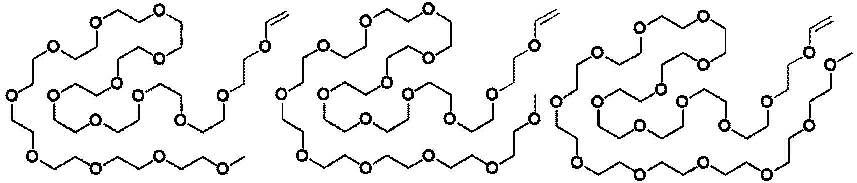

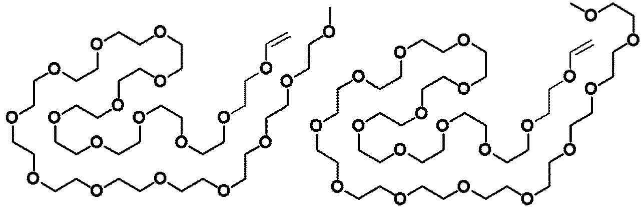

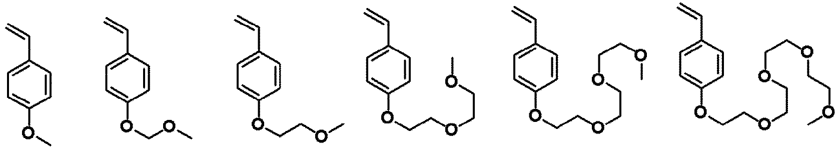

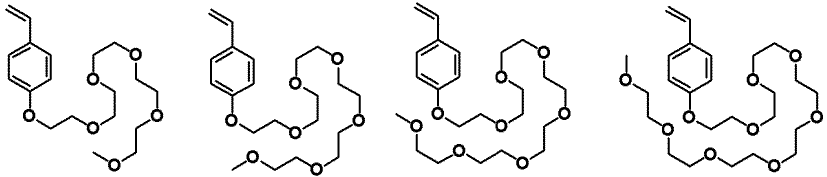

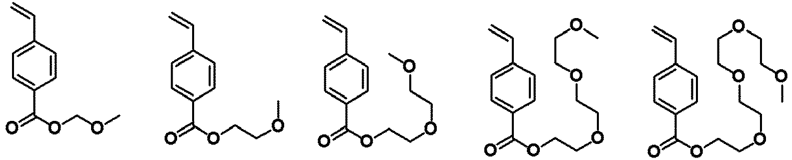

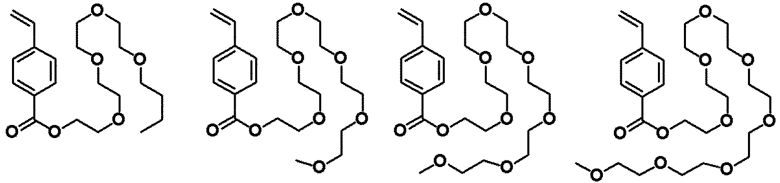

### (R represents a hydrogen atom or a methyl group.)

### (Repeating Unit-c)

In the component (A) of the bio-electrode composition of the present invention, a hydrophilic repeating unit-c having a hydroxy group, a carboxyl group, an ammonium salt, betaine, an amide group, pyrrolidone, a lactone ring, a lactam ring, a sultone ring, a sulfonic acid sodium salt, or a sulfonic acid potassium salt can also be copolymerized in addition to the above repeating units-a and -b, in order to improve the electric conductivity. Specific examples of a monomer to give the hydrophilic repeating unit-c are shown below. The copolymerization with the repeating unit containing these hydrophilic groups can enhance sensitivity to ions released from skin, thereby enhancing the sensitivity of the dry electrode.

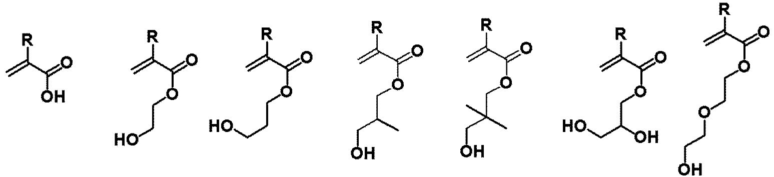

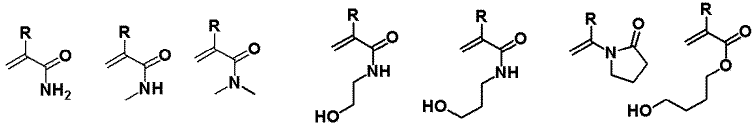

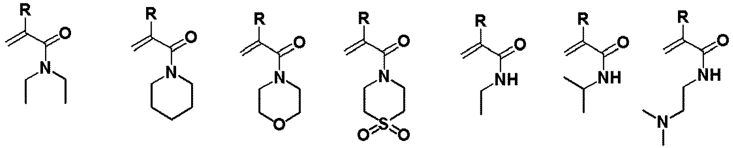

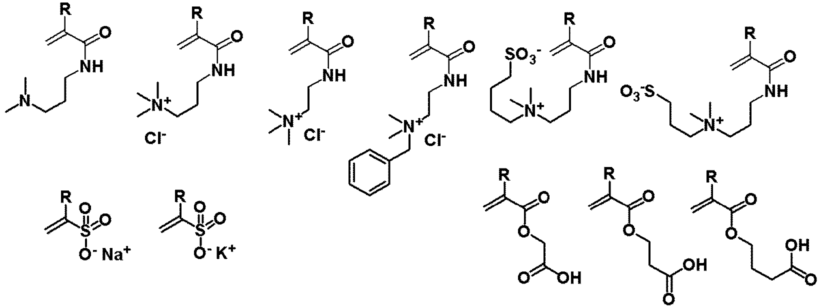

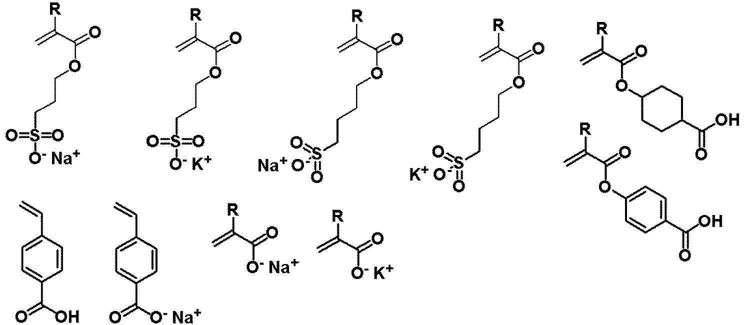

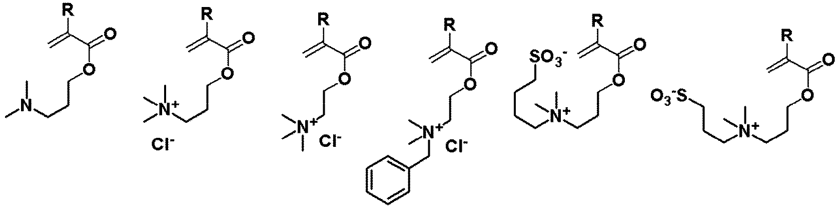

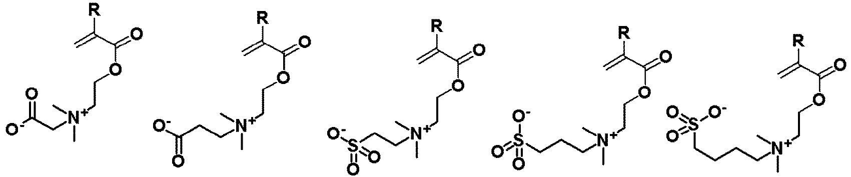

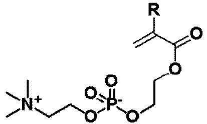

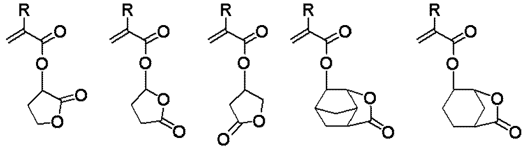

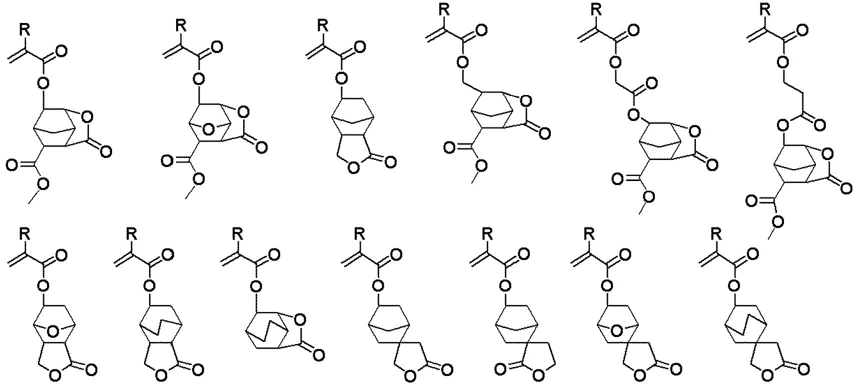

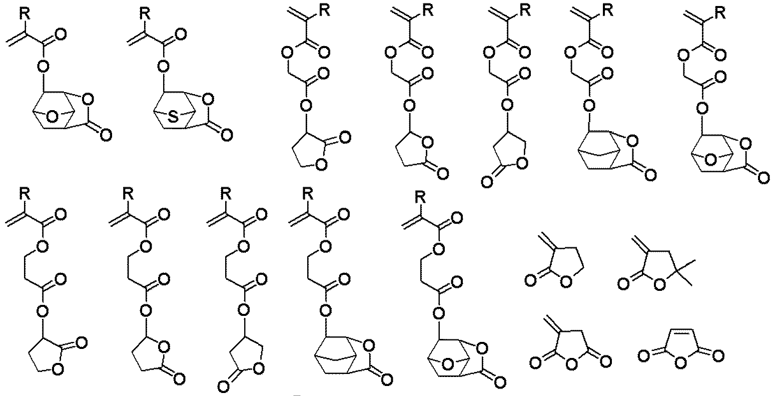

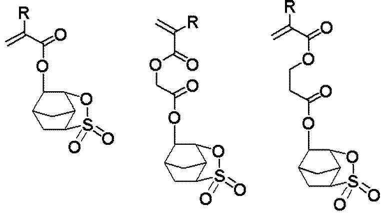

### (R represents a methyl group or a hydrogen atom.)

### (Repeating Unit-d)

The ionic resin (A) of the bio-electrode composition of the present invention can contain a repeating unit-d for imparting adhesion ability.

Specific examples of a monomer to give the repeating unit-d are shown below.

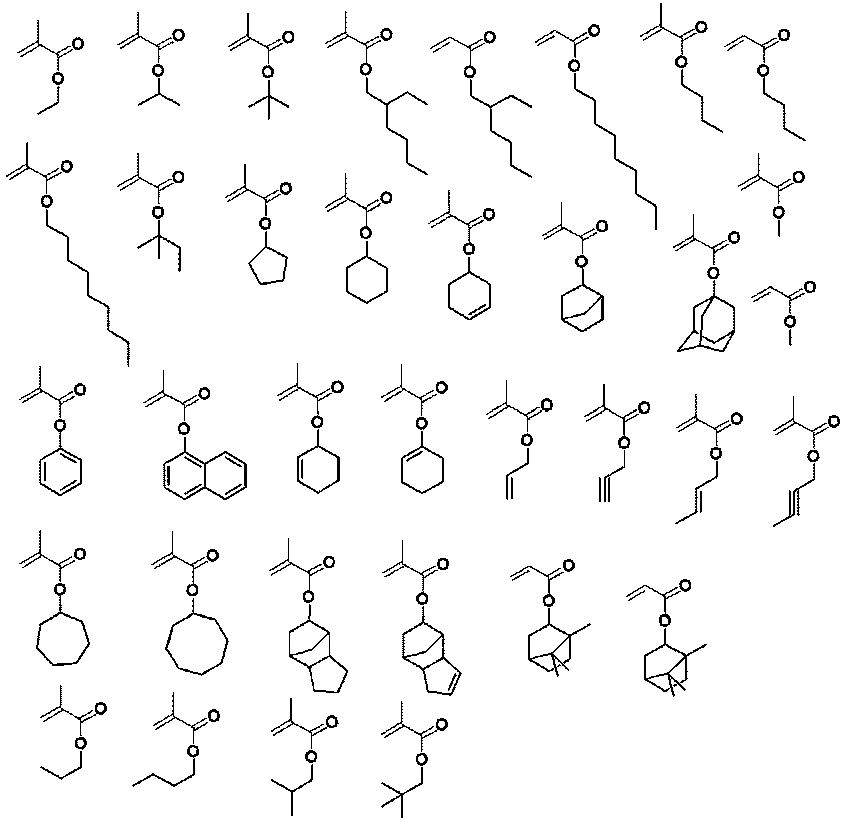

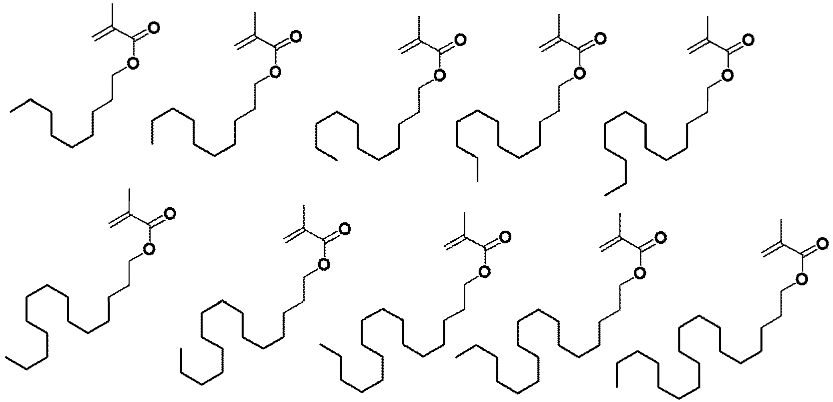

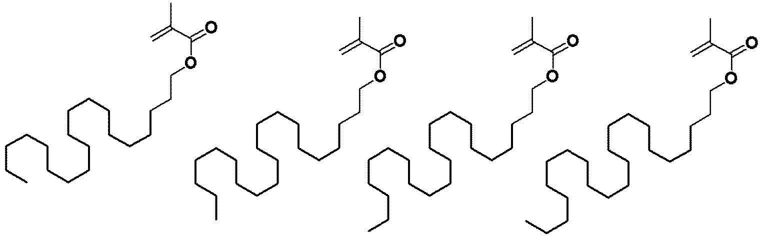

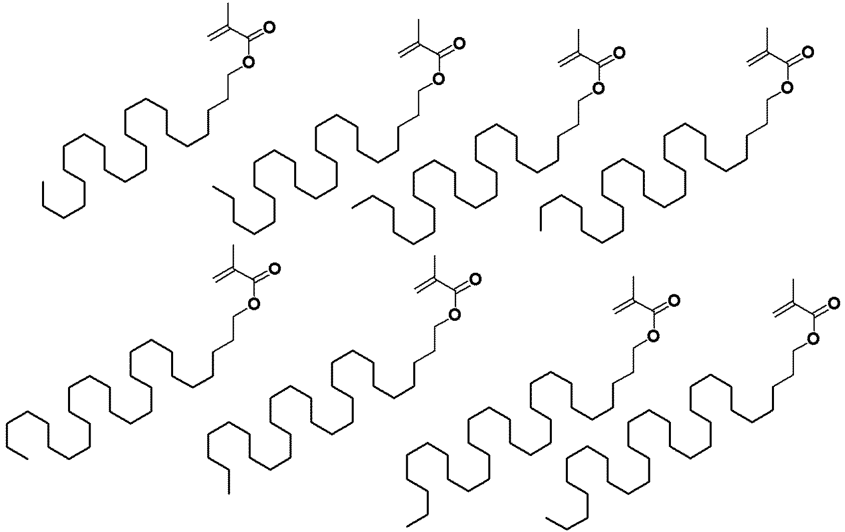

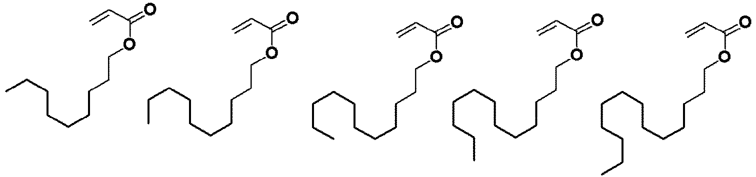

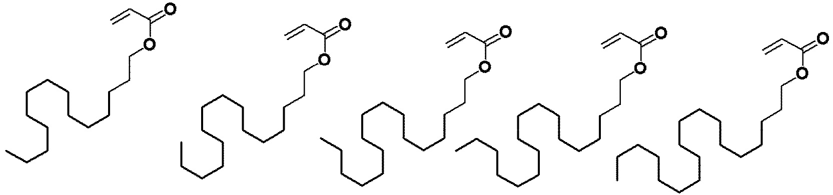

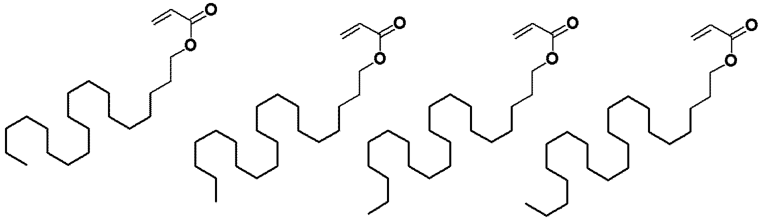

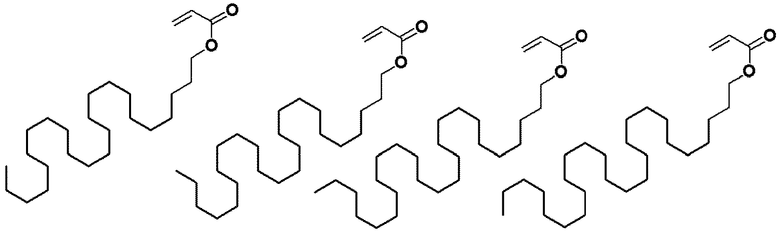

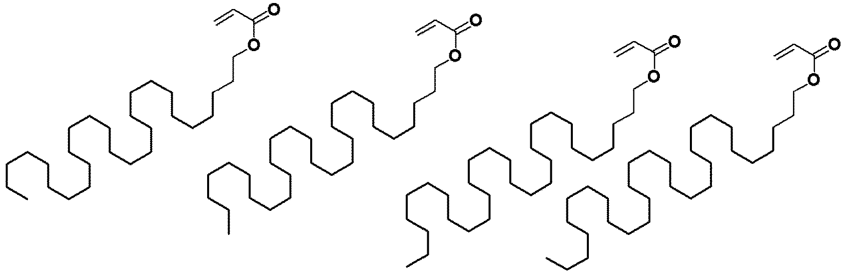

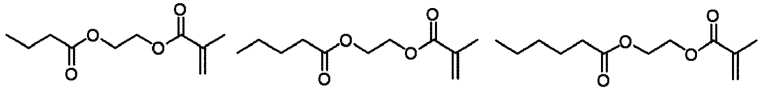

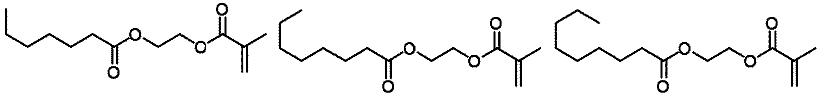

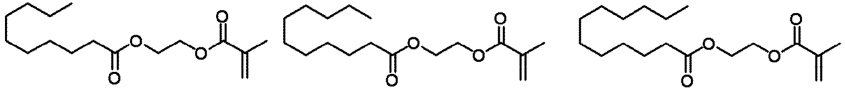

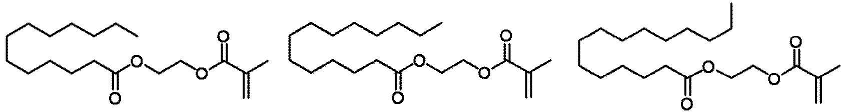

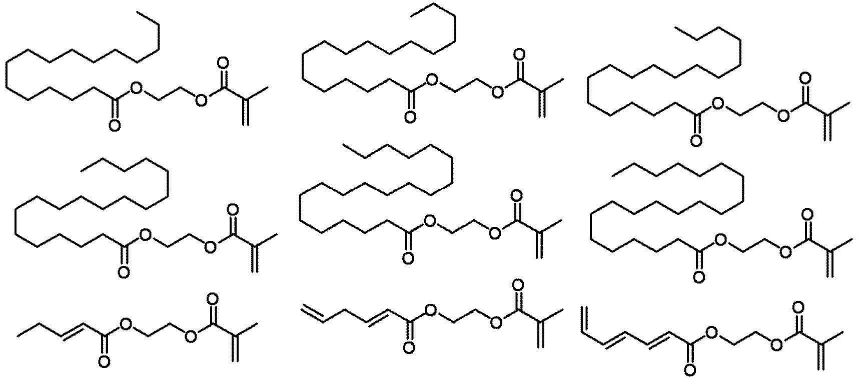

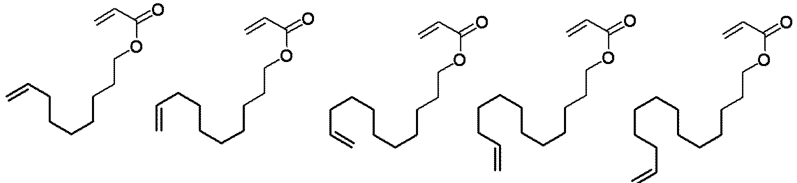

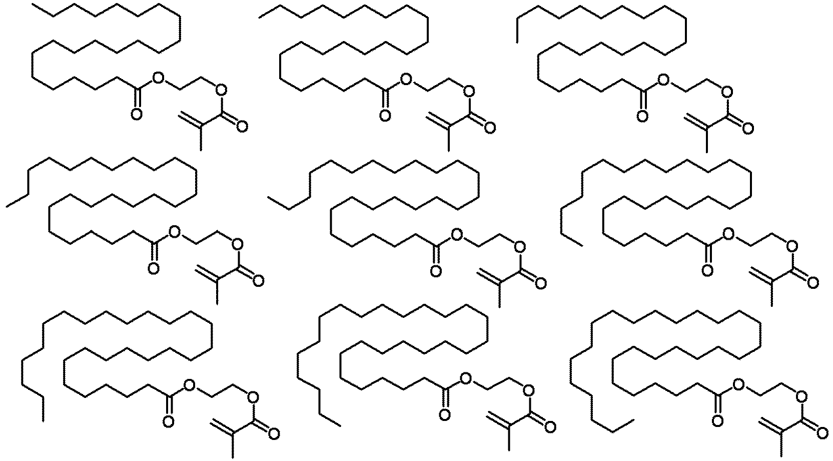

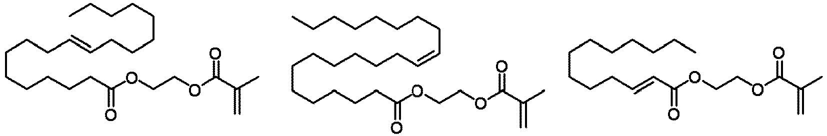

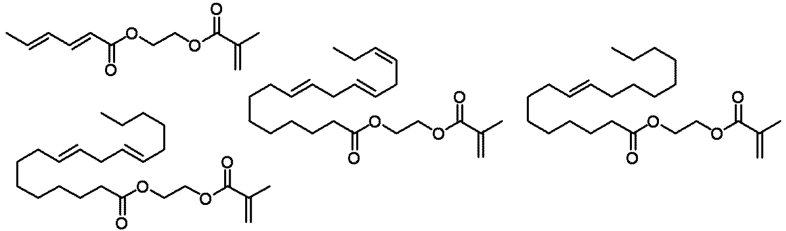

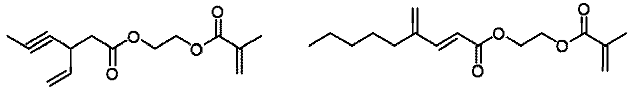

### (Repeating Unit-e)

Furthermore, it is also possible to copolymerize a crosslinkable repeating unit-e. Examples of the crosslinkable repeating unit include repeating units having an oxirane ring or an oxetane ring.

Specific examples of a monomer to give the repeating unit-e having an oxirane ring or an oxetane ring are shown below.

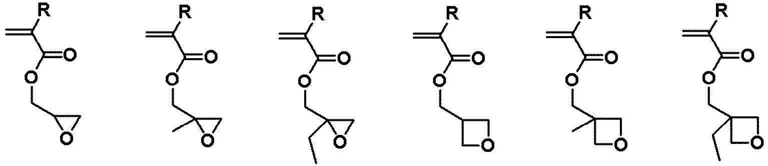

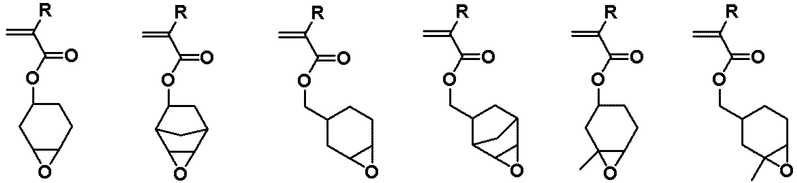

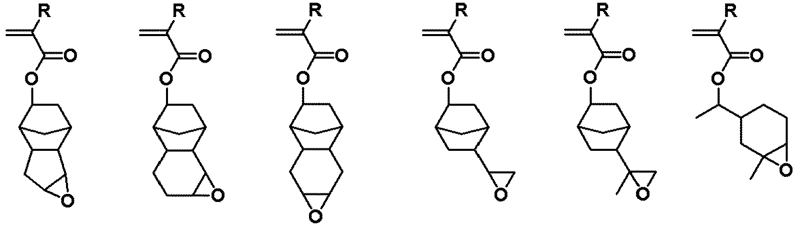

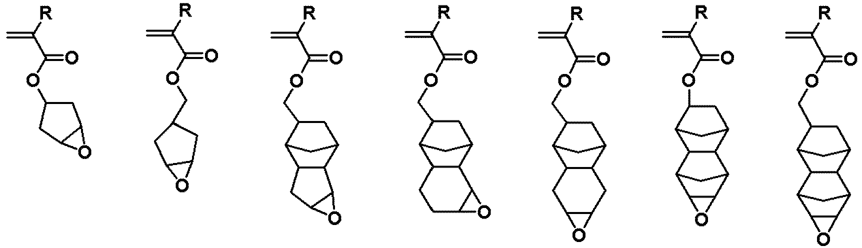

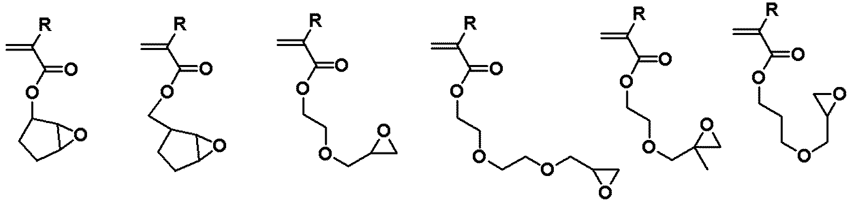

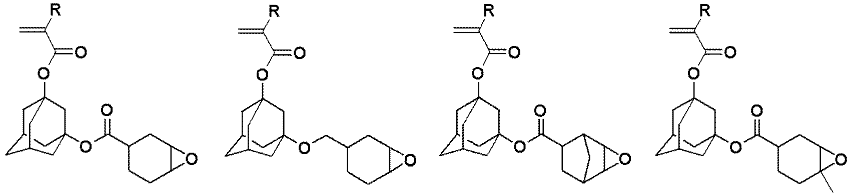

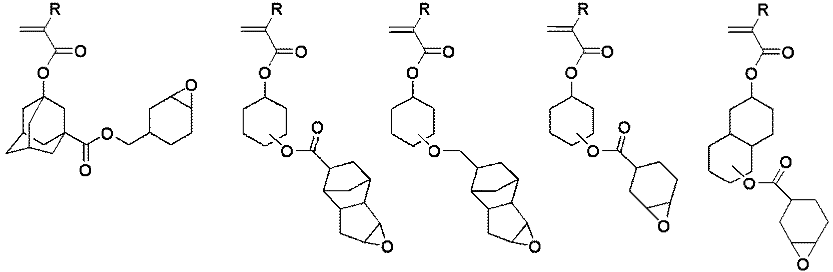

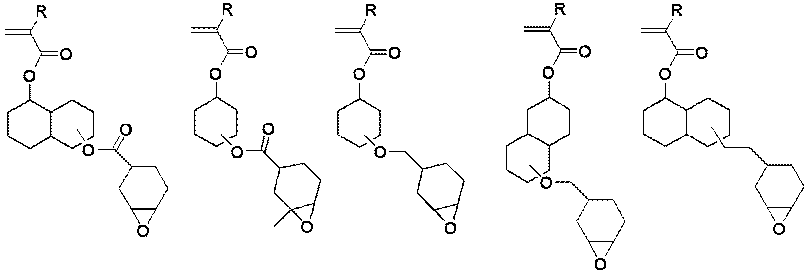

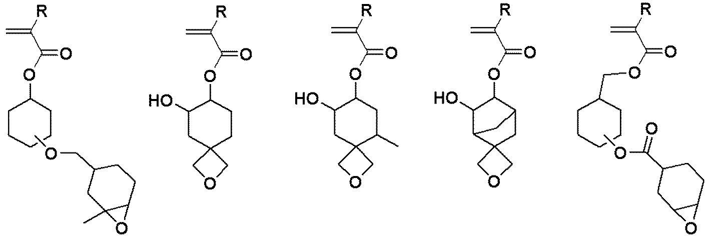

### (R represents a methyl group or a hydrogen atom.)

### (Repeating Unit-f)

The component (A) of the bio-electrode composition of the present invention can contain a repeating unit-f having silicon, in addition to the repeating units selected from the above repeating units-a, -b, -c, -d, and -e. Specific examples thereof include the following.

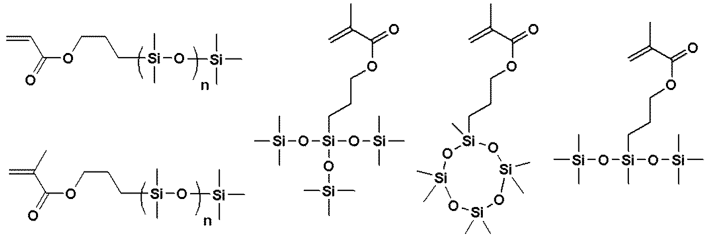

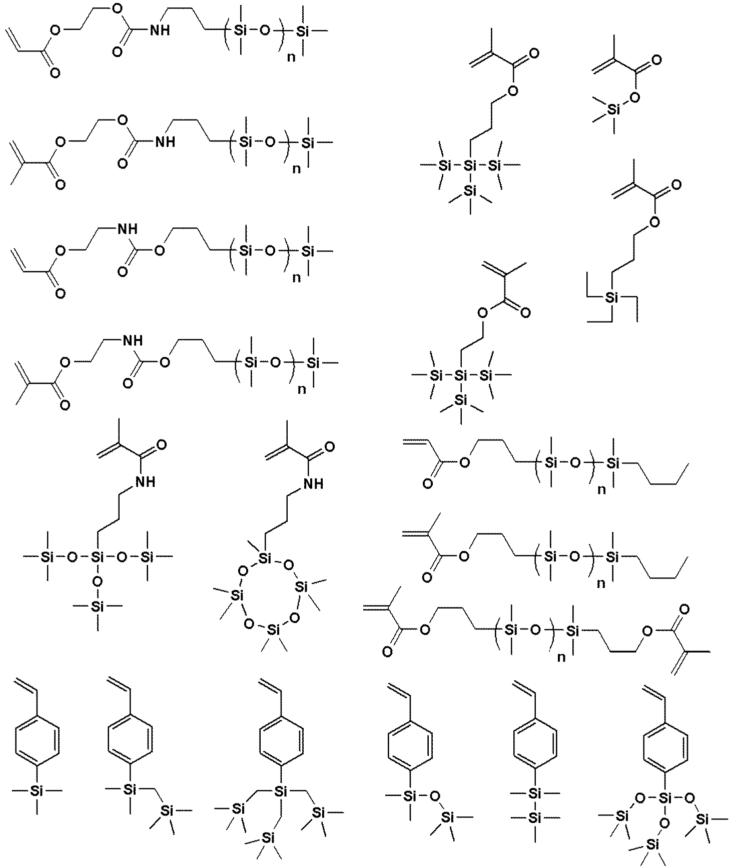

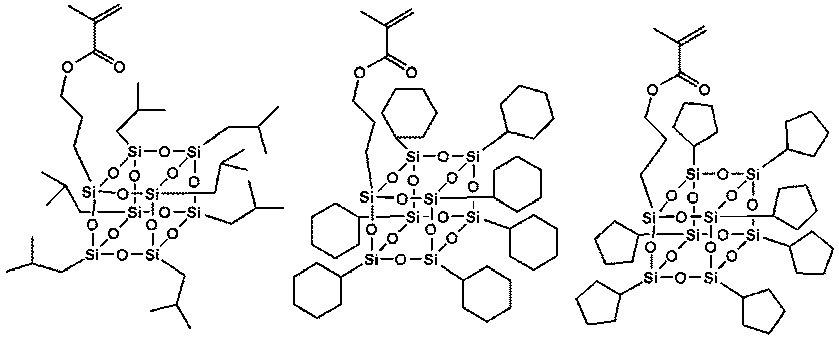

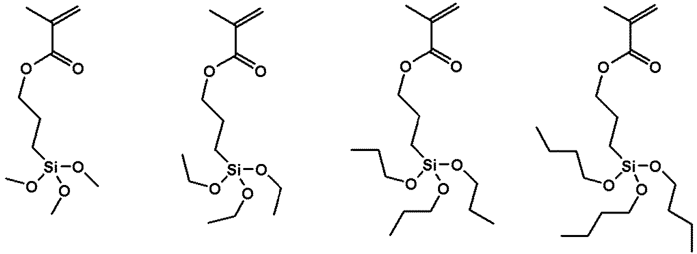

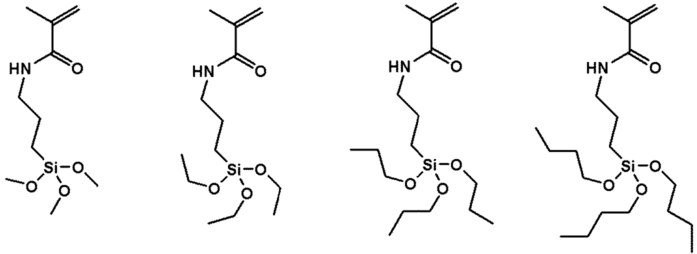

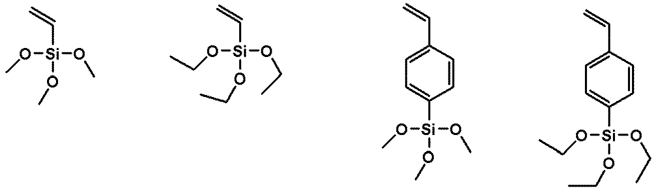

A silsesquioxane structure may be formed by copolymerizing the above repeating unit having an alkoxysilyl group and then hydrolyzing the alkoxysilyl group as disclosed in JP 2022-164579 A, or a complex with silica may be formed by reaction with silanol on the silica surface as disclosed in JP 2022-64291 A.

### (Repeating Unit-g)

The component (A) of the bio-electrode composition of the present invention can contain a repeating unit-g having fluorine, in addition to the repeating units selected from the above repeating units-a and -b to -f.

Specific examples of a monomer to give the repeating unit-g having fluorine are shown below.

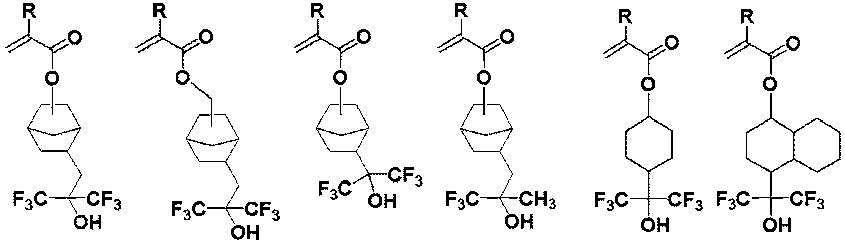

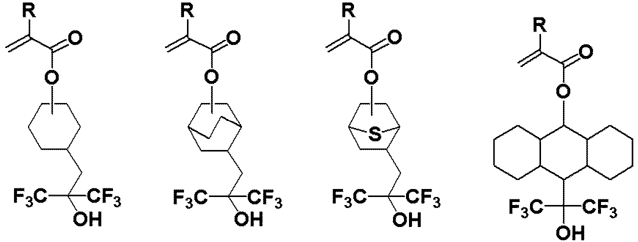

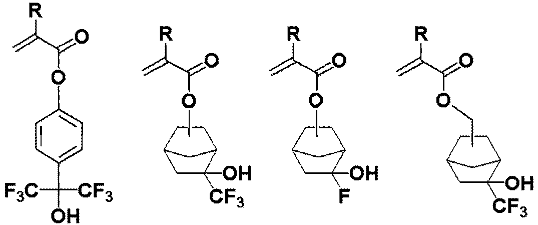

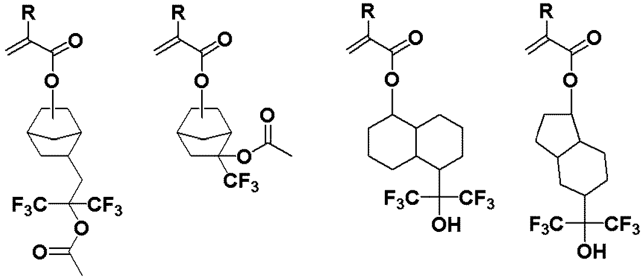

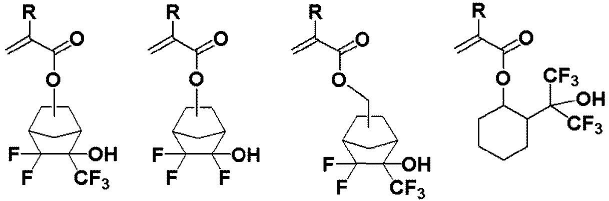

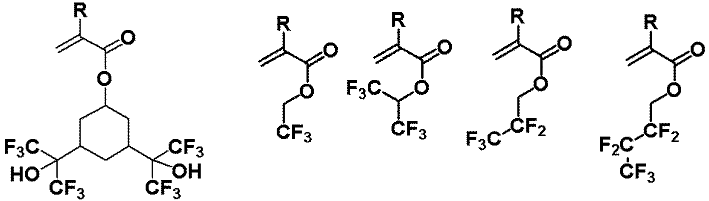

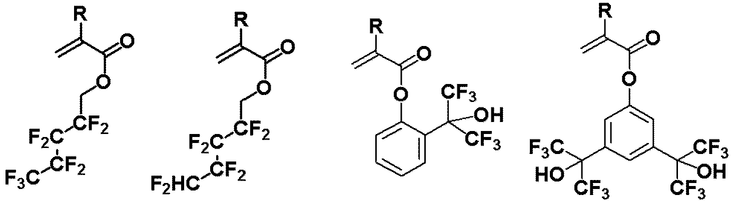

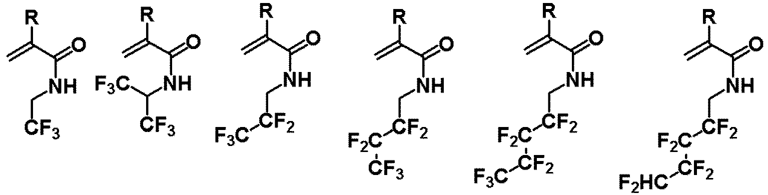

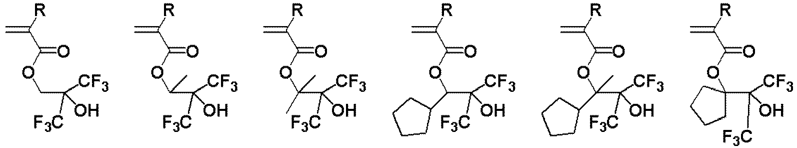

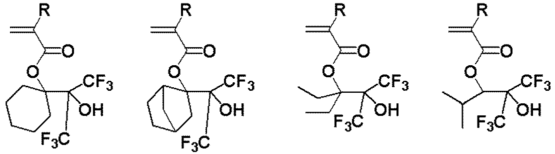

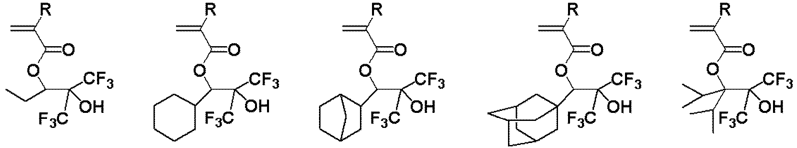

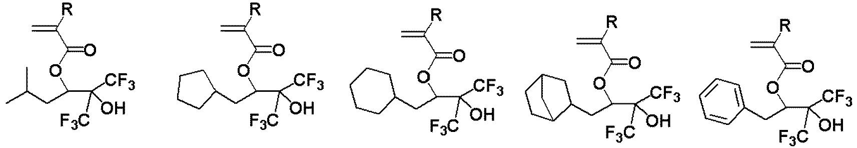

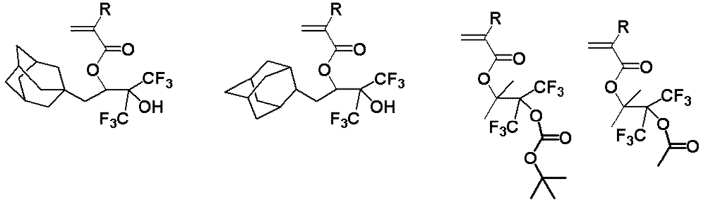

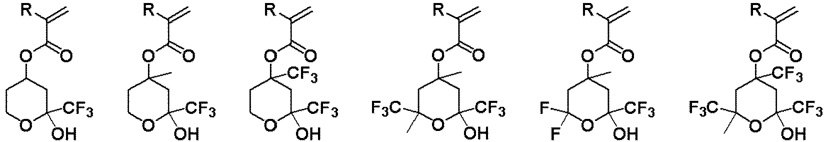

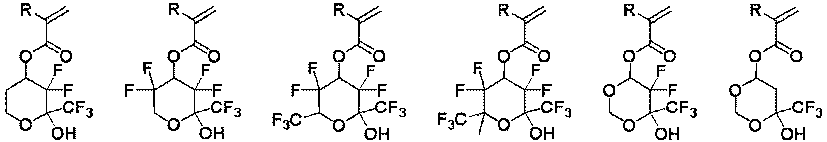

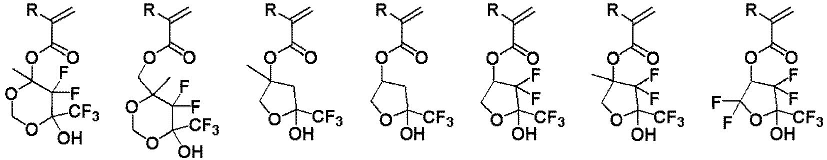

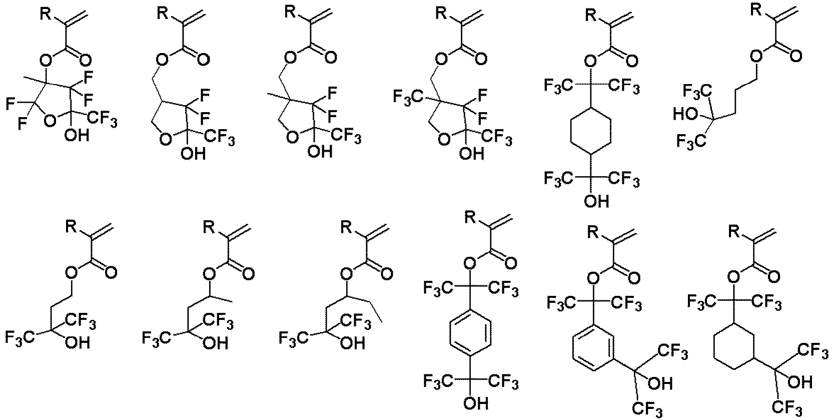

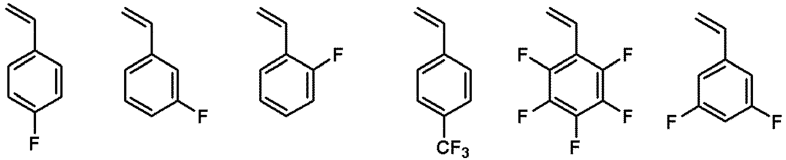

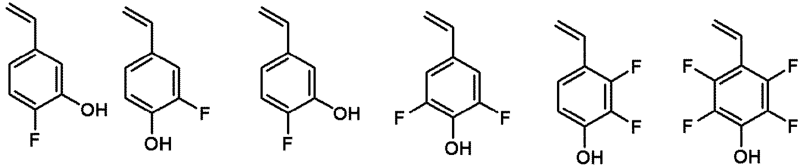

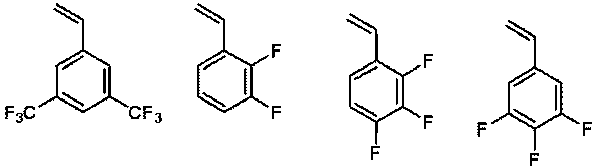

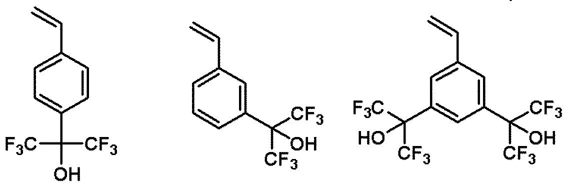

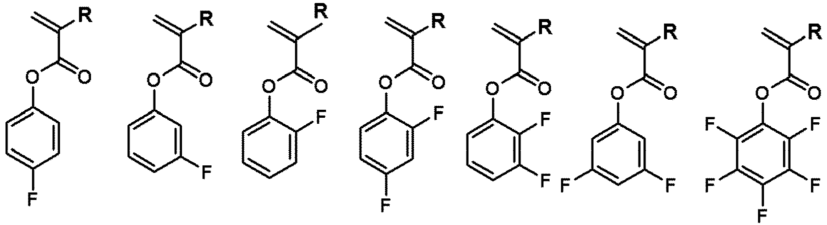

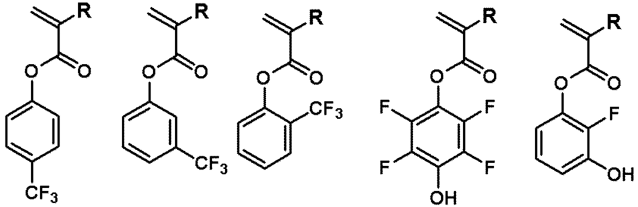

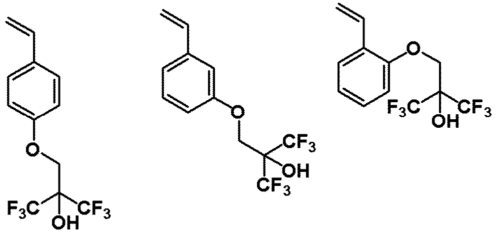

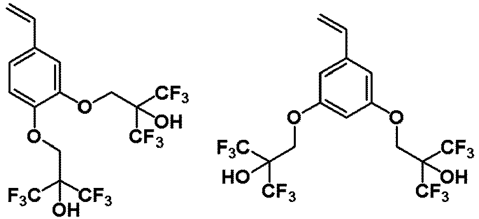

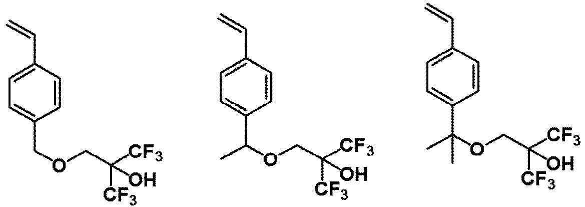

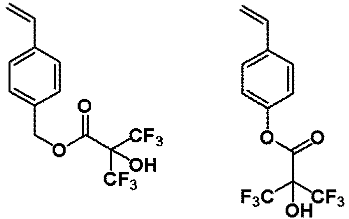

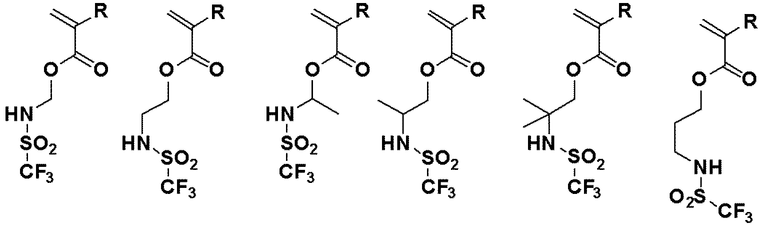

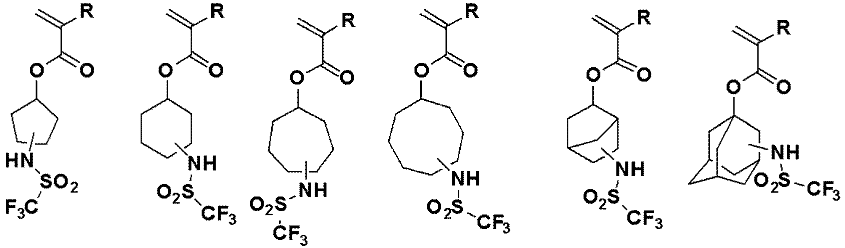

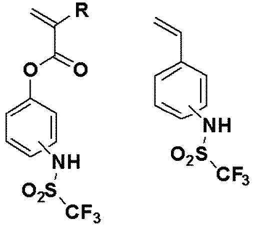

### (R represents a hydrogen atom or a methyl group.)

### (Repeating Unit-h)

The component (A) of the bio-electrode composition of the present invention can contain a repeating unit-h having a cyano group, in addition to the repeating units selected from the above repeating units-a and -b to -g.

Specific examples of a monomer to give the repeating unit-h having a cyano group are shown below.

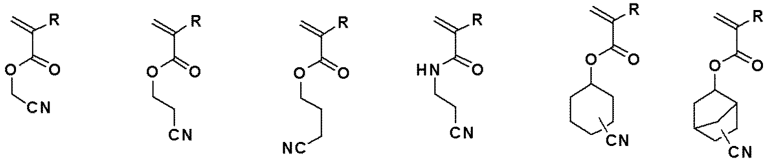

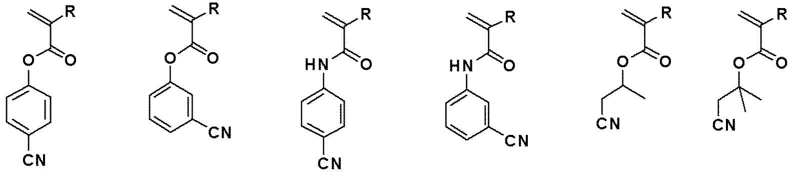

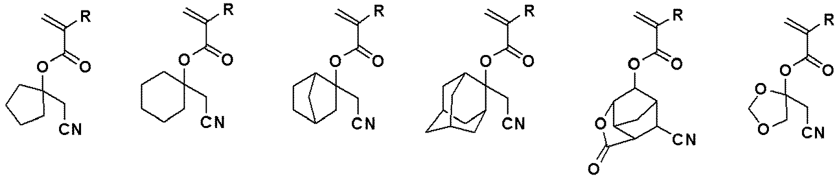

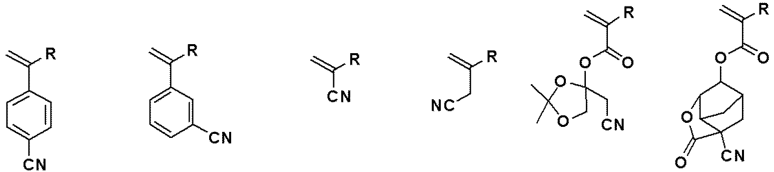

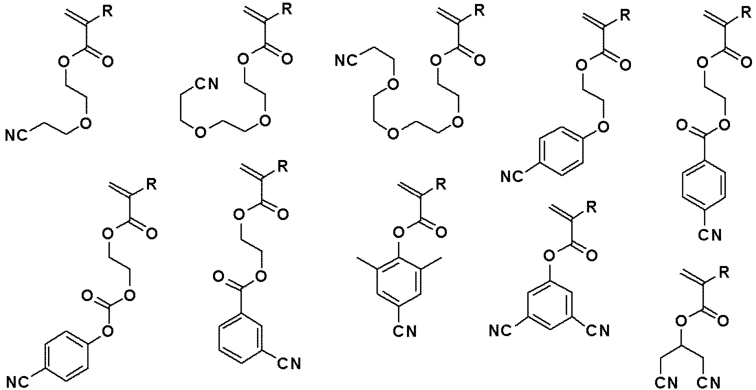

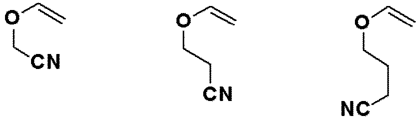

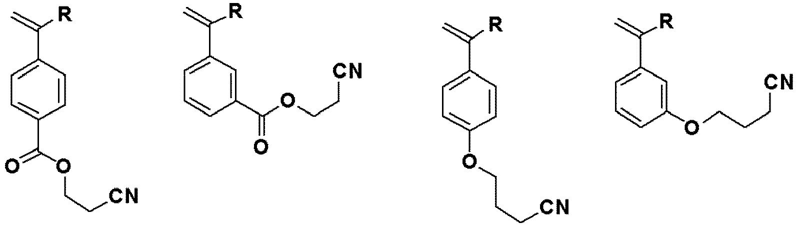

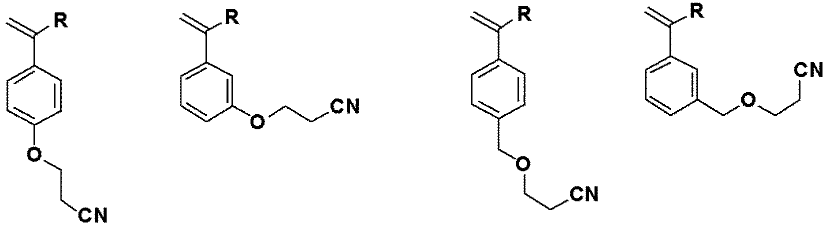

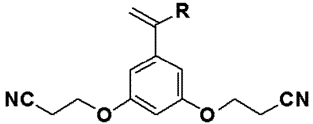

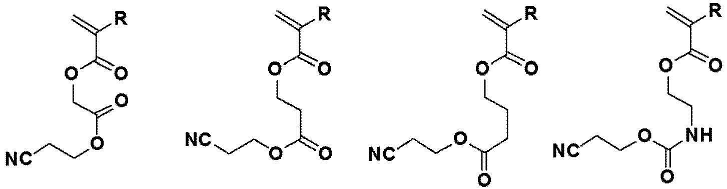

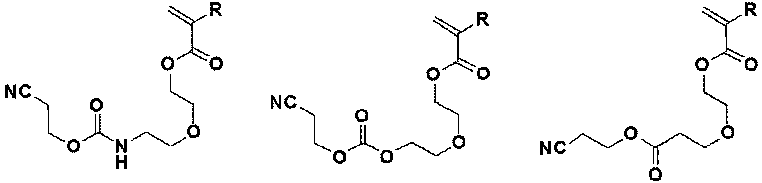

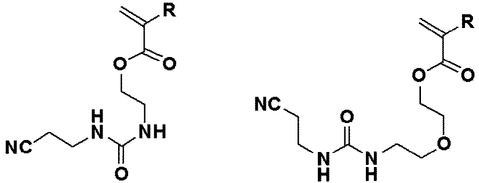

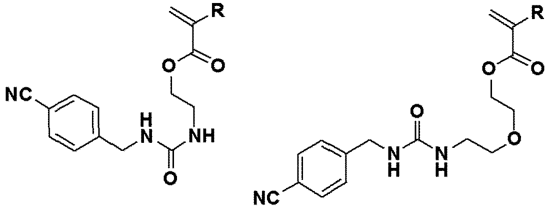

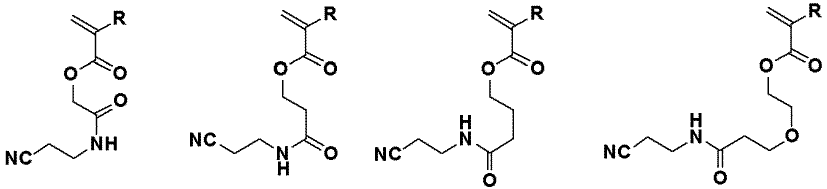

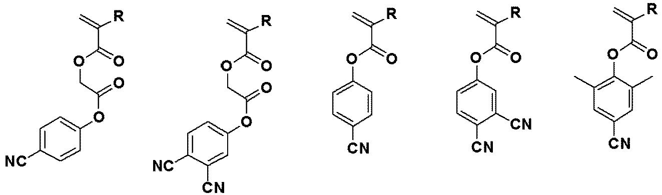

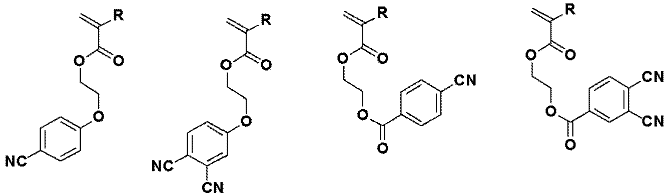

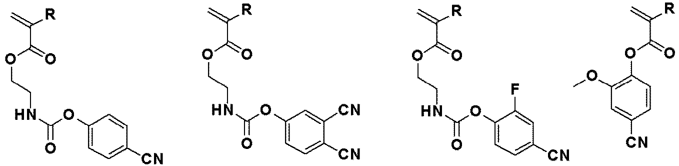

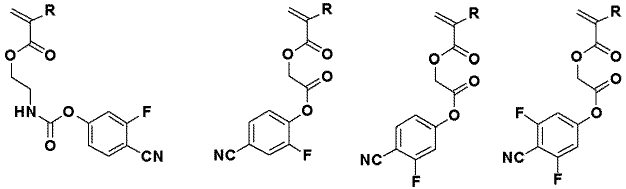

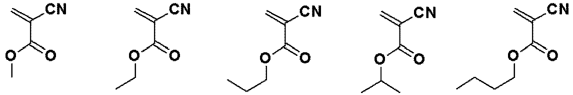

### (R represents a hydrogen atom or a methyl group.)

### (Repeating Unit-i)

The component (A) of the bio-electrode composition of the present invention can contain a repeating unit-i having a nitro group, in addition to the repeating units selected from the above repeating units-a and -b to -h.

Specific examples of a monomer to give the repeating unit-i having a nitro group are shown below.

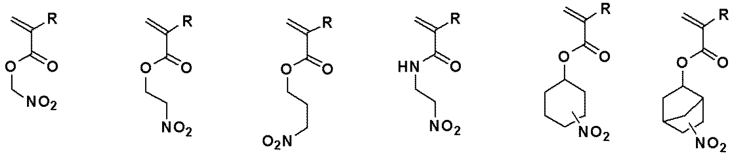

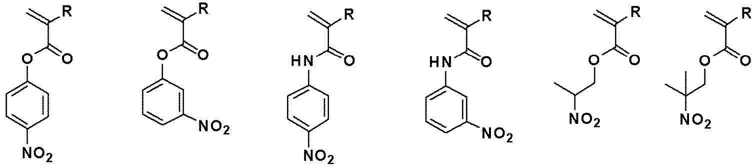

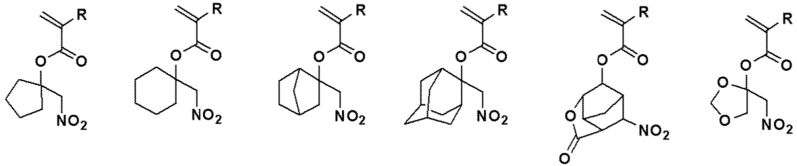

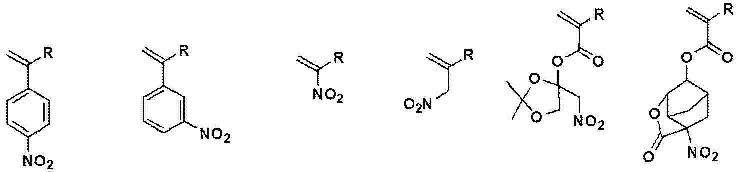

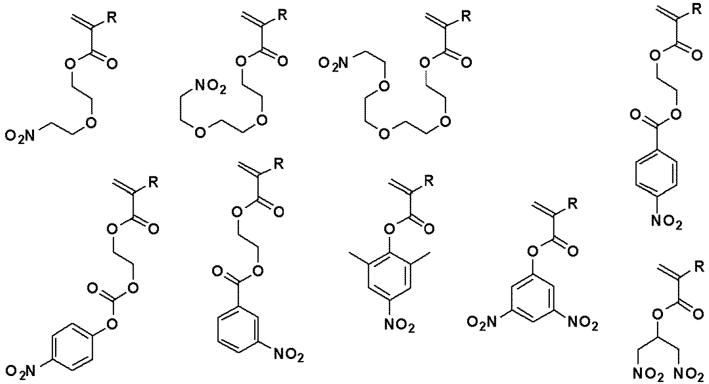

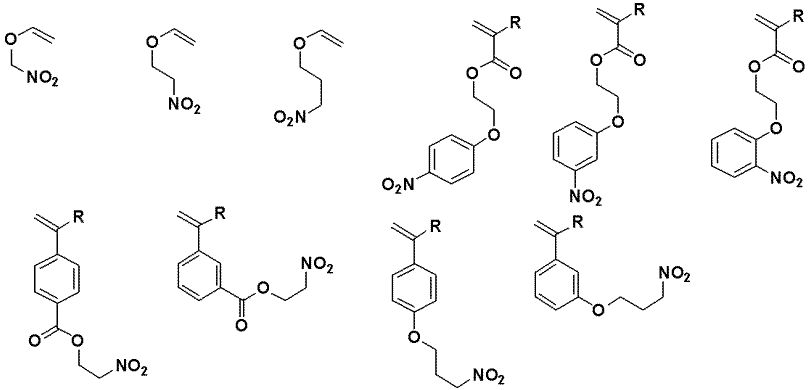

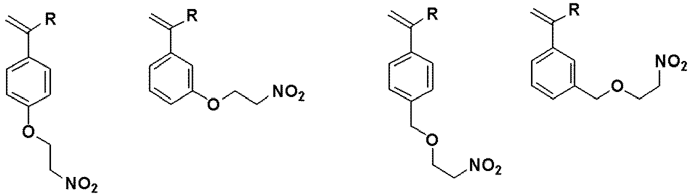

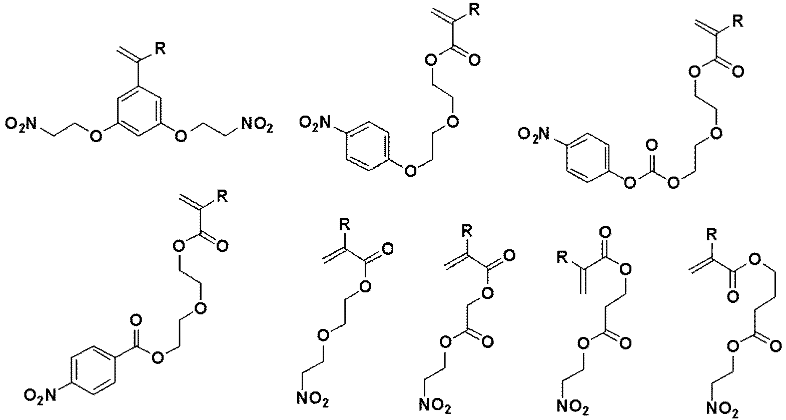

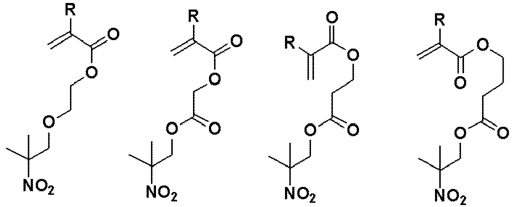

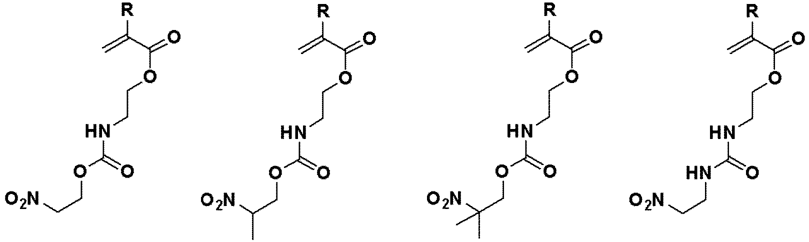

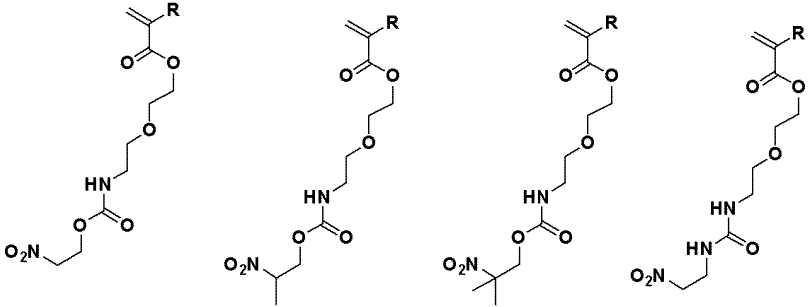

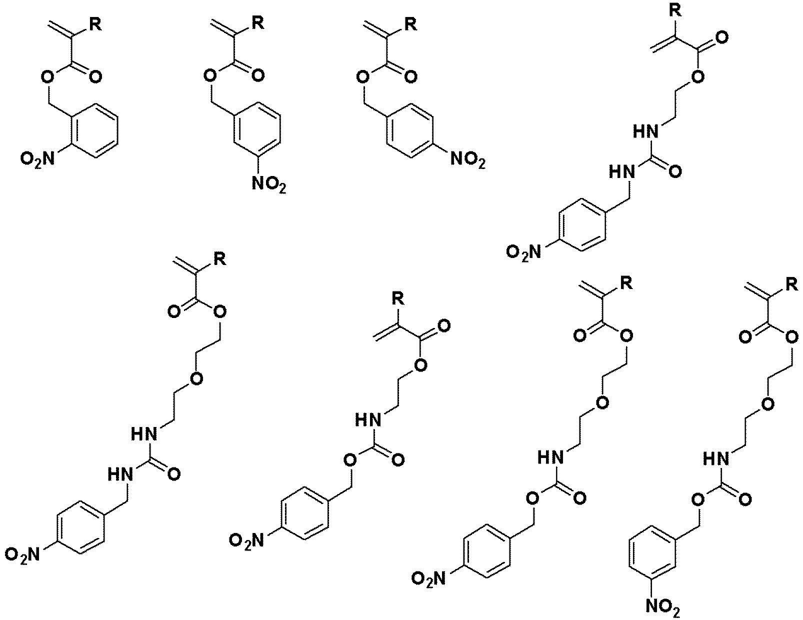

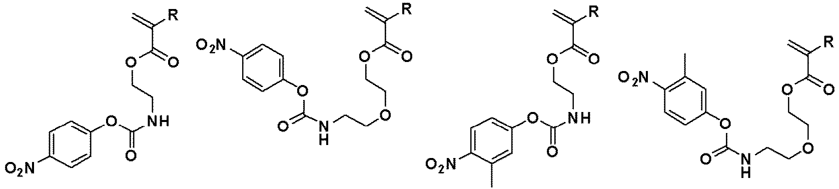

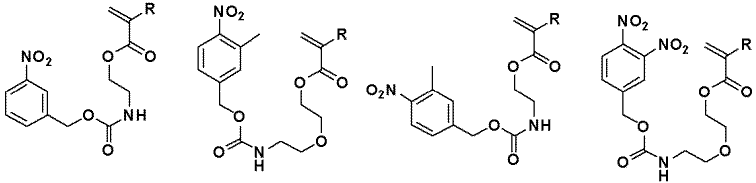

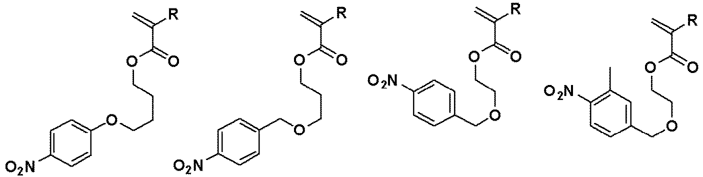

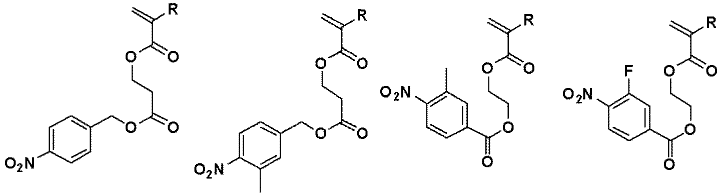

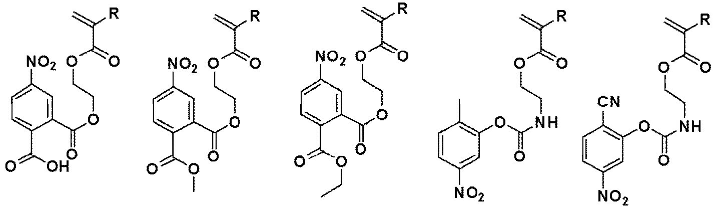

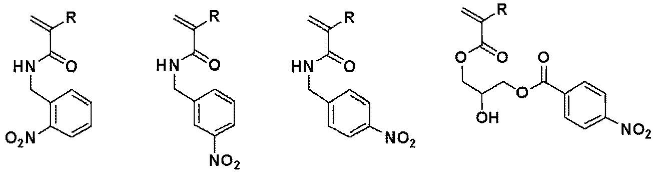

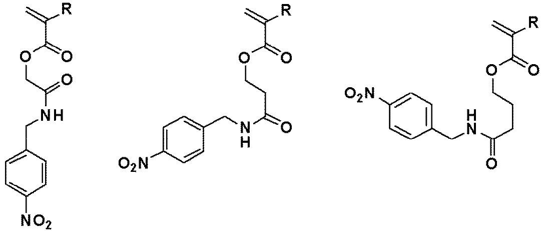

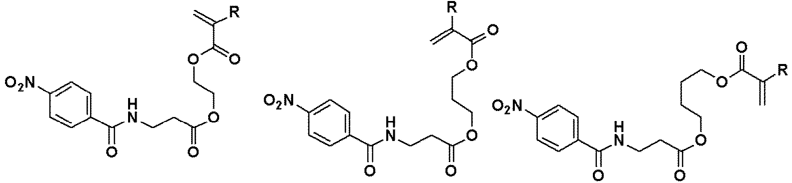

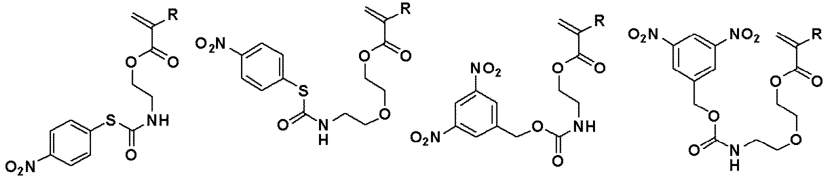

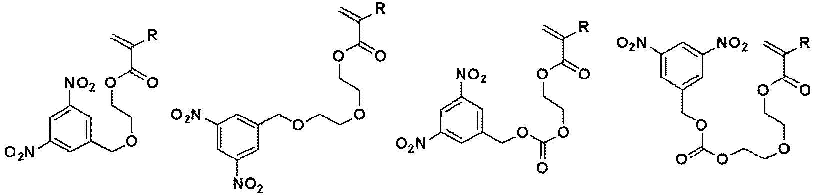

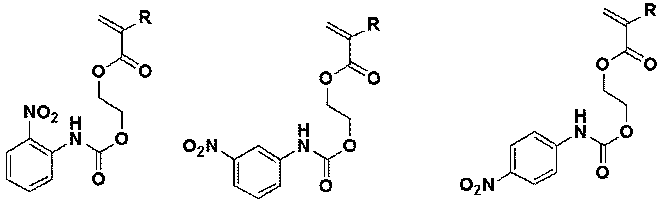

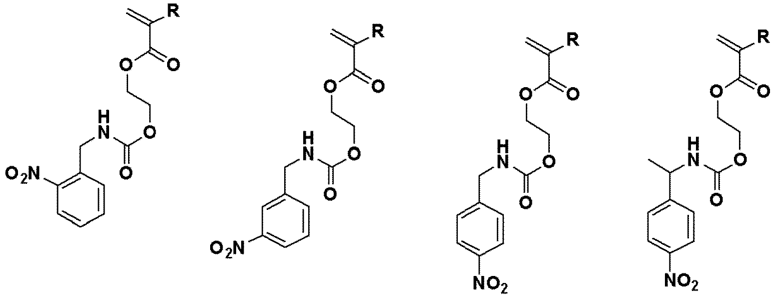

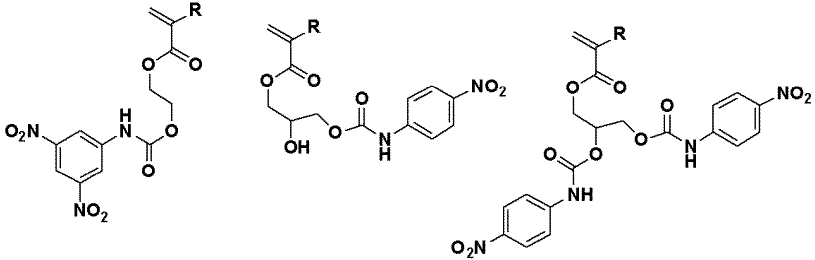

As one of methods for synthesizing the ionic resin of the component (A), a polymer compound of a copolymer can be obtained by heat polymerization of desired monomers among the monomers to give the repeating units-a, -b, -c, -d, -e, -f, -g, -h, and -i in an organic solvent, to which a radical polymerization initiator is added.

Examples of the organic solvent used for the polymerization include toluene, benzene, tetrahydrofuran, diethyl ether, dioxane, and the like.
Examples of the polymerization initiator include 2,2'-azobisisobutyronitrile (AIBN), 2,2'-azobis(2,4-dimethylvaleronitrile), dimethyl 2,2-azobis(2-methylpropionate), benzoyl peroxide, lauroyl peroxide, and the like. The heating temperature is preferably 50 to 80°C, and the reaction time is preferably 2 to 100 hours, more preferably 5 to 20 hours.

Here, the proportion of the repeating units-a, -b, -c, -d, -e, -f, -g, -h, and -i in the ionic resin of the component (A) satisfies 0<a≤1.0, 0≤b<1.0, 0≤c<1.0, 0≤d<1.0, 0≤e<0.9, 0≤f<0.9, 0≤g<0.9, 0≤h<0.9, and 0≤i<0.9; preferably 0.05≤a≤0.9, 0.01≤b≤0.9, 0≤c≤0.8, 0≤d≤0.8, 0≤e<0.8, 0≤f<0.8, 0≤g<0.8, 0≤h<0.8, and 0≤i<0.8; and more preferably 0.1≤a≤0.8, 0.05≤b≤0.8, 0≤c≤0.7, 0≤d≤0.5, 0≤e≤0.3, 0≤f≤0.7, 0≤g≤0.7, 0≤h≤0.7, and 0≤i≤0.7.

Incidentally, for example, a+b+c+d+e+f+g+h+i=1 indicates that the total amount of the repeating units-a, -b, -c, -d, -e, -f, -g, -h, and -i is 100 mol% relative to the total amount of the whole repeating units in the polymer compound containing the repeating units-a, -b, -c, -d, -e, -f, -g, -h, and -i. a+b+c+d+e+f+g+h+i<1 indicates that the total amount of the repeating units-a, -b, -c, -d, -e, -f, -g, -h, and - i is less than 100 mol% relative to the total amount of the whole repeating units and there are other repeating units in addition to the repeating units-a, -b, -c, -d, -e, -f, -g, -h, and -i.

Regarding the molecular weight of the ionic resin of the component (A), the weight-average molecular weight is preferably 500 or more, more preferably 1,000 or more and 1,000,000 or less, and further preferably 2,000 or more and 500,000 or less. Furthermore, when only a small amount of an ionic monomer (residual monomer) is not incorporated into the polymer compound of the component (A) after the polymerization, there is no risk of permeation thereof into skin in a biocompatibility test to cause allergies. Accordingly, it is preferable to reduce the amount of the residual monomer. The amount of the residual monomer is preferably 10 parts by mass or less relative to 100 parts by mass of the entire polymer compound of the component (A) before condensation reaction.
Furthermore, the component (A) may be used alone or in combination of two or more kinds, which are different in the molecular weight, dispersity, and polymerization monomer.

Incidentally, in the present invention, the molecular weight (Mw) and the dispersity (Mw/Mn) of a polymer can be determined by gel permeation chromatography (GPC) using tetrahydrofuran (THF) as a solvent. While GPC measurement (if performed) is usually carried out at a room temperature of about 23 degrees, it may be carried out at a higher or lower temperature than this.

In the bio-electrode composition of the present invention, the blending amount of the component (A) is not particularly limited, and can be 1 to 50 parts by mass relative to 100 parts by mass of the composition, for example. Alternatively, the blending amount of the component (A) is preferably 0.1 to 300 parts by mass, more preferably 1 to 200 parts by mass, relative to 100 parts by mass of the component (B). Moreover, the component (A) may be used alone or used as a mixture of two or more kinds thereof.

### (B) Resin

The resin (B) (resin other than the component (A)) to be blended into the bio-electrode composition of the present invention is a component that is compatible with the above ionic resin (salt) (A) so as to prevent elution of the salt, retains an electric conductivity improver such as metal powder, carbon powder, silicon powder, and lithium titanate powder, and further improves adhesiveness. When the ionic resin (A) has sufficient adhesiveness, the resin (B) is not necessarily required. Note that the resin (B) can be a resin other than the above-described component (A), and is preferably either or both of a thermosetting resin and a photo-curing resin, particularly preferably one or more selected from a silicone resin, a (meth)acrylate resin, and an urethane resin.

Examples of the adhesive silicone resin include resins which are curable through addition reaction or radical crosslinking reaction. Usable as the curable resin through addition reaction is, for example, the one containing diorganosiloxane having an alkenyl group, MQ resin having R₃SiO_{0.5} and SiO₂ units, organohydrogenpolysiloxane having a plurality of SiH groups, a platinum catalyst, an addition reaction inhibitor, and an organic solvent as disclosed in JP 2015-193803 A. Furthermore, usable as the curable resin through radical crosslinking reaction is, for example, the one containing diorganopolysiloxane with or without an alkenyl group, MQ resin having R₃SiO_{0.5} and SiO₂ units, organic peroxide, and an organic solvent as disclosed in JP 2015-193803 A. Here, R represents a substituted or unsubstituted monovalent hydrocarbon group having 1 to 10 carbon atoms.

It is also possible to use a polysiloxane-resin integrated compound that is formed by condensation reaction of polysiloxane having silanol at the terminal or the side chain of the polymer and MQ resin. While the MQ resin contains a lot of silanol and thus addition thereof improves adhesive strength, the MQ resin is not crosslinkable and thus does not bind to the polysiloxane in molecular level. Integration of the polysiloxane and the resin as described above can increase the adhesive strength.

Additionally, to the silicone resin, modified siloxane can also be added, which has a group selected from an amino group, an oxirane group, an oxetane group, a polyether group, a hydroxy group, a carboxyl group, a mercapto group, a methacryl group, an acryl group, a phenol group, a silanol group, a carboxylic anhydride group, an aryl group, an aralkyl group, an amide group, an ester group, and a lactone ring. The addition of the modified siloxane can improve dispersibility of the component (A) in the silicone resin. The modified siloxane may be modified at any of the single end, both ends, or side chain of the siloxane.

Furthermore, it is preferable to contain, as the component (B), a silicone resin having a RₓSiO_{(4-x)/2} unit (R represents a substituted or unsubstituted monovalent hydrocarbon group having 1 to 10 carbon atoms; and "x" is within a range of 2.5 to 3.5) and a SiO₂ unit.

As the adhesive (meth)acrylate resin, for example, it is possible to use the one having a hydrophilic (meth)acrylic ester and a hydrophobic long chain (meth)acrylic ester, as disclosed in JP 2016-011338 A, as the repeating units. In some cases, it is also possible to copolymerize a (meth)acrylic ester having a functional group or a (meth)acrylic ester having a siloxane bond.

As the adhesive urethane resin, for example, it is possible to use the one having an urethane bond as well as a polyether bond, a polyester bond, a polycarbonate bond, or a siloxane bond as disclosed in JP 2016-065238 A.

Furthermore, in order to prevent reduced electric conductivity caused by separation of the component (A) from a living body contact layer, in the bio-electrode composition of the present invention, the resin (B) preferably has high compatibility with the above-described component (A). Additionally, in order to prevent peeling off of the living body contact layer from an electro-conductive base material, in the bio-electrode composition of the present invention, the resin (B) preferably has high adhesiveness to the electro-conductive base material. In order to make the resin (B) have high compatibility with the electro-conductive base material and the salt, it is effective to use a resin with high polarity. Examples of such resin include resins having one or more selected from an ether bond, an ester bond, an amide bond, an imide bond, an urethane bond, a thiourethane bond, and a thiol group, polyacrylic resins, polyamide resins, polyimide resins, polyurethane resins, polythiourethane resins, and the like. Meanwhile, the living body contact layer is in contact with a living body and thus susceptible to perspiration from the living body. Accordingly, in the bio-electrode composition of the present invention, the resin (B) is preferably highly water-repellent and difficult to hydrolyze. To make the resin highly water-repellent and difficult to hydrolyze, it is effective to use a silicon-containing resin.

The silicon atom-containing polyacrylic resin includes a polymer having a silicone in the main chain and a polymer having a silicon atom on the side chain, both of which can be suitably used. As the polymer having a silicone in the main chain, it is possible to use siloxane or silsesquioxane having a (meth)acrylpropyl group, or the like. In this case, the (meth)acryl moiety can be polymerized and cured by adding a photoradical generator.

As the silicon-atom containing polyamide resin, for example, polyamide silicone resins disclosed in JP 2011-079946 A and US 5981680 A, etc. can be suitably used. Such polyamide silicone resins can be synthesized by combining, for example, a silicone or non-silicone compound having amino groups at both terminals with non-silicone or silicone having carboxyl groups at both terminals.

Moreover, polyamide acid before cyclization thereof may be used, which is obtained by reacting carboxylic anhydride and amine. The carboxyl group of the polyamide acid may be crosslinked by using an epoxy or oxetane crosslinking agent, or esterification reaction between the carboxyl group and hydroxyethyl(meth)acrylate may be performed for photoradical crosslinking of the (meth)acrylate moiety.

As the silicon atom-containing polyimide resin, for example, the polyimide silicone resin disclosed in JP 2002-332305 A, etc. can be suitably used. Although a polyimide resin has very high viscosity, the viscosity can be reduced by blending a (meth)acrylic monomer as a solvent and a crosslinking agent.

Examples of the silicon atom-containing polyurethane resin include polyurethane silicone resins. Such polyurethane silicone resins can be crosslinked through an urethane bond by blending a compound having isocyanate groups at both terminals and a compound having a hydroxy group at the terminal, followed by heating thereof. Note that in this case, a silicon atom (siloxane bond) should be contained in either or both of the compound having isocyanate groups at both terminals and the compound having a hydroxy group at the terminal. Alternatively, it is also possible to blend and photo-crosslink polysiloxane and an urethane(meth) acrylate monomer, as disclosed in JP 2005-320418 A. It is also possible to photo-crosslink a polymer having both of a siloxane bond and an urethane bond and having a (meth)acrylate group at the terminal. Particularly, the material including a silicone chain attached to a side chain and a polyurethane main chain, as disclosed in JP 2018-123304 A and JP 2019-70109 A, is preferable because of its high strength and high stretchability.

The silicon atom-containing polythiourethane resin can be obtained by reaction of a compound having a thiol group and a compound having an isocyanate group, provided that either of them contains a silicon atom. Photo-curing is also possible if a (meth)acrylate group is contained at the terminal.

In the silicone-based resin, compatibility with the above-described salt can be enhanced by adding the diorganosiloxane having an alkenyl group, the MQ resin having R₃SiO_{0.5} and SiO₂ units, and the organohydrogenpolysiloxane having a plurality of SiH groups as described above, as well as the modified siloxane having a group selected from an amino group, an oxirane group, an oxetane group, a polyether group, a hydroxy group, a carboxyl group, a mercapto group, a methacryl group, an acryl group, a phenol group, a silanol group, a carboxylic anhydride group, an aryl group, an aralkyl group, an amide group, an ester group, and a lactone ring.

The diorganosiloxane having an alkenyl group and the organohydrogenpolysiloxane having a plurality of SiH groups can be crosslinked with each other by addition reaction with a platinum catalyst.

Examples of the platinum catalyst include: platinum-based catalysts such as chloroplatinic acid, an alcohol solution of chloroplatinic acid, a reaction product of chloroplatinic acid and alcohol, a reaction product of chloroplatinic acid and an olefin compound, a reaction product of chloroplatinic acid and vinyl group-containing siloxane, a platinum-olefin complex, and a complex of platinum and vinyl group-containing siloxane; platinum group metal-based catalysts such as a rhodium complex and a ruthenium complex; and the like. Furthermore, these catalysts may be used after dissolved or dispersed in an alcohol, hydrocarbon, or siloxane solvent.

Note that the platinum catalyst is preferably added in an amount within a range of 5 to 2,000 ppm, particularly 10 to 500 ppm, relative to 100 parts by mass of the resins as the components (A) and (B) in total.

In the bio-electrode composition of the present invention, the blending amount of the component (B) is preferably 0 to 2000 parts by mass, more preferably 10 to 1000 parts by mass, relative to 100 parts by mass of the ionic resin (A). Moreover, the component (B) may be used alone or used as a mixture of two or more kinds thereof.

Furthermore, in a case of using the addition-curable silicone resin, an addition reaction inhibitor may be added. This addition reaction inhibitor is added as a quencher to prevent the action of the platinum catalyst in the solution and under a low temperature environment after formation of a coating film and before heat curing. Specific examples thereof include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyn, 3-methyl-3-trimethylsiloxy-1-pentyn, 3,5-dimethyl-3-trimethylsiloxy-1-hexyn, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, 1,1,3,3-tetramethyl-1,3-divinyldisiloxane, and the like.

The addition reaction inhibitor is preferably added in an amount within a range of 0 to 10 parts by mass, particularly 0.05 to 3 parts by mass, relative to 100 parts by mass of the resins as the components (A) and (B) in total.

When the component (B) has a radical-crosslinkable double bond, addition of a radical generator is effective. Examples of the radical generator include photoradical generators and thermal-radical generators.

Examples of the photoradical generator include acetophenone, 4,4'-dimethoxybenzyl, benzyl, benzoin, benzophenone, 2-benzoylbenzoic acid, 4,4'-bis(dimethylamino)benzophenone, 4,4'-bis(diethylamino)benzophenone, benzoin methyl ether, benzoin ethyl ether, benzoin isopropyl ether, benzoin butyl ether, benzoin isobutyl ether, 4-benzoylbenzoic acid, 2,2'-bis(2-chlorophenyl)-4,4',5,5'-tetraphenyl-1,2'-biimidazole, methyl 2-benzoylbenzoate, 2-(1,3-benzodioxol-5-yl)-4,6-bis(trichloromethyl)-1,3,5-triazine, 2-benzyl-2-(dimethylamino)-4'-morpholinobutyrophenone, 4,4'-dichlorobenzophenone, 2,2-diethoxyacetophenone, 2,2-dimethoxy-2-phenylacetophenone, 2,4-diethylthioxanthene-9-one, diphenyl(2,4,6-trimethylbenzoyl)phosphine oxide (BAPO), 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isonitrosopropiophenone, and 2-phenyl-2-(p-toluenesulfonyloxy)acetophenone.

The curing can also be performed by adding a radical generator of a heat decomposition type. Examples of the thermal-radical generator include 2,2'-azobis(isobutyronitrile), 2,2'-azobis(2,4-dimethylvaleronitrile), 2,2'-azobis(2-methylbutyronitrile), 2,2'-azobis(methylpropionamidine)hydrochloride, 2,2'-azobis[2-(2-imidazolin-2-yl)propane]hydrochloride, 2,2'-azobis(4-methoxy-2,4-dimethylvaleronitrile), 2,2'-azobis(2-methylbutyronitrile), 2,2'-azobis(cyclohexane-1-carbonitrile), 1[(1-cyano-1-methylethyl)azo]formamide, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl-2,2'-azobis(isobutylate), 4,4'-azobis(4-cyanopentanoic acid), dimethyl-2,2'-azobis(2-methylpropionate), benzoyl peroxide, tert-butyl hydroperoxide, cumene hydroperoxide, di-tert-butyl peroxide, di-tert-amyl peroxide, di-n-butyl peroxide, dicumyl peroxide, and the like.

Note that the radical generator is preferably added in an amount within a range of 0.1 to 50 parts by mass relative to 100 parts by mass of the resins as the components (A) and (B) in total.

Note that the living body contact layer is a cured material of the bio-electrode composition as described later. By virtue of curing, the living body contact layer has favorable adhesiveness to both of skin and the electro-conductive base material. Note that the curing means is not particularly limited, and common means can be used. For example, either or both of heat curing and photo-curing, crosslinking reaction with an acid catalyst or a base catalyst, etc. can be used. The crosslinking reaction can be carried out by appropriately selecting, for example, the method described in Yasuharu, Nakayama, Crosslinking Reaction Handbook, Maruzen Publishing Co., Ltd. 2013, Chapter 2, pp.51-371.

### Ionic Polymer

To the bio-electrode composition of the present invention, an ionic polymer other than the component (A) can be added. Preferable for use as the ionic polymer are those disclosed in JP 2018-126496 A and JP 2018-130533 A. The ionic polymer is preferably added in an amount within a range of 0.1 to 100 parts by mass relative to 100 parts by mass of the resins as the components (A) and (B) in total.

### (C) Carbon Powder and/or Metal Powder

To the bio-electrode composition of the present invention, carbon powder and/or metal powder can be added in order to enhance electronic conductivity.

### Metal Powder

To the bio-electrode composition of the present invention, powder of a metal selected from gold, silver, platinum, copper, tin, titanium, nickel, aluminum, tungsten, molybdenum, ruthenium, chromium, and indium can also be added in order to enhance electronic conductivity. The metal powder is preferably added in an amount within a range of 1 to 50 parts by mass relative to 100 parts by mass of the resins as the components (A) and (B) in total.

As the kind of the metal powder, gold, silver, and platinum are preferred from the viewpoint of electric conductivity, while silver, copper, tin, titanium, nickel, aluminum, tungsten, molybdenum, ruthenium, and chromium are preferred from the viewpoint of cost. Noble metal is preferred from the viewpoint of biocompatibility, and silver is the most preferable from comprehensive viewpoints including the above.

The shape of the metal powder can include spherical, disk, flaky, and needle shapes. Addition of the flaky powder leads to the highest electric conductivity and is thus preferable. The flake preferably has a relatively lower density and large specific surface area, with a metal powder size of 100 µm or less, a tap density of 5 g/cm³ or less, and a specific surface area of 0.5 m²/g or more.

### Carbon Powder

The carbon powder can be added as an electric conductivity improver. Examples of the carbon powder include carbon black, plumbago (graphite), carbon nanotube, carbon fiber, graphene, and the like. The carbon nanotube may be either single-layer or multilayer carbon nanotube, and the surface thereof may be modified with an organic group. The carbon powder is particularly preferably one or both of carbon black and carbon nanotube. The carbon powder is preferably added in an amount within a range of 1 to 50 parts by mass relative to 100 parts by mass of the resins as the components (A) and (B) in total.

### Silicon Powder

To the bio-electrode composition of the present invention, silicon powder can be added in order to enhance ion reception sensitivity. Examples of the silicon powder include powder of silicon, silicon monoxide, or silicon carbide. The powder has a particle size of preferably smaller than 100 µm, more preferably 1 µm or smaller. Since finer particles have a larger surface area, the resulting bio-electrode can receive a larger number of ions and has high sensitivity. The silicon powder is preferably added in an amount within a range of 1 to 50 parts by mass relative to 100 parts by mass of the resins as the components (A) and (B) in total.

### Lithium Titanate Powder

To the bio-electrode composition of the present invention, lithium titanate powder can be added in order to enhance ion reception sensitivity. Examples of the lithium titanate powder include those represented by molecular formulae Li₂TiO₃, LiTiO₂, and Li₄Ti₅O₁₂ with a spinel structure. The lithium titanate powder preferably has a spinel structure. It is also possible to use composite particles of lithium titanate with carbon. The powder has a particle size of preferably smaller than 100 µm, more preferably 1 µm or smaller. Since finer particles have a larger surface area, the resulting bio-electrode can receive a large number of ions and has high sensitivity. These may be composite powder with carbon. The lithium titanate powder is preferably added in an amount within a range of 1 to 50 parts by mass relative to 100 parts by mass of the resins as the components (A) and (B) in total.

### Crosslinking Agent

To the bio-electrode composition of the present invention, an epoxy crosslinking agent can also be added. The crosslinking agent in this case is a compound having a plurality of epoxy groups or oxetane groups in one molecule. The addition amount thereof is 1 to 30 parts by mass relative to 100 parts by mass of the resins as the components (A) and (B) in total.

### Crosslinking Catalyst

To the bio-electrode composition of the present invention, a catalyst for crosslinking the epoxy groups or the oxetane groups can also be added. As the catalyst in this case, those disclosed in paragraphs 0027 to 0029 in JP 2019-503406 A can be used. The addition amount thereof is 0.01 to 10 parts by mass relative to 100 parts by mass of the resins as the components (A) and (B) in total.

### Ionic Additive

To the bio-electrode composition of the present invention, an ionic additive for increasing ionic conductivity can be added. By taking into consideration the biocompatibility, examples thereof include sodium chloride, potassium chloride, calcium chloride, magnesium chloride, saccharin sodium salt, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, betaine, and salts disclosed in JP 2018-44147 A, JP 2018-59050 A, JP 2018-59052 A, and JP 2018-130534 A.

### (D) Organic Solvent

Additionally, an organic solvent can be added to the bio-electrode composition of the present invention. Specific examples of the organic solvent include: aromatic hydrocarbon solvents such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; aliphatic hydrocarbon solvents such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undeca-4-ene, n-dodecane, n-tridecane, n-pentadecane, n-hexadecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin; ketone solvents such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, and methyl n-pentyl ketone; alcohol solvents such as 3-methoxybutanol, 3-methyl-3-methoxybutanol, 1-methoxy-2-propanol, and 1-ethoxy-2-propanol; ether solvents such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropyl ether, diisobutyl ether, diisopentyl ether, di-n-pentyl ether, methylcyclopentyl ether, methylcyclohexyl ether, di-n-butyl ether, di-sec butyl ether, diisopentyl ether, di-sec-pentyl ether, di-tert-amyl ether, di-n-hexyl ether, and anisole; ester solvents such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactone solvents such as γ-butyrolactone; water; and the like.

Note that the organic solvent is preferably added in an amount within a range of 10 to 50,000 parts by mass relative to 100 parts by mass of the resins as the components (A) and (B) in total.

### Other Additives

With the bio-electrode composition of the present invention, silica particles, polyether silicone, and polyglycerin silicone can also be mixed. A silica particle has a hydrophilic surface and favorable compatibility with a hydrophilic ion polymer, polyether silicone, and polyglycerin silicone, and can improve dispersibility of the ionic polymer, polyether silicone, and polyglycerin silicone in the hydrophobic silicone adhesive. Both the dry and wet silica particles can be preferably used.

### Silicone Compound Having Polyglycerin Structure

To the bio-electrode composition of the present invention, a silicone compound having a polyglycerin structure can also be added in order to enhance the moisture-retaining property of a film to enhance sensitivity to ions released from skin as well as ionic conductivity. The silicone compound having a polyglycerin structure is preferably blended in an amount of 0.01 to 100 parts by mass, more preferably 0.5 to 60 parts by mass, relative to 100 parts by mass of the components (A) and (B) in total. Additionally, the silicone compound having a polyglycerin structure may be used alone or used as a mixture of two or more kinds thereof.

The silicone compound having a polyglycerin structure is preferably represented by any of the following general formulae (4)' and (5)'. 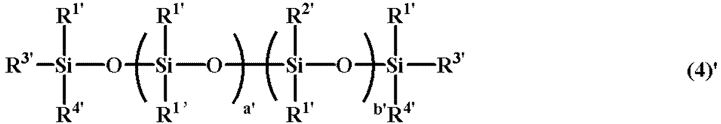 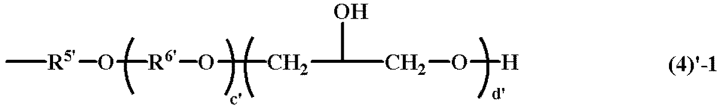 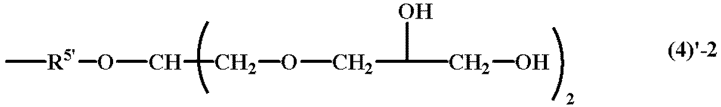 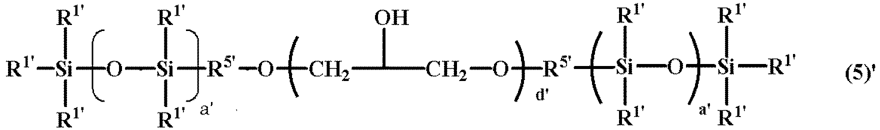 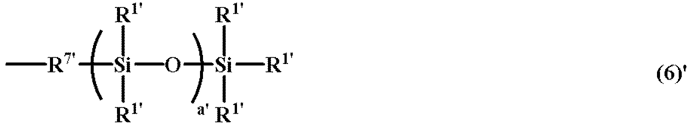 (In the formulae (4)' and (5)', each of R¹' may be identical to or different from each other and independently represents a hydrogen atom, a linear or branched alkyl group having 1 to 50 carbon atoms, or a phenyl group, and optionally contains an ether group, or each of R¹' independently represents a silicone chain represented by a general formula (6)'; R²' represents a group having a polyglycerin group structure represented by a formula (4)'-1 or (4)'-2; each of R³' may be identical to or different from each other, and independently represents the R¹' group or the R²' group; each of R⁴' may be identical to or different from each other and independently represents the R¹' group, the R²' group, or an oxygen atom, and when R⁴' represents an oxygen atom, two R⁴' groups bond together to form one ether group, which optionally forms a ring with a silicon atom; "a'" may be identical to or different from each other, and represents 0 to 100, "b'" represents 0 to 100, and a'+b' is 0 to 200, provided that when "b'" represents 0, at least one R³' represents the R²' group. In the formulae (4)'-1, (4)'-2, (5)', and (6)', R⁵' represents an alkylene group having 2 to 10 carbon atoms, or an aralkylene group having 7 to 10 carbon atoms; R⁶' and R⁷' represent an alkylene group having 2 to 6 carbon atoms, where R⁷' may represent an ether bond; "c'" represents 0 to 20; and "d'" represents 1 to 20.)

Examples of such silicone compound having a polyglycerin structure are shown below. 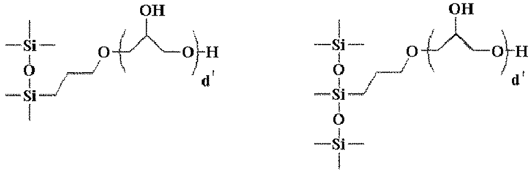 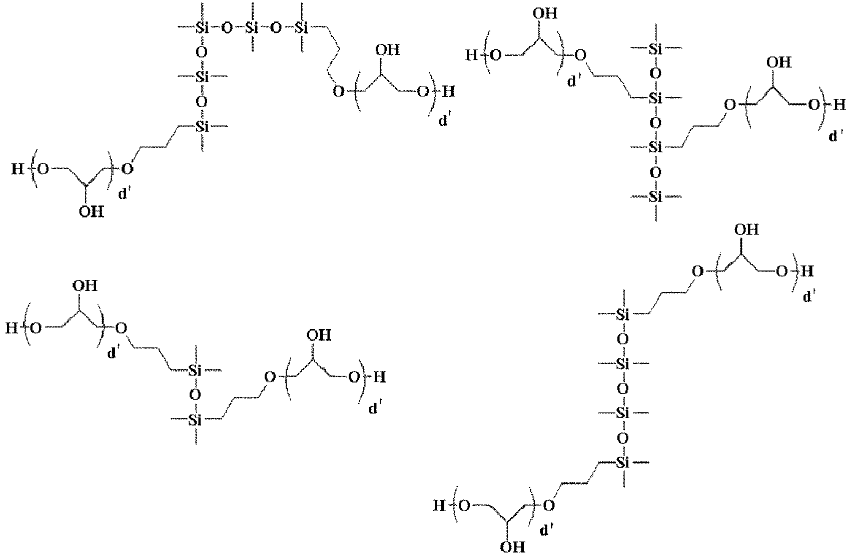 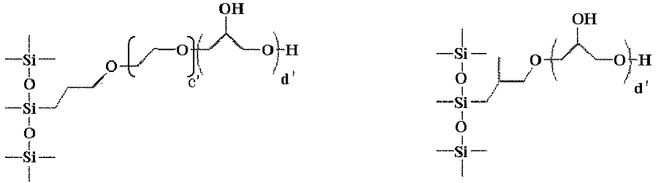 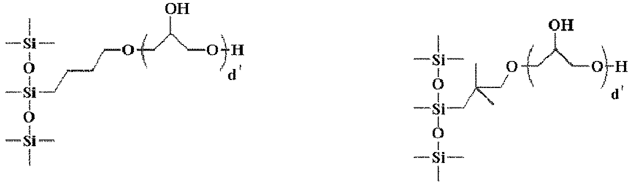 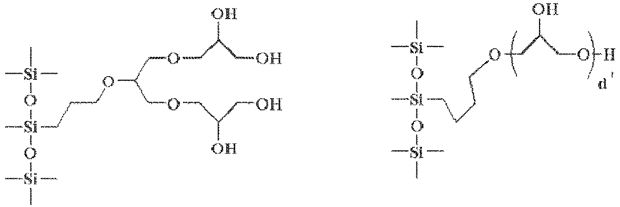 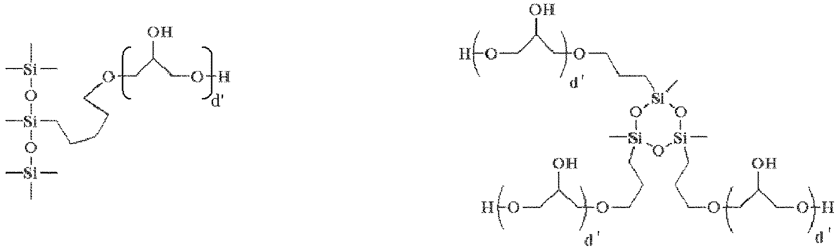 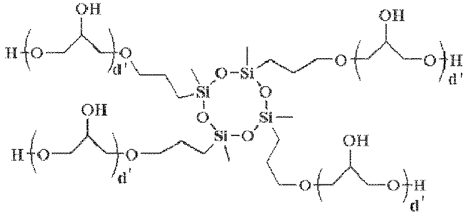 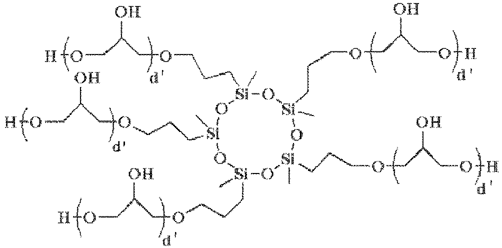 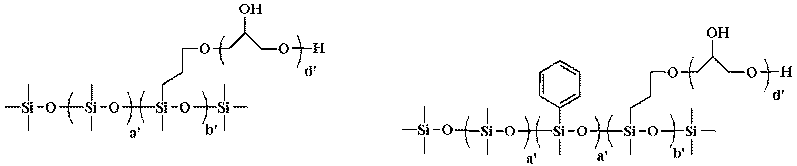 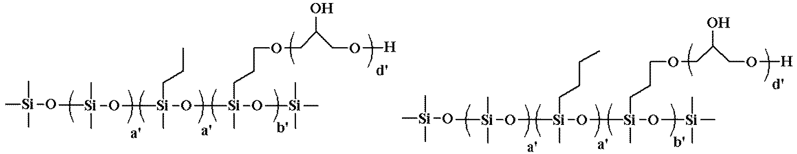 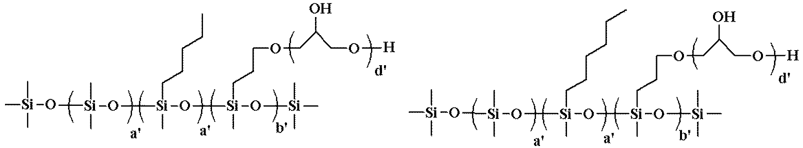 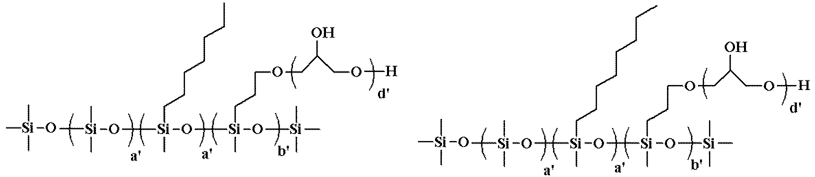 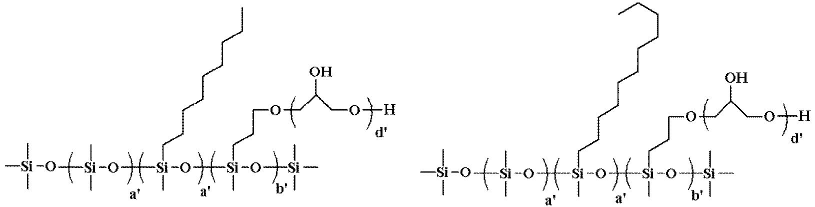 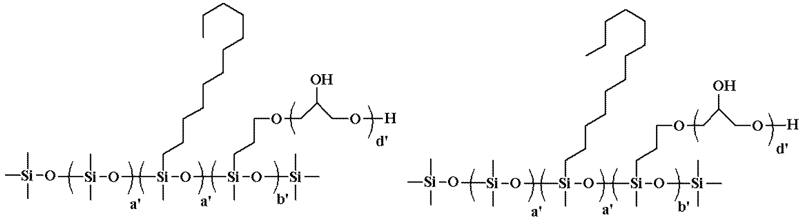 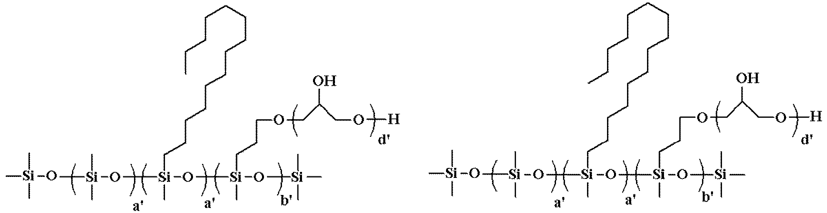 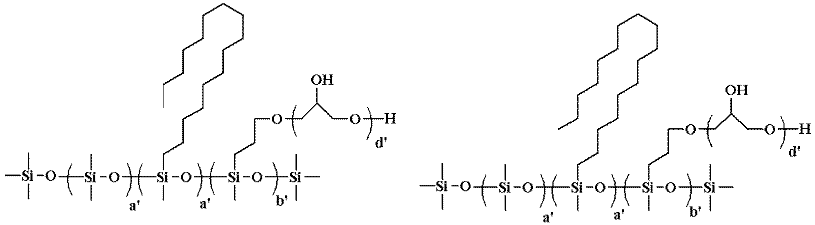 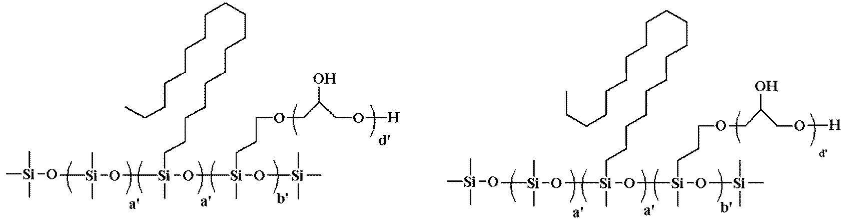 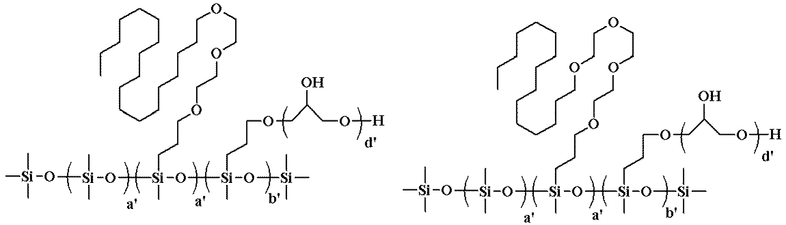 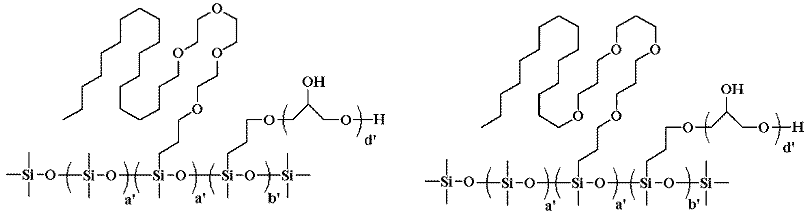 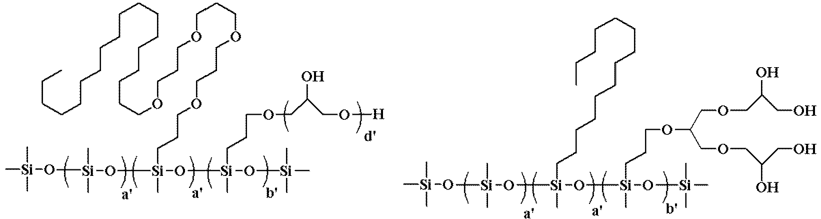 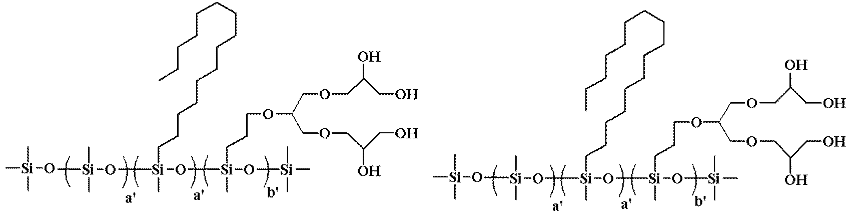 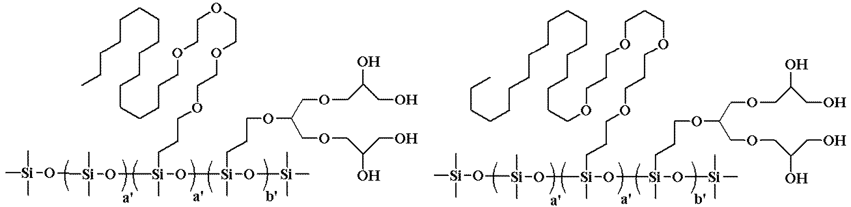 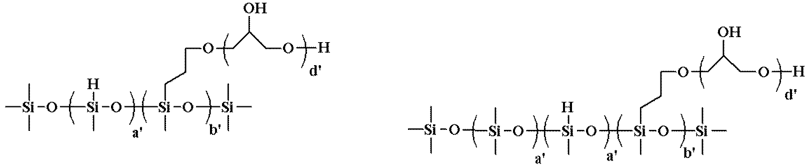 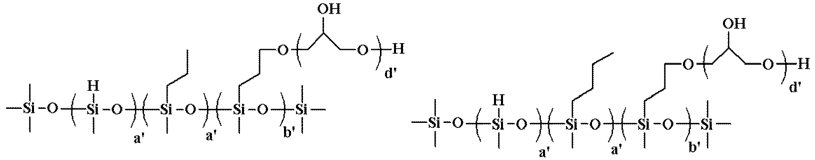 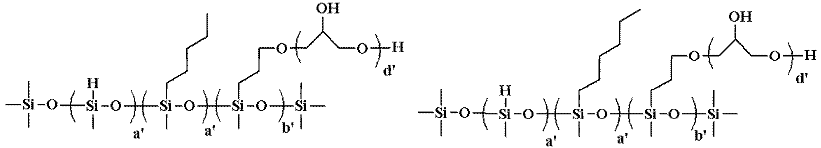 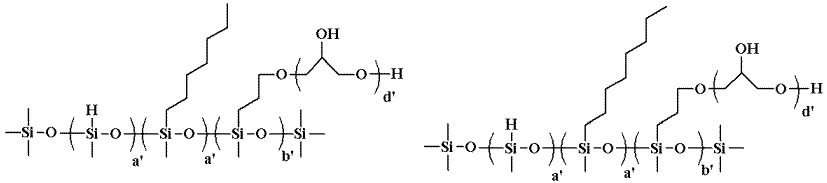 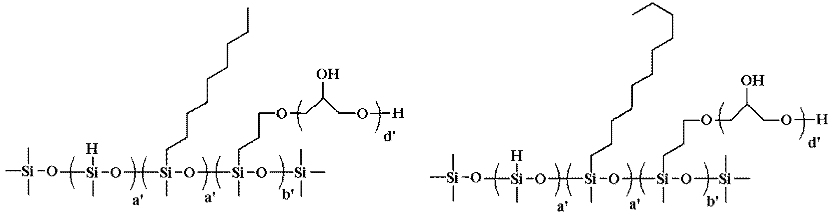 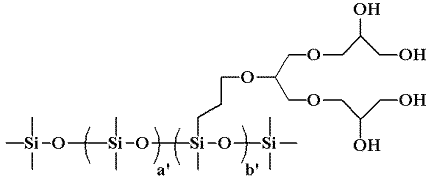 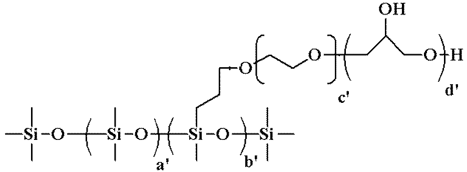 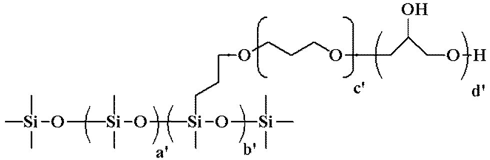 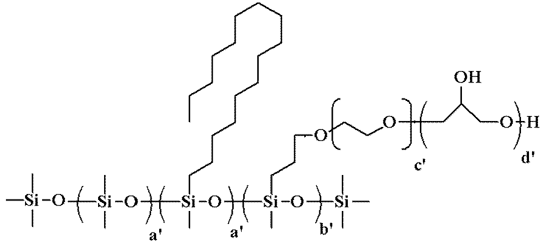 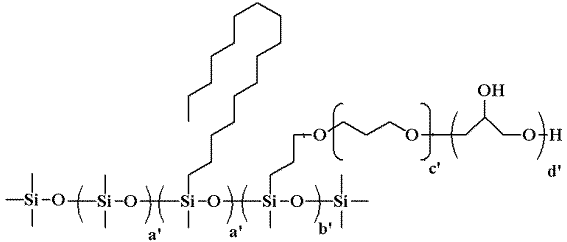 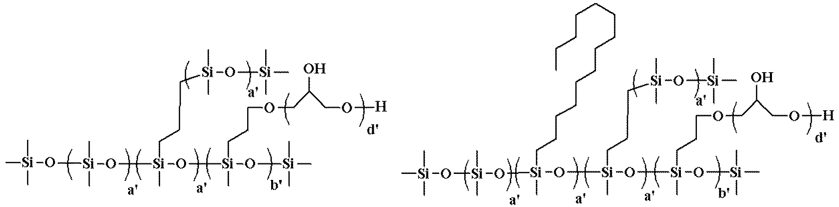 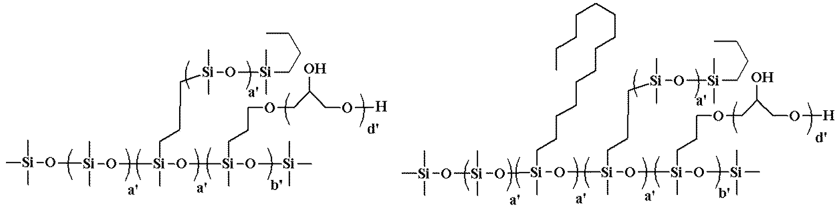 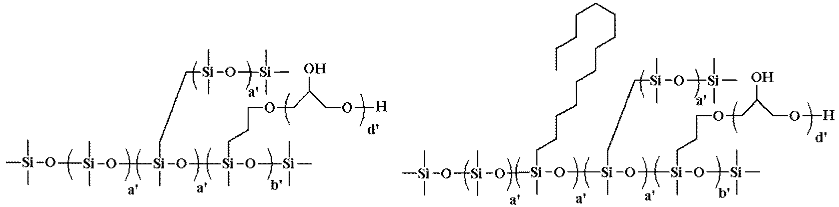 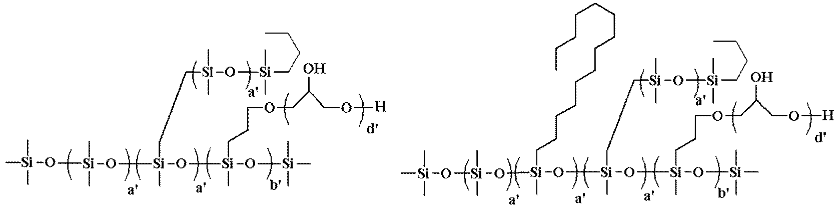 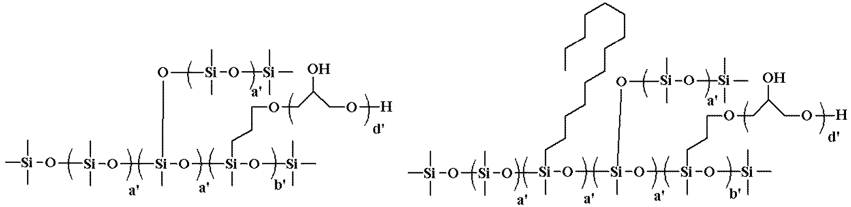 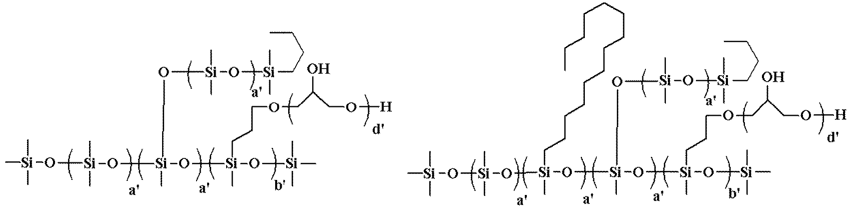 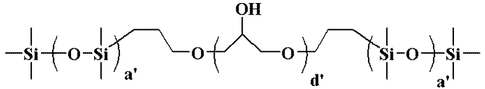 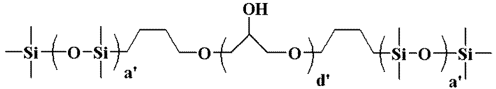 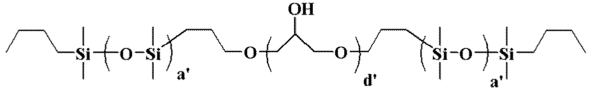 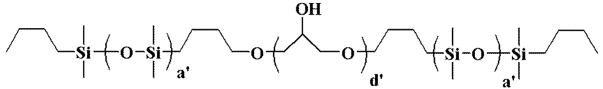 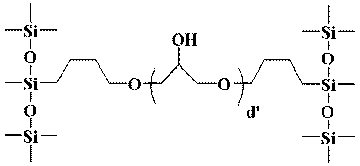 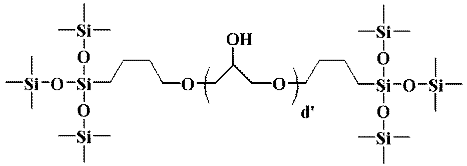 (In the formulae, "a'", "b''", "c'", and "d'" are as described above.)

The bio-electrode composition that contains such silicone compound having a polyglycerin structure is capable of forming a living body contact layer that can exhibit a more excellent moisture-retaining property and as a result, more excellent sensitivity to ions released from skin.

As described above, the bio-electrode composition of the present invention is capable of forming a living body contact layer for a bio-electrode, which is highly adhesive so as to have sufficient adhesiveness even when detached from skin and then reattached; is capable of efficiently transmitting electric signals from the skin to a device (i.e. excellent electric conductivity); has no risk of causing allergies even when the bio-electrode is attached to the skin for a long period of time (i.e. excellent biocompatibility); is lightweight; can be produced at low cost; and causes no significant decrease in the electric conductivity even when the bio-electrode gets wet from water or is dried. Moreover, addition of the carbon material can further improve the electric conductivity, and a combined use with the resin having adhesiveness and stretchability enables to produce a bio-electrode with particularly high adhesive strength and stretchability. Further, the additives and the like can improve the stretchability and the adhesiveness to the skin, and appropriate adjustment of the composition of the resin and the thickness of the living body contact layer can also control the stretchability and the adhesiveness.

### <Bio-Electrode>

The present invention also provides a bio-electrode including an electro-conductive base material and a living body contact layer formed on the electro-conductive base material, wherein the living body contact layer is a cured material of the above-described bio-electrode composition of the present invention.

Hereinafter, the bio-electrode of the present invention will be described in detail with reference to drawings. However, the present invention is not limited thereto.

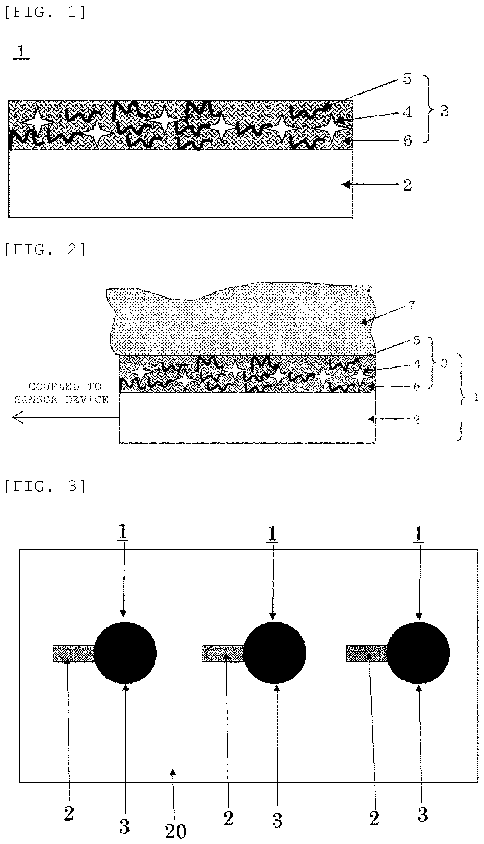

FIG. 1 is a schematic cross-sectional view of a bio-electrode according to an embodiment of the present invention. A bio-electrode 1 in FIG. 1 includes an electro-conductive base material 2 and a living body contact layer 3 formed on the electro-conductive base material 2. The living body contact layer 3 is formed from a cured material of the bio-electrode composition of the present invention. The living body contact layer 3 contains an ionic resin (A)5. The living body contact layer 3 can further contain a resin (B)6 other than the ionic resin (A), and electro-conductive powder 4. Hereinafter, with reference to FIGs. 1 and 2, an aspect where the living body contact layer 3 is a layer with the ionic resin (A)5 and the electro-conductive powder 4 dispersed in the resin (B)6 will be described. However, the bio-electrode of the present invention is not limited to this aspect.

When such bio-electrode 1 in FIG. 1 is used, as illustrated in FIG. 2, the living body contact layer 3 (i.e. the layer with the ionic resin (A)5 and the electro-conductive powder 4 dispersed in the resin (B)6) is brought into contact with a living body 7; electric signals are picked from the living body 7 through the ionic resin (A)5 and the electro-conductive powder 4; and the electric signals are conducted to a sensor device or the like (not shown) via the electro-conductive base material 2. In this manner, the bio-electrode of the present invention can achieve both of electric conductivity and biocompatibility by using the above-described ionic resin (A), keep the contact area with the skin constant thanks to the adhesiveness thereof, and stably obtain the electric signals from the skin with high sensitivity.

Hereinafter, each constituent material of the bio-electrode of the present invention will be described in more detail.

### Electro-Conductive Base Material

The bio-electrode of the present invention include an electro-conductive base material. This electro-conductive base material is usually electrically coupled to a sensor device or the like, and conducts electric signals picked from a living body through a living body contact layer to the sensor device or the like.

The electro-conductive base material is not particularly limited as long as it has electric conductivity. For example, it preferably includes one or more selected from gold, silver, silver chloride, platinum, aluminum, magnesium, tin, tungsten, iron, copper, nickel, stainless steel, chromium, titanium, carbon, and an electro-conductive polymer.

Furthermore, the electro-conductive base material is not particularly limited, and may be a hard electro-conductive substrate, an electro-conductive film having flexibility, a cloth with its surface coated with electro-conductive paste, a cloth into which an electro-conductive polymer is kneaded, or the like. The electro-conductive base material may be flat, uneven, or mesh of woven metal wire, which may be selected as appropriate depending on the intended use of the bio-electrode, etc.

### Living Body Contact Layer

The bio-electrode of the present invention includes the living body contact layer formed on the electro-conductive base material. This living body contact layer is a part to be actually in contact with the living body during use of the bio-electrode, and has electric conductivity and adhesiveness. The living body contact layer is a cured material of the above-described bio-electrode composition of the present invention, i.e. an adhesive resin layer formed from the cured material of the composition containing the above component (A), as well as the components (B), (C), and (D) and other components as necessary.

Note that the living body contact layer preferably has an adhesive strength within a range of 0.01 N/25 mm or more and 20 N/25 mm or less. The adhesive strength is commonly measured by the method shown in JIS Z 0237, where a metal substrate such as SUS (steel use stainless) and PET (polyethylene terephthalate) substrates can be used as a base material, or human skin can be used for the measurement. Surface energy of human skin is lower than surface energy of metals and various plastics, and as low as the energy of Teflon^{®}, such that the human skin is characterized by difficulty in adhesion.

In applications where attachment and peeling off are repeated many times, releasability is more important than adhesiveness, and thus the adhesiveness is not necessarily required.

The living body contact layer of the bio-electrode has a thickness of preferably 1 µm or more and 5 mm or less, more preferably 2 µm or more and 3 mm or less. The thinner the living body contact layer, the lower the adhesive strength; while flexibility is improved, resulting in lightweight and good fitting to skin. The thickness of the living body contact layer can be selected based on the balance of adhesiveness and feel on the skin.

Furthermore, the bio-electrode of the present invention may be additionally provided with an adhesive film on the living body contact layer, similarly to conventional bio-electrodes (for example, the bio-electrode disclosed in JP 2004-033468 A), in order to prevent peeling off of the bio-electrode from a living body during use. In the case of additionally providing the adhesive film, the adhesive film may be formed by using an adhesive film raw material, such as acrylic, urethane, and silicone types. In particular, the silicone type is suitable because its high oxygen permeability enables dermal respiration while keeping the bio-electrode attached to skin, its high water repellency leads to less decrease in adhesiveness due to perspiration, and further it causes less irritation to the skin. Note that in the bio-electrode of the present invention, peeling off from a living body can be prevented by adding a tackifier to the bio-electrode composition or using a resin having favorable adhesiveness to the living body as described above, and thus it is not necessarily required to provide the additional adhesive film as described above.

When the bio-electrode of the present invention is used as a wearable device, wiring between the bio-electrode and the sensor device, and other members are not particularly limited. For example, those disclosed in JP 2004-033468 A are applicable.

As described above, in the bio-electrode of the present invention, the living body contact layer is formed from the cured material of the above-described bio-electrode composition of the present invention, so that the bio-electrode is capable of efficiently transmitting electric signals from skin to a device (i.e. excellent electric conductivity), has no risk of causing allergies even when attached to the skin for a long period of time (i.e. excellent biocompatibility), is lightweight, can be produced at low cost, and does not cause significant decrease in the electric conductivity even when gets wet from water or when dried. Moreover, the electric conductivity can be further improved by adding the electro-conductive powder, and a combined use with the resin having adhesiveness and stretchability enables to produce a bio-electrode with particularly high adhesive strength and stretchability. Furthermore, stretchability and adhesiveness to the skin can be improved by the additives and the like, and appropriate adjustment of the composition of the resin and the thickness of the living body contact layer can also control the stretchability and adhesiveness. Accordingly, such bio-electrode of the present invention is particularly suitable as a bio-electrode used for a medical wearable device.

### <Method for Producing Bio-Electrode>

The present invention also provides a method for producing a bio-electrode including an electro-conductive base material and a living body contact layer formed on the electro-conductive base material. The method includes: applying the above-described bio-electrode composition of the present invention onto the electro-conductive base material; and curing the bio-electrode composition to form the living body contact layer.

Note that the electro-conductive base material, etc. used for the method for producing a bio-electrode of the present invention may be the same as those described above.

The method for applying the bio-electrode composition onto the electro-conductive base material is not particularly limited. Examples of the suitable method includes dip coating, spray coating, spin coating, bar coating, comma coating, die coating, roll coating, flow coating, doctor coating, calendar coating, screen printing, flexographic printing, gravure printing, inkjet printing, etc.

The method for curing the resin is not particularly limited and may be selected as appropriate depending on the components (A) and (B) used in the bio-electrode composition. For example, curing by either or both of heat and light is preferable. Furthermore, the curing can also be performed by adding a catalyst for generating acid or a base in the above bio-electrode composition, thereby causing crosslinking reaction.

Note that the heating temperature is not particularly limited and may be selected as appropriate depending on the components (A) and (B) used in the bio-electrode composition. For example, the preferable temperature is about 50 to 250°C.

Furthermore, in a case of combining heating and light irradiation, the heating and light irradiation may be simultaneously performed, the heating may be performed after the light irradiation, or the light irradiation may be performed after the heating. Furthermore, air-drying may be performed for the purpose of evaporating the solvent before heating the applied film.

Fitting to skin can be improved by attaching water droplets to the cured film surface or spraying water vapor or mist, thereby enabling to quickly obtain biological signals. Water mixed with alcohol can also be used to reduce the size of water droplets in the water vapor or mist. The film surface can also be wetted by bringing the film surface into contact with an absorbent cotton or cloth containing water.

The water for making the surface of the cured film wet may contain a salt. The water-soluble salt to be mixed with the water is selected from a sodium salt, a potassium salt, a calcium salt, a magnesium salt, and betaine.

Specifically, the water-soluble salt can be a salt selected from sodium chloride, potassium chloride, calcium chloride, magnesium chloride, saccharin sodium salt, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, and betaine. Note that the ionic resin (A) as described above are excluded from the water-soluble salt.

More specific examples thereof include, besides those described above, sodium acetate, sodium propionate, sodium pivalate, sodium glycolate, sodium butyrate, sodium valerate, sodium caproate, sodium enanthate, sodium caprylate, sodium pelargonate, sodium caprate, sodium undecylate, sodium laurate, sodium tridecanoate, sodium myristate, sodium pentadecylate, sodium palmitate, sodium margarate, sodium stearate, sodium benzoate, disodium adipate, disodium maleate, disodium phthalate, sodium butyrate, sodium 2-hydroxybutyrate, sodium 3-hydroxybutyrate, sodium 2-oxobutyrate, sodium stearate, sodium gluconate, sodium methanesulfonate, sodium 1-nonanesulfonate, sodium 1-decanesulfonate, sodium 1-dodecanesulfonate, sodium 1-undecanesulfonate, sodium cocoyl isethionate, sodium lauroyl methylaminopropionate, sodium methyl cocoyl taurate, sodium cocoyl glutamate, sodium cocoyl sarcosinate, sodium lauroyl methyl taurate, lauramidopropyl, potassium isobutyrate, potassium propionate, potassium pivalate, potassium glycolate, potassium gluconate, potassium methanesulfonate, calcium stearate, calcium glycolate, calcium gluconate, calcium 3-methyl-2-oxobutyrate, and calcium methanesulfonate. Betaine is a general term for inner salts, and specifically refers to a compound in which three methyl groups are added to an amino group of amino acid. More specific examples thereof include trimethylglycine, carnitine, trimethylglycine, and proline betaine.

The water-soluble salt can further contain a monohydric or polyhydric alcohol having 1 to 4 carbon atoms, and the alcohol is preferably selected from ethanol, isopropyl alcohol, ethylene glycol, diethylene glycol, triethylene glycol, glycerin, polyethylene glycol, polypropylene glycol, polyglycerin, diglycerin, or a silicone compound having a polyglycerin structure, where the silicone compound having a polyglycerin structure is more preferably any of those represented by the above general formulae (4)' to (6)'.

In a pretreatment method with an aqueous solution containing the water-soluble salt, the cured bio-electrode film can be wetted by a spraying method, a water droplet dispense method, etc. The bio-electrode film can also be wetted under a high-temperature, high-humidity condition like a sauna. To prevent drying after the wetting, a protective film can be further laminated on the permeated layer to cover the same. Since the protective film should be removed immediately before the bio-electrode is attached to skin, it can be coated with a release agent, or a peelable Teflon^{®} film can be used therefor. For long-term storage, the dry electrode covered with the peelable film is preferably sealed in a bag covered with aluminum or the like. To prevent drying in the bag covered with aluminum, water is preferably enclosed therein.

Before the bio-electrode of the present invention is attached to skin, the skin can be moisturized with water, alcohol, etc., or the skin can be wiped with a cloth or absorbent cotton containing water, alcohol, etc. The water and the alcohol may also contain the above-described salts.

As described above, the method for producing a bio-electrode of the present invention makes it possible to easily produce the bio-electrode of the present invention at low cost, where the bio-electrode is excellent in electric conductivity and biocompatibility and lightweight, and causes no significant decrease in the electric conductivity even when gets wet from water or when dried.

### EXAMPLE

Hereinafter, the present invention will be specifically described with reference to Examples and Comparative Examples. However, the present invention is not limited thereto.

### (Synthesis of Monomers 1 to 11)

The following monomer 1 was obtained by reaction of bis(cyclohexanesulfonyl)methane with 4-methacryloyloxybenzenesulfonyl chloride in the presence of sodium hydride and neutralization reaction with trimethylbenzylammonium in a THF solvent. The bis(cyclohexanesulfonyl)methane and/or 4-methacryloyloxybenzenesulfonyl chloride were changed to other raw materials, and the trimethylbenzylammonium was subjected to ion exchange reaction with another cation, thereby synthesizing monomers 2 to 11.

The monomers 1 to 11 are shown below.

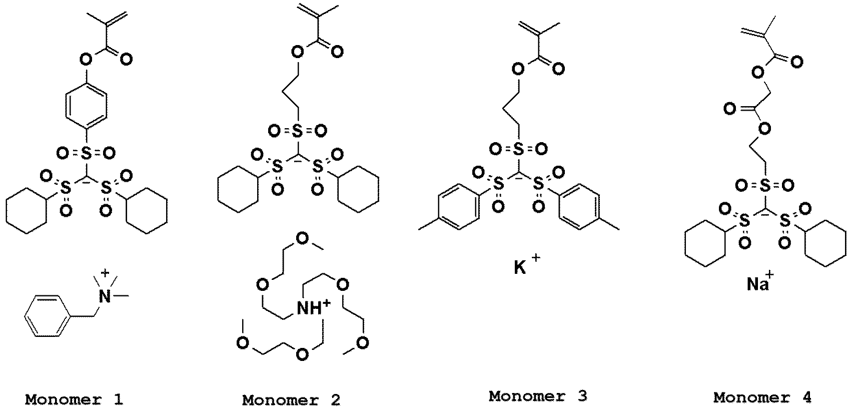

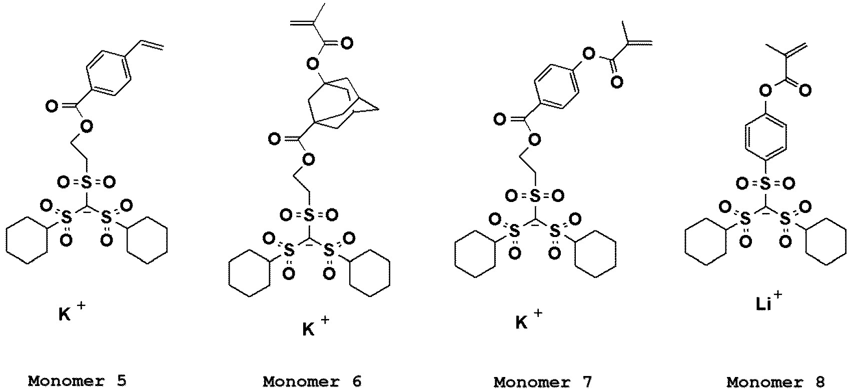

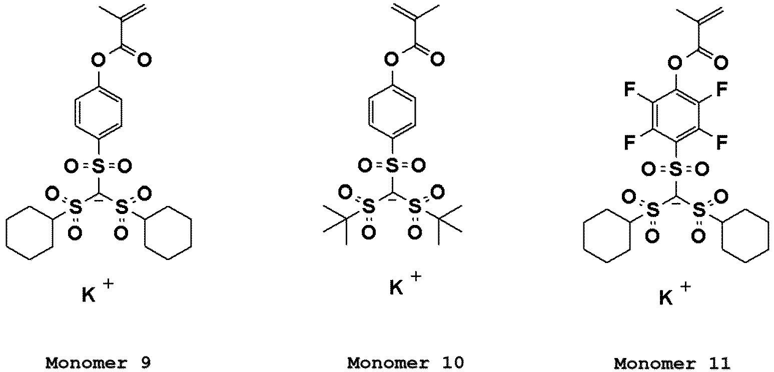

### (Synthesis of Ionic Resins 1-1 to 1-11, and Comparative Ionic Resin 1)

Ionic resins 1-1 to 1-11 and a comparative ionic resin 1, which were each blended into a bio-electrode composition solution as an ionic material (electro-conductive material), were synthesized as follows. A 30 mass% solution of each monomer in cyclopentanone was introduced into a reaction vessel and mixed. The reaction vessel was cooled to -70°C under a nitrogen atmosphere, degassed under reduced pressure, and subjected to nitrogen blowing three times. After raising the temperature to the room temperature, azobisisobutyronitrile (AIBN) was added thereto as a polymerization initiator in an amount of 0.02 moles per mole of the whole monomers. The resultant was warmed to 60°C and then allowed to react for 15 hours. The composition of the obtained polymer was determined by ¹H-NMR after drying the solvent. Additionally, the molecular weight (Mw) and the dispersity (Mw/Mn) of the obtained polymer were determined by gel permeation chromatography (GPC) using tetrahydrofuran (THF) as a solvent. The ionic resins 1-1 to 1-11 and the comparative ionic resin 1 synthesized in this manner are shown below.

### Ionic Resin 1-1

Mw=83,000
Mw/Mn=2.94

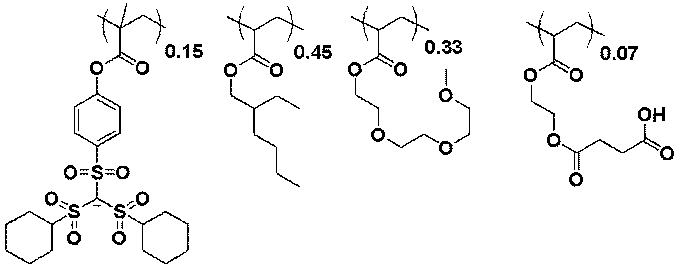

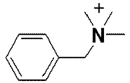

### Ionic Resin 1-2

Mw=77,100
Mw/Mn=2.93

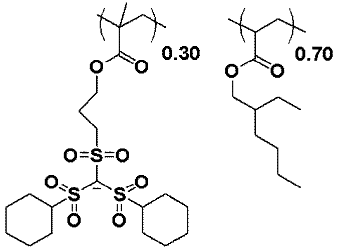

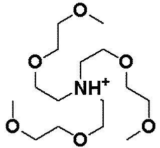

### Ionic Resin 1-3

Mw=69, 100
Mw/Mn=2. 64

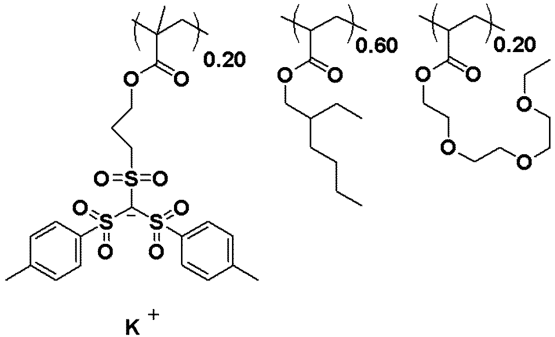

### Ionic Resin 1-4

Mw=52,200
Mw/Mn=2.73

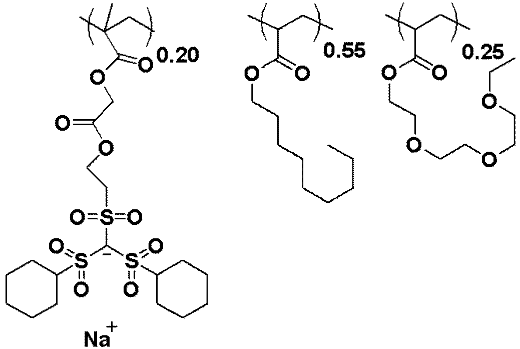

### Ionic Resin 1-5

Mw=31,900
Mw/Mn=2.77

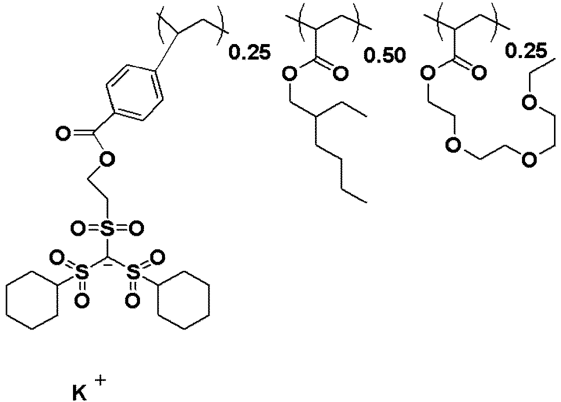

### Ionic Resin 1-6

Mw=40, 300
Mw/Mn=2.83

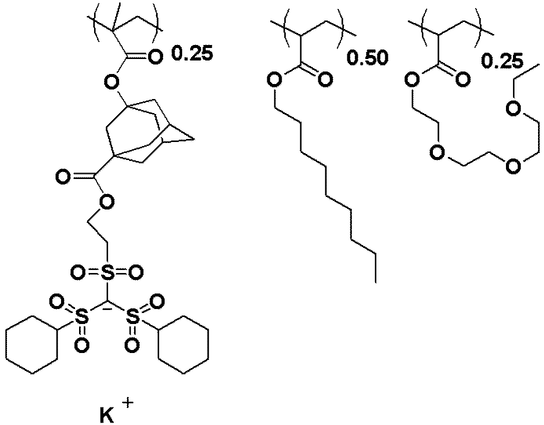

### Ionic Resin 1-7

Mw=38,100
Mw/Mn=2.77

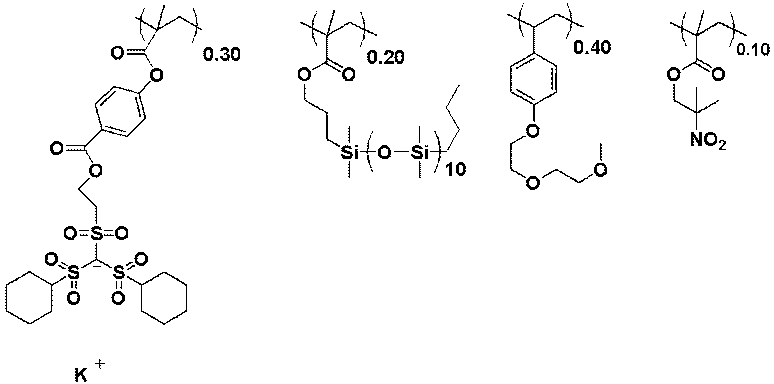

### (The repeating number in the formula shows the average value.)

### Ionic Resin 1-8

Mw=42,700
Mw/Mn=1.95

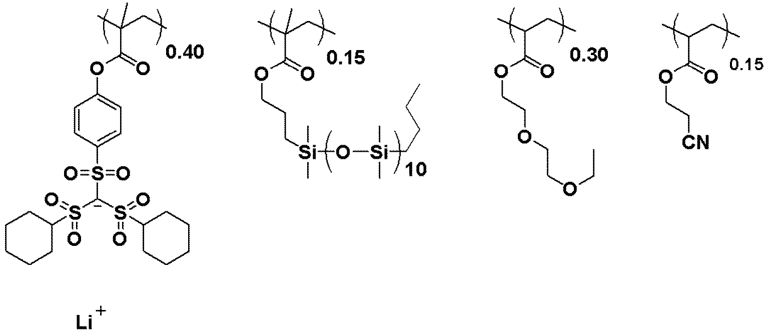

### (The repeating number in the formula shows the average value.)

### Ionic Resin 1-9

Mw=31,500
Mw/Mn=2.33

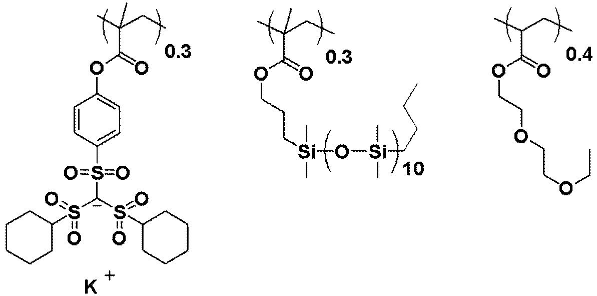

### (The repeating number in the formula shows the average value.)

### Ionic Resin 1-10

Mw=29,800
Mw/Mn=1.95

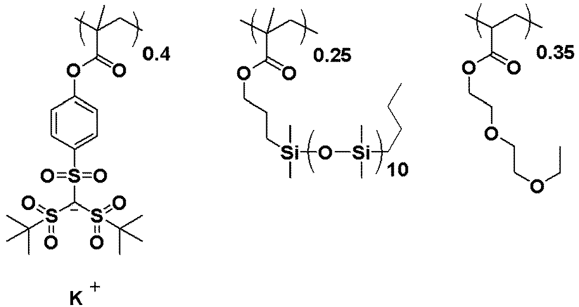

### (The repeating number in the formula shows the average value.)

### Ionic Resin 1-11

Mw=33,500
Mw/Mn=1.91

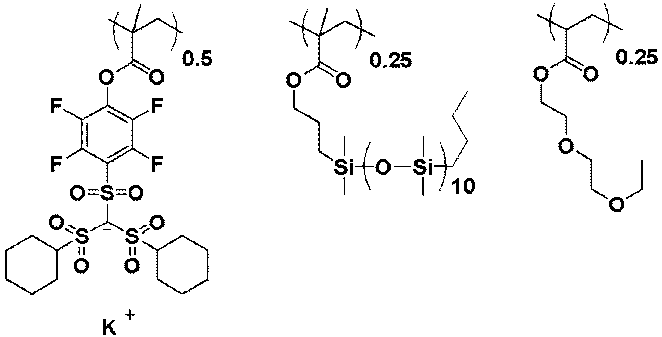

### (The repeating number in the formula shows the average value.)

### Comparative Ionic Resin 1

Mw=26,500
Mw/Mn=1.85

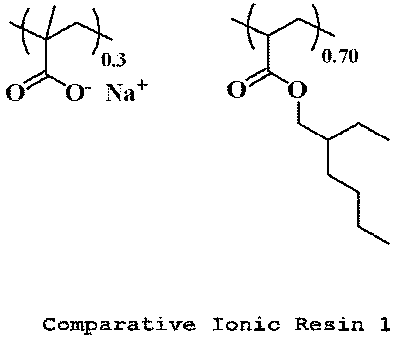

Siloxane compounds 1 to 4 blended into the bio-electrode composition solution as a silicone-based resin are shown below.

### (Siloxane Compound 1)

The siloxane compound 1 was a vinyl group-containing polydimethylsiloxane, which had a viscosity of 27,000 mPa·s in a 30% toluene solution, an alkenyl group content of 0.007 mol/100 g, and the molecular chain terminal blocked with a SiMe₂Vi group.

### (Siloxane Compound 2)

The siloxane compound 2 was a 60% toluene solution of polysiloxane of MQ resin composed of Me₃SiO_{0.5} and SiO₂ units (Me₃SiO_{0.5} unit/SiO₂ unit=0.8).

### (Siloxane Compound 3)

The siloxane compound 3 was polydimethylsiloxane-bonded MQ resin obtained by heating under reflux for 4 hours and then cooling a solution composed of: 40 parts by mass of vinyl group-containing polydimethylsiloxane having a viscosity of 42,000 mPa·s in a 30% toluene solution, an alkenyl group content of 0.007 mol/100 g, and the molecular chain terminal blocked with OH; 100 parts by mass of a 60% toluene solution of polysiloxane of MQ resin composed of Me₃SiO_{0.5} and SiO₂ units (Me₃SiO_{0.5} unit/SiO₂ unit=0.8); and 26.7 parts by mass of toluene.

### (Siloxane Compound 4)

As methyl hydrogen silicone oil, KF-99 manufactured by Shin-Etsu Chemical Co., Ltd. was used.

An acrylic resin blended into the bio-electrode composition solution as an acrylic-based resin is shown below.

### Acrylic Resin 1

Mw=129,000
Mw/Mn=2.45

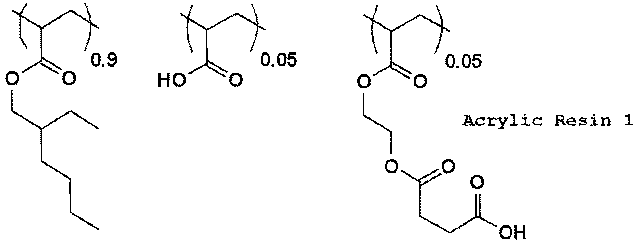

An urethane resin blended into the bio-electrode composition solution as an urethane-based resin is shown below.

### Urethane Resin 1

Mw=83,000
Mw/Mn=4.02

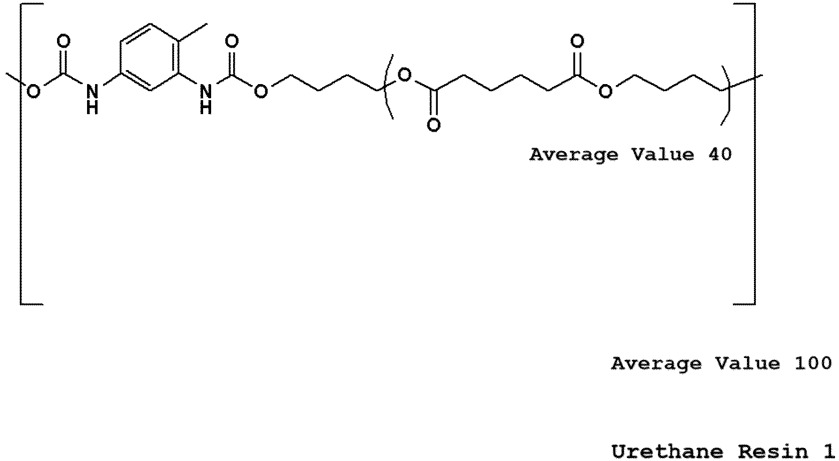

A polyglycerin silicone compound blended into the bio-electrode composition solution is shown below.

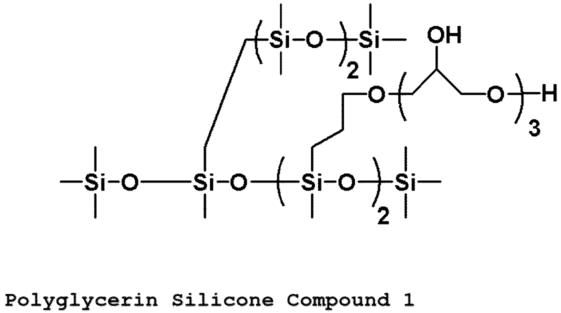

A crosslinking agent blended into the bio-electrode composition solution is shown below.

### Epoxy Crosslinking Agent 1

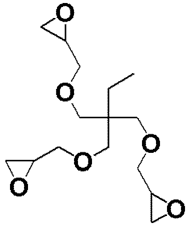

Organic solvents blended into the bio-electrode composition solution are shown below.
EDE: diethylene glycol diethyl ether
ISOPAR G^{™}: isoparaffinic solvent available from Standard Oil
ISOPAR M^{™}: isoparaffinic solvent available from Standard Oil

A platinum catalyst and an electric conductivity improver (carbon black, carbon nanotube), which were blended into the bio-electrode composition solution as additives, are shown below.
Platinum catalyst: CAT-PL-50T manufactured by Shin-Etsu Chemical Co., Ltd.
Carbon black: DENKA BLACK Li-400 manufactured by Denka Company Limited
Multilayer carbon nanotube: manufactured by Sigma-Aldrich Co., LLC, diameter: 110 to 170 nm, length: 5 to 9 µm

### Examples 1 to 11, Comparative Example 1

Each of the bio-electrode composition solutions (bio-electrode composition solutions 1 to 11, comparative bio-electrode composition solution 1) was prepared by blending the ionic resin, the resin, the organic solvents, and the additives (platinum catalyst, electric conductivity improver) according to the compositions shown in Tables 1 and 2.

**[Table 1]**

| Bio-electrode composition solution | Ionic resin (parts by mass) | Resin (parts by mass) | Organic solvent (parts by mass) | Additive (parts by mass) |
|---|---|---|---|---|
| Bio-electrode composition solution 1 | Ionic resin 1-1 (70) | Acrylic resin 1 (30) | EDE(60) | Epoxy crosslinking agent 1(0.2) |
| | | | Cyclopentanone (47) | |
| | | | | Carbon black(8) |
| Bio-electrode composition solution 2 | Ionic resin 1-2 (70) | Acrylic resin 1 (30) | EDE(60) | Epoxy crosslinking agent 1(0.2) |
| | | | Cyclopentanone (47) | |
| | | | | Carbon black(8) |
| Bio-electrode composition solution 3 | Ionic resin 1-3 (30) | Acrylic resin 1 (70) | EDE(60) | Epoxy crosslinking agent 1(0.2) |
| | | | Cyclopentanone (47) | |
| | | | | Carbon black(8) |
| bio-electrode composition solution 4 | Ionic resin 1-4 (30) | Acrylic resin 1 (70) | EDE(60) | Epoxy crosslinking agent 1(0.2) |
| | | | Cyclopentanone (47) | |
| | | | | Carbon black(8) |
| Bio-electrode composition solution 5 | Ionic resin 1-5 (40) | Acrylic resin 1 (60) | EDE(60) | Epoxy crosslinking agent 1(0.2) |
| | | | Cyclopentanone (47) | |
| | | | | Carbon black(8) |
| Bio-electrode composition solution 6 | Ionic resin 1-6 (40) | Urethane resin 1 (60) | EDE(60) | Multilayer carbon nanotube (3) |
| | | | Cyclopentanone (47) | |
| Bio-electrode composition solution 7 | Ionic resin 1-7 (20) | Siloxane compound 1 (40) | ISOPAR G (60) | CAT-PL-50T (1.5) |
| | | Siloxane compound 2 (100) | | Carbon black (8) |
| | | | Cyclopentanone (47) | |
| | | | | Polyglycerin compound 1 (8) |
| | | Siloxane compound 4 (3) | | |
| Bio-electrode composition solution 8 | Ionic resin 1-8 (10) | Siloxane compound 1 (40) | ISOPAR G (60) | CAT-PL-50T (1.5) |
| | | Siloxane compound 2 (100) | | Carbon black (8) |
| | | | Cyclopentanone (47) | |
| | | | | Polyglycerin compound 1 (8) |
| | | Siloxane compound 4 (3) | | |
| Bio-electrode composition solution 9 | Ionic resin 1-9 (20) | Siloxane compound 3 (126) | ISOPAR M (60) | CAT-PL-50T(1.5) |
| | | | | Carbon black(8) |
| | | | | Polyglycerin compound 1 (8) |
| | | Siloxane compound 4 (3) | Cyclopentanone (47) | |
| Bio-electrode composition solution 10 | Ionic resin 1-10 (12) | Siloxane compound 3 (126) | ISOPAR M (60) | CAT-PL-50T(1.5) |
| | | | | Carbon black(12) |
| | | | | Polyglycerin compound 1 (8) |
| | | Siloxane compound 4 (3) | Cyclopentanone (47) | |
| Bio-electrode composition solution 11 | Ionic resin 1-11 (12) | Siloxane compound 3 (126) | ISOPAR M (60) | CAT-PL-50T(1.5) |
| | | | | Carbon black(12) |
| | | | | Polyglycerin compound 1 (8) |
| | | Siloxane compound 4 (3) | Cyclopentanone (47) | |

**[Table 2]**

| Bio-electrode composition solution | Ionic resin (parts by mass) | Resin (parts by mass) | Organic solvent (parts by mass) | Additive (parts by mass) |
|---|---|---|---|---|
| Comparative bio-electrode composition solution 1 | Comparative ionic resin 1 (70) | Acrylic resin 1 (30) | EDE(60) | Epoxy crosslinking agent 1(0.2) |
| | | | Cyclopentanone(47) | |
| | | | | Carbon black (8) |

### Biological Signal Evaluation

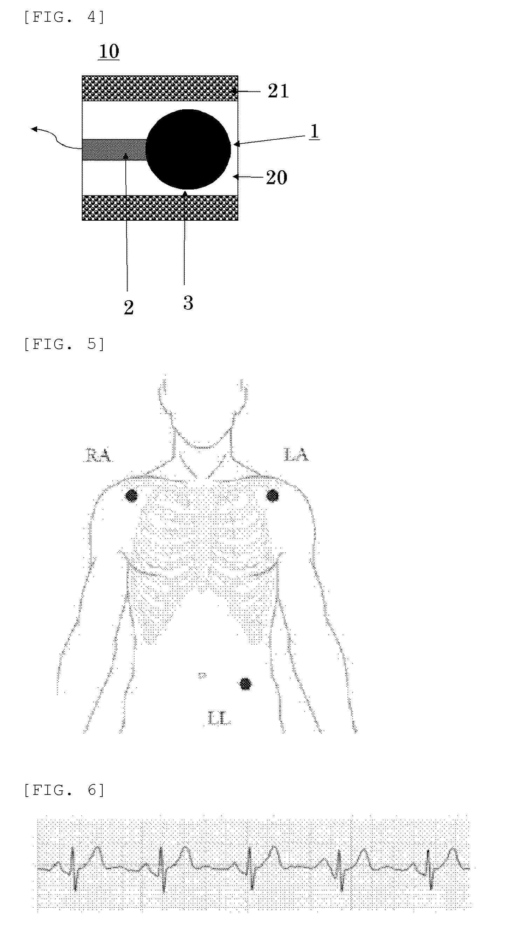

As shown in FIG. 3, a thermoplastic urethane (TPU) film 20, ST-604 available from Bemis Associates Inc., was coated with electro-conductive paste, DOTITE FA-333 manufactured by Fujikura Kasei Co., Ltd., by screen printing, and baked in an oven at 120°C for 10 minutes to print a keyhole-shaped electro-conductive pattern 2 having a circular portion with a diameter of 2 cm. Each of the bio-electrode composition solutions shown in Tables 1 and 2 was applied onto the circular portion by screen printing, subjected to air-drying at the room temperature for 10 minutes, baked using an oven at 125°C for 10 minutes to evaporate the solvents, and cured to form the living body contact layer 3, thereby preparing the bio-electrode 1. Next, as shown in FIG. 4, the urethane film 20 having the printed bio-electrode 1 was cut out and a double-sided tape 21 was attached thereto, thereby fabricating three bio-electrode samples 10 for each of the composition solutions.

### (Thickness Measurement of Living Body Contact Layer)

For the bio-electrode fabricated in the above biological signal evaluation test, the thickness of the living body contact layer was measured by using a micrometer. The results are shown in Table 3.

### (Biological Signal Measurement)

The electro-conductive wiring pattern formed from the electro-conductive paste of the bio-electrode was coupled to a portable electrocardiogram HCG-901 manufactured by OMRON HEALTHCARE Co., Ltd. through electro-conductive wire. The positive electrode of the electrocardiogram was attached to a position LA on a human body in FIG. 5, the negative electrode was attached to a position LL, and the earth was attached to a position RA. Immediately after the attachment, electrocardiogram measurement was started to measure the time till an electrocardiogram waveform including P, Q, R, S, and T waves appeared as shown in FIG. 6. The results are shown in Table 3.

**[Table 3]**

| Example | Bio-electrode composition solution | Resin thickness (micron) | Time till ECG signal appeared (minute) |
|---|---|---|---|
| Example 1 | Bio-electrode composition solution 1 | 54 | 5 |
| Example 2 | Bio-electrode composition solution 2 | 50 | 4 |
| Example 3 | Bio-electrode composition solution 3 | 55 | 2 |
| Example 4 | Bio-electrode composition solution 4 | 62 | 3 |
| Example 5 | Bio-electrode composition solution 5 | 51 | 1 |
| Example 6 | Bio-electrode composition solution 6 | 52 | 2 |
| Example 7 | Bio-electrode composition solution 7 | 53 | 1 |
| Example 8 | Bio-electrode composition solution 8 | 49 | 1 |
| Example 9 | Bio-electrode composition solution 9 | 52 | 1 |
| Example 10 | Bio-electrode composition solution 10 | 53 | 0 |
| Example 11 | Bio-electrode composition solution 11 | 57 | 0 |
| Comparative Example 1 | Comparative bio-electrode composition solution 1 | 52 | Not appeared |

As shown in Table 3, in Examples 1 to 11, where the living body contact layer was formed by using the bio-electrode composition of the present invention containing the resin having a structure selected from an ammonium salt, a lithium salt, a sodium salt, and a potassium salt of trissulfonium methide, biological signals were obtained within a short time after the attachment to the body. On the other hand, when the ion component having a specific structure was not contained, biological signals could not be obtained.

The present description includes the following inventions.
[1]: A bio-electrode composition comprising (A) an ionic resin, wherein the component (A) contains a resin having a structure selected from an ammonium salt, a lithium salt, a sodium salt, and a potassium salt of trissulfonium methide.
[2]: The bio-electrode composition according to [1], wherein the resin having a structure selected from an ammonium salt, a lithium salt, a sodium salt, and a potassium salt of trissulfonium methide has a chemical structure represented by the following general formula (1), 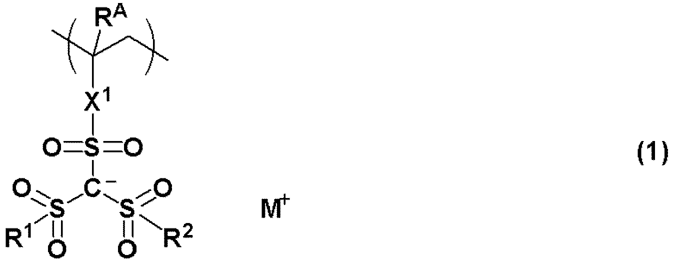 wherein R^{A} represents a hydrogen atom or a methyl group; X¹ each independently represents a single bond, a phenylene group, or a linking group having 1 to 20 carbon atoms and containing at least one selected from an ester bond, an ether bond, an urethane bond, a lactone ring, and a halogen atom; each of R¹ and R² independently represents a hydrocarbyl group having 1 to 20 carbon atoms and optionally containing a heteroatom; and M⁺ represents any of an ammonium ion, a lithium ion, a sodium ion, and a potassium ion.
[3]: The bio-electrode composition according to [2], wherein the resin having a structure selected from an ammonium salt, a lithium salt, a sodium salt, and a potassium salt of trissulfonium methide contains an ammonium ion represented by the following general formula (2) as the M⁺, 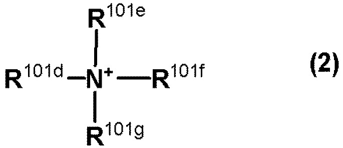 wherein each of R^{101d}, R^{101e}, R^{101f}, and R^{101g} represents a hydrogen atom, a linear, branched, or cyclic alkyl group having 1 to 12 carbon atoms, a linear, branched, or cyclic alkenyl group or alkynyl group having 2 to 12 carbon atoms, or an aromatic group having 4 to 20 carbon atoms, and optionally contains one or more selected from an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring with a nitrogen atom attached thereto, and when forming the ring, R^{101d} and R^{101e}, or R^{101d}, R^{101e}, and R^{101f} represent an alkylene group having 3 to 10 carbon atoms, or form a heteroaromatic ring having the nitrogen atom in the formula in the ring.
[4]: The bio-electrode composition according to any one of [1] to [3], further comprising, as a component (B), a resin other than the component (A).
[5]: The bio-electrode composition according to [4], wherein the component (B) is one or more selected from a silicone resin, a (meth)acrylate resin, and an urethane resin.
[6]: The bio-electrode composition according to [4] or [5], wherein the component (B) has adhesiveness.
[7]: The bio-electrode composition according to any one of [4] to [6], comprising, as the component (B), a silicone resin having a RₓSiO_{(4-x)/2} unit (R represents a substituted or unsubstituted monovalent hydrocarbon group having 1 to 10 carbon atoms; and "x" is within a range of 2.5 to 3.5) and a SiO₂ unit.
[8]: The bio-electrode composition according to any one of [1] to [7], further comprising carbon powder and/or metal powder as a component (C).
[9]: The bio-electrode composition according to [8], wherein the carbon powder is one or both of carbon black and carbon nanotube.
[10]: The bio-electrode composition according to [8] or [9], wherein the metal powder is powder of a metal selected from gold, silver, platinum, copper, tin, titanium, nickel, aluminum, tungsten, molybdenum, ruthenium, chromium, and indium.
[11]: The bio-electrode composition according to any one of [8] to [10], wherein the metal powder is silver powder.
[12]: The bio-electrode composition according to any one of [1] to [11], further comprising an organic solvent as a component (D).
[13]: A bio-electrode comprising an electro-conductive base material and a living body contact layer formed on the electro-conductive base material, wherein the living body contact layer is a cured material of the bio-electrode composition according to any one of [1] to [12].
[14]: The bio-electrode according to [13], wherein the electro-conductive base material comprises one or more selected from gold, silver, silver chloride, platinum, aluminum, magnesium, tin, tungsten, iron, copper, nickel, stainless steel, chromium, titanium, carbon, and an electro-conductive polymer.
[15]: A method for producing a bio-electrode comprising an electro-conductive base material and a living body contact layer formed on the electro-conductive base material, the method comprising: applying the bio-electrode composition according to any one of [1] to [12] onto the electro-conductive base material; and curing the bio-electrode composition to form the living body contact layer.
[16]: The method for producing a bio-electrode according to [15], wherein the electro-conductive base material to be used comprises one or more selected from gold, silver, silver chloride, platinum, aluminum, magnesium, tin, tungsten, iron, copper, nickel, stainless steel, chromium, titanium, carbon, and an electro-conductive polymer.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A bio-electrode composition comprising (A) an ionic resin,
wherein the component (A) contains a resin having a structure selected from an ammonium salt, a lithium salt, a sodium salt, and a potassium salt of trissulfonium methide.

2. The bio-electrode composition according to claim 1, wherein the resin having a structure selected from an ammonium salt, a lithium salt, a sodium salt, and a potassium salt of trissulfonium methide has a chemical structure represented by the following general formula (1), 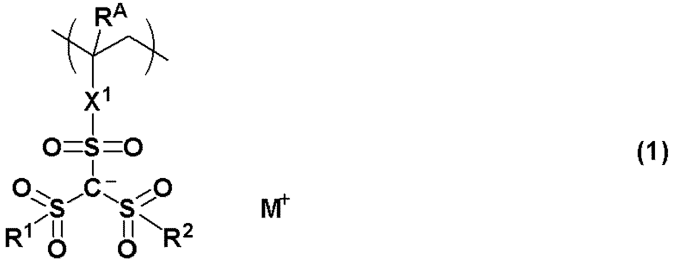 wherein R^{A} represents a hydrogen atom or a methyl group; X¹ each independently represents a single bond, a phenylene group, or a linking group having 1 to 20 carbon atoms and containing at least one selected from an ester bond, an ether bond, an urethane bond, a lactone ring, and a halogen atom; each of R¹ and R² independently represents a hydrocarbyl group having 1 to 20 carbon atoms and optionally containing a heteroatom; and M⁺ represents any of an ammonium ion, a lithium ion, a sodium ion, and a potassium ion.

3. The bio-electrode composition according to claim 2, wherein the resin having a structure selected from an ammonium salt, a lithium salt, a sodium salt, and a potassium salt of trissulfonium methide contains an ammonium ion represented by the following general formula (2) as the M⁺, 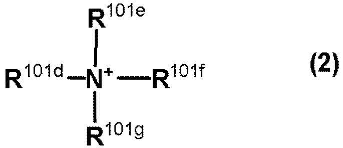 wherein each of R^{101d}, R^{101e}, R^{101f}, and R^{101g} represents a hydrogen atom, a linear, branched, or cyclic alkyl group having 1 to 12 carbon atoms, a linear, branched, or cyclic alkenyl group or alkynyl group having 2 to 12 carbon atoms, or an aromatic group having 4 to 20 carbon atoms, and optionally contains one or more selected from an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring with a nitrogen atom attached thereto, and when forming the ring, R^{101d} and R^{101e}, or R^{101d}, R^{101e}, and R^{101f} represent an alkylene group having 3 to 10 carbon atoms, or form a heteroaromatic ring having the nitrogen atom in the formula in the ring.

4. The bio-electrode composition according to any one of claims 1 to 3, further comprising, as a component (B), a resin other than the component (A).

5. The bio-electrode composition according to claim 4, wherein the component (B) is one or more selected from a silicone resin, a (meth)acrylate resin, and an urethane resin.

6. The bio-electrode composition according to claim 4 or 5, wherein the component (B) has adhesiveness.

7. The bio-electrode composition according to any one of claims 4 to 6, comprising, as the component (B), a silicone resin having a RₓSiO_{(4-x)/2} unit (R represents a substituted or unsubstituted monovalent hydrocarbon group having 1 to 10 carbon atoms; and "x" is within a range of 2.5 to 3.5) and a SiO₂ unit.

8. The bio-electrode composition according to any one of claims 1 to 7, further comprising carbon powder and/or metal powder as a component (C).

9. The bio-electrode composition according to claim 8, wherein the carbon powder is one or both of carbon black and carbon nanotube.

10. The bio-electrode composition according to claim 8 or 9, wherein the metal powder is powder of a metal selected from gold, silver, platinum, copper, tin, titanium, nickel, aluminum, tungsten, molybdenum, ruthenium, chromium, and indium.

11. The bio-electrode composition according to any one of claims 8 to 10, wherein the metal powder is silver powder.

12. The bio-electrode composition according to any one of claims 1 to 11, further comprising an organic solvent as a component (D).

13. A bio-electrode comprising an electro-conductive base material and a living body contact layer formed on the electro-conductive base material, wherein the living body contact layer is a cured material of the bio-electrode composition according to any one of claims 1 to 12.

14. The bio-electrode according to claim 13, wherein the electro-conductive base material comprises one or more selected from gold, silver, silver chloride, platinum, aluminum, magnesium, tin, tungsten, iron, copper, nickel, stainless steel, chromium, titanium, carbon, and an electro-conductive polymer.

15. A method for producing a bio-electrode comprising an electro-conductive base material and a living body contact layer formed on the electro-conductive base material, the method comprising: applying the bio-electrode composition according to any one of claims 1 to 12 onto the electro-conductive base material; and curing the bio-electrode composition to form the living body contact layer, preferably,
wherein the electro-conductive base material to be used comprises one or more selected from gold, silver, silver chloride, platinum, aluminum, magnesium, tin, tungsten, iron, copper, nickel, stainless steel, chromium, titanium, carbon, and an electro-conductive polymer.
